# EUROPEAN PATENT APPLICATION

(11) **EP 4 464 700 A1**
(43) Date of publication of application: **20.11.2024**
(21) Application number: 23740093.2
(22) Date of filing: 13.01.2023
(51) Int. Cl.: C07D 413/12, C07D 401/12, C07D 498/08, C07D 498/10, C07D 283/00, C07D 331/04, C07D 295/04, C07F 5/04, A61K 31/4436, A61K 31/4427, A61P 35/00, A61P 17/00

(54) **DIARYL COMPOUND AS TUBULIN/SRC DUAL TARGET INHIBITOR**

(30) Priority: 14.01.2022 CN 202210044016; 15.04.2022 CN 202210399397; 19.09.2022 CN 202211139978; 06.01.2023 CN 202310020643
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); YANG, Hui, Wuhan, Hubei 430075 (CN); LEI, Sijun, Wuhan, Hubei 430075 (CN); WEI, Wenjun, Wuhan, Hubei 430075 (CN); WU, Zhiqiang, Wuhan, Hubei 430075 (CN); LIU, Lifei, Wuhan, Hubei 430075 (CN); ZHANG, Xin, Wuhan, Hubei 430075 (CN); LI, Li'e, Wuhan, Hubei 430075 (CN); YANG, Jun, Wuhan, Hubei 430075 (CN)
(74) Representative: Haseltine Lake Kempner LLP
(86) International application number: PCT/CN2023/072143
(87) International publication number: WO 2023/134752

(57) **Abstract**

The present invention provides a diaryl compound as a tubulin/Src dual target inhibitor. The present invention also provides a diaryl compound represented by formula I, a tautomer, a stereoisomer, a solvate, a pharmaceutically acceptable salt, or a prodrug thereof. The diaryl compound can be used as a dual target inhibitor against tubulin and Src kinase, and can also be used as a mono-target inhibitor against tubulin or Src kinase. The compound of the present invention can significantly inhibit the polymerization of tubulin monomers and cell proliferation.

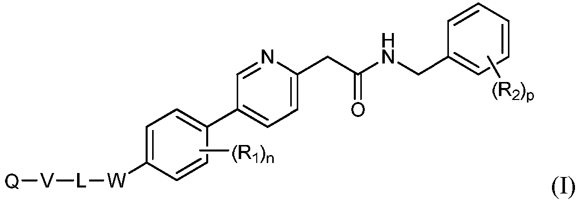

## Description

The present application claims the right of the priorities of Chinese patent application 202210044016.7 filed on January 14, 2022, Chinese patent application 202210399397.0 filed on April 15, 2022, Chinese patent application 202211139978.7 filed on September 19, 2022, and Chinese patent application 202310020643.1 filed on January 06, 2023. The contents of the above Chinese patent applications are incorporated herein by reference in their entireties.

### TECHNICAL FIELD

The present disclosure relates to a diaryl compound as a tubulin/Src dual target inhibitor.

### BACKGROUND

Microtubules are an important component of the cytoskeleton in eukaryotic cells and play an important role in a variety of cellular functions such as maintaining cell morphology, signal transmission, organelle transport, cell movement, cell division, and mitosis (Jordan M A et al. Nature Reviews Cancer, 2004, 4(4): 253-265.).

Microtubules are composed of two types of tubulin subunits, namely α-tubulin and β-tubulin. α-tubulin and β-tubulin form tubulin heterodimers, which are the basic units of microtubule assembly. Microtubule-targeting agents (MTAs) can destroy the dynamic stability and structure of microtubules, interfere with the formation of mitotic spindles, induce cell cycle arrest in the G2/M phase, and promote cell apoptosis (Shuai W et al. Journal of Medicinal Chemistry, 2021, 64(12).).

Microtubules are involved in many important cellular processes and have become one of the most important drug targets for the treatment of hyperproliferative diseases. Several microtubule-targeting agents approved by FDA of the United States, such as vinblastine and taxane compounds, are widely used to treat multiple solid tumors and hematological malignancies, but the drug resistance and dose-limiting toxicity of microtubule-targeting drugs limit their clinical efficacy. Compared with single-target drugs, dual-target inhibitors overcome drug resistance, can improve therapeutic effects, and have become a research hotspot, such as: tubulin-SRC dual-target inhibitors, tubulin-receptor tyrosine kinase (RTK) dual-target inhibitors, tubulin-histone deacetylases (HDAC) dual-target inhibitors (Shuai W et al. Journal of Medicinal Chemistry, 2021, 64(12).).

Actinic keratosis (AK) is a skin disease related to long-term exposure to ultraviolet light. AK is the second most common disease among dermatologists in the United States, characterized by uncontrolled proliferation of mutant keratinocytes, which is considered as a precancerous lesion. If not treated in time, 20% of cases may develop into cutaneous squamous cell carcinoma (SCC). At present, Tirbanibulin, a tubulin/SRC dual target inhibitor, has a remarkable clinical effect on local treatment of AK (NCT03285477), which has been approved for marketing by FDA. This indicates that it may be a promising direction to develop a new tubulin/SRC dual target inhibitor with better effect for local treatment of AK.

### CONTENT OF THE PRESENT INVENTION

The object of the present disclosure is to provide a diaryl compound as a tubulin/Src dual target inhibitor, a preparation method therefor, and a use thereof, the diaryl compound having a structure of formula I as described in the present disclosure. The diaryl compound can be used as a dual-target inhibitor against tubulin and Src kinase, and can also be used as a mono-target inhibitor against tubulin or Src kinase.

In a first aspect of the present disclosure, provided is a diaryl compound of formula I, a tautomer, a stereoisomer, a solvate (such as a hydrate), a pharmaceutically acceptable salt, or a prodrug thereof, having a structure of: 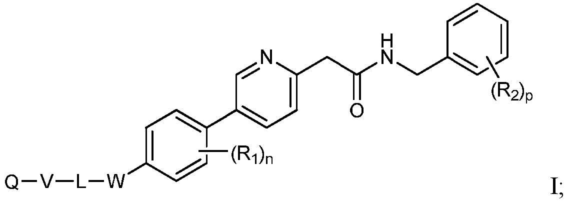
wherein W is selected from: -O-, -S-, -NH-, -N(C₁-C₆ alkyl)-;
L is C₁-C₆ alkylene;
V is absent or selected from: -O-, -S-, -NH-, -N(C₁-C₆ alkyl)-;
when V is absent, Q is ring A which is unsubstituted or substituted by m R₃, or ring B which is unsubstituted or substituted by m R₃;
when V is selected from: -O-, -S-, -NH-, -N(C₁-C₆ alkyl)-, Q is ring C which is unsubstituted or substituted by m R₃;
the ring A is a 6- to 15-membered heterocyclyl ring; and when ring A is a 6-membered heterocyclyl ring, the ring A is 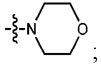
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;

R₁, R₂, and R₃ are each independently hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl,-CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₁, R₂, and R₃ are optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
n is 1, 2, or 3; when there is more than one R₁, R₁ groups are the same or different;
p is 1, 2, or 3; when there is more than one R₂, R₂ groups are the same or different;
m is 1, 2, or 3; when there is more than one R₃, R₃ groups are the same or different;
provided that when Q is ring A, R₁ and R₂ are not hydrogen at the same time;
when the ring A is 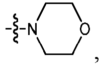 R₃ is not hydrogen;
or when the ring A is 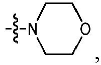 at least one of R₁ and R₂ is selected from: cyano, C₂-C₆ alkenyl, C₂-C₆ alkynyl, -C(O)NR₁₁R₁₂.

In the present disclosure, the definitions of some substituents in the diaryl compound of formula I, the tautomer, the stereoisomer, the solvate (such as hydrate), the pharmaceutically acceptable salt, or the prodrug thereof may be described as follows, and the definitions of unmentioned substituents are as described in any one of the embodiments of the present disclosure (hereinafter referred to as "in a preferred example", "in a preferred embodiment", or "in a preferred implementation"):
wherein W is selected from: -O-, -S-, -NH-, -N(C₁-C₆ alkyl)-;
L is C₁-C₆ alkylene;
V is absent or selected from: -O-, -S-, -NH-, -N(C₁-C₆ alkyl)-;
when V is absent, Q is ring A which is unsubstituted or substituted by m R₃, or ring B which is unsubstituted or substituted by m R₃;
when V is selected from: -O-, -S-, -NH-, -N(C₁-C₆ alkyl)-, Q is ring C which is unsubstituted or substituted by m R₃;
the ring A is a 6- to 15-membered heterocyclyl ring; and when ring A is a 6-membered heterocyclyl ring, the ring A is 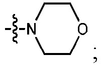
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
R₁, R₂, and R₃ are each independently hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, - CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₁, R₂, and R₃ are optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
n is 1, 2, or 3; when there is more than one R₁, R₁ groups are the same or different;
p is 1, 2, or 3; when there is more than one R₂, R₂ groups are the same or different;
m is 1, 2, or 3; when there is more than one R₃, R₃ groups are the same or different;
the diaryl compound of formula I satisfies 1, 2, or 3 of the following conditions:
   (1) V is selected from: -O-, -S-, -NH-, -N(C₁-C₆ alkyl)-;
   (2) Q is a 6- to 12-membered sulfonyl-containing heterocyclyl ring which is unsubstituted or substituted by m R₃;
   (3) R₁ is selected from: hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂; or, R₁ is halogen, and ring A, ring B, or ring C is a fused ring, a bridged ring, or a spiro ring.

In a preferred example, V is selected from: -O-, -S-, -NH-, -N(C₁-C₆ alkyl)-.

In a preferred example, V is selected from -NH-.

In a preferred example, Q is a 6- to 12-membered sulfonyl-containing heterocyclyl ring which is unsubstituted or substituted by m R₃.

In a preferred example, Q is an unsubstituted 6- to 12-membered sulfonyl-containing heterocyclyl ring.

In a preferred example, R₁ is selected from: hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl,-S(O)₂-3- to 6-membered cycloalkyl, -C(O)-C₁-C₆ alkyl-NR₁₁R₁₂.

In a preferred example, R₁ is selected from: cyano, C₁-C₆ alkyl, and C₂-C₆ alkynyl.

In a preferred example, R₁ is halogen, and ring A, ring B, or ring C is a fused ring, a bridged ring, or a spiro ring.

In a preferred example, the diaryl compound of formula I is 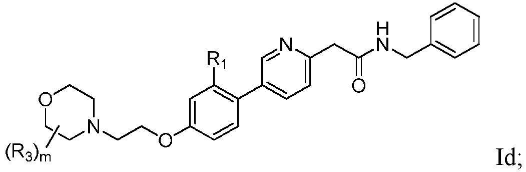
m is 1 or 2;
R₃ is selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₆ alkenyl, C₂-C₆ alkynyl, -C(O)NR₁₁R₁₂.

In a preferred example, the diaryl compound of formula I is 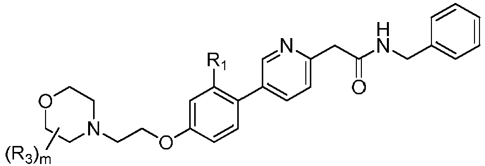 Id; m is 1; R₃ is C₁-C₆ alkyl.

In a preferred example, the diaryl compound of formula I is 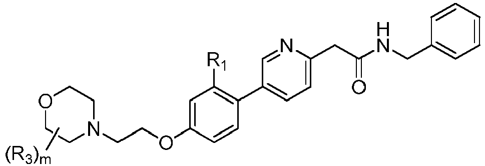 Id; m is 1; R₃ is C₁-C₆ alkyl; R₁ is selected from: cyano, C₂-C₆ alkenyl, C₂-C₆ alkynyl, -C(O)NR₁₁R₁₂.

In a preferred example, provided that when Q is ring A, R₁ and R₂ are not hydrogen at the same time;
and when the ring A is 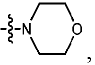 R₃ is not hydrogen, or at least one of R₁ and R₂ is selected from: cyano, C₂-C₆ alkenyl, C₂-C₆ alkynyl, -C(O)NR₁₁R₁₂.

In a preferred example, W is selected from -O-.

In a preferred example, L is C₁-C₆ alkylene.

In a preferred example, V is absent or selected from: -NH-;
when V is absent, Q is ring A which is unsubstituted or substituted by m R₃, or ring B which is unsubstituted or substituted by m R₃;
when V is selected from: -NH-, Q is ring C which is unsubstituted or substituted by m R3;
the ring A is a 6- to 15-membered heterocyclyl ring; and when ring A is a 6-membered
heterocyclyl ring, the ring A is 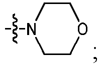
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
m is 1, 2, or 3; when there is more than one R₃, R₃ groups are the same or different;
R₃ is C₁-C₆ alkyl or -CO-C₁-C₆ alkyl; R₃ is optionally substituted by one or more than one 3- to 6-membered cycloalkyl; when there is more than one substituent, the substituents are the same or different.

In a preferred example, n is 1, 2, or 3; when there is more than one R₁, R₁ groups are the same or different;
R₁ is hydrogen, halogen, cyano, unsubstituted C₁-C₆ alkyl, or unsubstituted C₂-C₆ alkynyl.

In a preferred example, n is 1;
R₁ is hydrogen, halogen, cyano, unsubstituted C₁-C₆ alkyl, or unsubstituted C₂-C₆ alkynyl.

In a preferred example, p is 1, 2, or 3; when there is more than one R₂, R₂ groups are the same or different;
R₂ is hydrogen or halogen.

In a preferred example, p is 1; R₂ is hydrogen.

In a preferred example, the diaryl compound of formula I satisfies 1, 2, or 3 of the following conditions:
(1) V is selected from -NH-;
(2) Q is a 6- to 12-membered sulfonyl-containing heterocyclyl ring which is unsubstituted or substituted by m R₃;
(3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring.

In a preferred example, W is selected from -O-;
L is C₁-C₆ alkylene;
V is absent or selected from: -NH-;
when V is absent, Q is ring A which is unsubstituted or substituted by m R₃, or ring B which is unsubstituted or substituted by m R₃;
when V is selected from: -NH-, Q is ring C which is unsubstituted or substituted by m R3;
the ring A is a 6- to 15-membered heterocyclyl ring; and when ring A is a 6-membered heterocyclyl ring, the ring A is 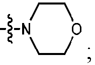
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
n is 1, 2, or 3; when there is more than one R₁, R₁ groups are the same or different;
R₁ is hydrogen, halogen, cyano, unsubstituted C₁-C₆ alkyl, or unsubstituted C₂-C₆ alkynyl;
p is 1, 2, or 3; when there is more than one R₂, R₂ groups are the same or different;
R₂ is hydrogen or halogen;
m is 1, 2, or 3; when there is more than one R₃, R₃ groups are the same or different;
R₃ is C₁-C₆ alkyl or -CO-C₁-C₆ alkyl; R₃ is optionally substituted by one or more than one 3- to 6-membered cycloalkyl; when there is more than one substituent, the substituents are the same or different;
the diaryl compound of formula I satisfies 1, 2, or 3 of the following conditions:
   (1) V is selected from -NH-;
   (2) Q is a 6- to 12-membered sulfonyl-containing heterocyclyl ring which is unsubstituted or substituted by m R₃;
   (3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring.

In a preferred example, W is selected from -O-;
L is C₁-C₆ alkylene;
V is absent or selected from: -NH-;
when V is absent, Q is ring A which is unsubstituted or substituted by m R₃, or ring B which is unsubstituted or substituted by m R₃;
when V is selected from: -NH-, Q is ring C which is unsubstituted or substituted by m R₃;
the ring A is a 6- to 15-membered heterocyclyl ring; and when ring A is a 6-membered heterocyclyl ring, the ring A is 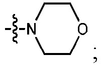
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
n is 1;
R₁ is hydrogen, halogen, cyano, unsubstituted C₁-C₆ alkyl, or unsubstituted C₂-C₆ alkynyl;
pis 1;
R₂ is hydrogen;
m is 1, 2, or 3; when there is more than one R₃, R₃ groups are the same or different;
R₃ is C₁-C₆ alkyl or -CO-C₁-C₆ alkyl; R₃ is optionally substituted by one or more than one 3- to 6-membered cycloalkyl; when there is more than one substituent, the substituents are the same or different;
the diaryl compound of formula I satisfies 1, 2, or 3 of the following conditions:
   (1) V is selected from -NH-;
   (2) Q is a 6- to 12-membered sulfonyl-containing heterocyclyl ring which is unsubstituted or substituted by m R₃;
   (3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring.

In a preferred example, L is -CH₂-, -CH₂CH₂-, -CH₂CH₂CH₂-; preferably, L is-CH₂CH₂-.

In a preferred example, in the ring A, the 6- to 15-membered heterocyclyl ring is a 6-to 8-membered heterocyclyl ring.

In a preferred example, in the ring A, the 6- to 15-membered heterocyclyl ring is a monocyclic ring, a fused ring, a bridged ring, or a spiro ring.

In a preferred example, in the ring A, the 6- to 15-membered heterocyclyl ring is a saturated ring.

In a preferred example, in the ring A, the heteroatoms in the 6- to 15-membered heterocyclyl ring are N and/or O.

In a preferred example, in the ring A, the heteroatoms in the 6- to 15-membered heterocyclyl ring are N and O.

In a preferred example, in the ring A, the 6- to 15-membered heterocyclyl ring is connected to V through an N atom.

In a preferred example, the ring A is 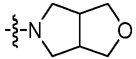 (for example,

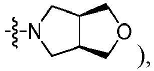

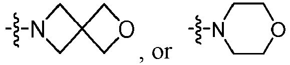

In a preferred example, in the ring C, the 4- to 15-membered heterocyclyl ring is a 6-to 8-membered heterocyclyl ring.

In a preferred example, in the ring C, the 4- to 15-membered heterocyclyl ring is a monocyclic ring, a fused ring, a bridged ring, or a spiro ring.

In a preferred example, in the ring C, the 4- to 15-membered heterocyclyl ring is a saturated ring.

In a preferred example, in the ring C, the heteroatoms in the 4- to 15-membered heterocyclyl ring are N and/or O.

In a preferred example, in the ring C, the heteroatoms in the 4- to 15-membered heterocyclyl ring are N and O.

In a preferred example, in the ring C, the 4- to 15-membered heterocyclyl ring is connected to V through an N atom.

In a preferred example, in the ring C, the 4- to 15-membered heterocyclyl ring is 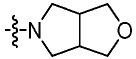 (for example,

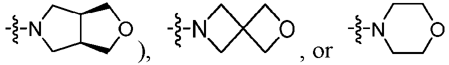

In a preferred example, in the ring B, the 6- to 12-membered sulfonyl-containing heterocyclyl ring is a 6- to 8-membered sulfonyl-containing heterocyclyl ring.

In a preferred example, in the ring B, the 6- to 12-membered sulfonyl-containing heterocyclyl ring is a monocyclic ring, a fused ring, a bridged ring, or a spiro ring.

In a preferred example, in the ring B, the 6- to 12-membered sulfonyl-containing heterocyclyl ring is a monocyclic ring or a spiro ring.

In a preferred example, in the ring B, the 6- to 12-membered sulfonyl-containing heterocyclyl ring is a saturated ring.

In a preferred example, in the ring B, the heteroatoms in the 6- to 12-membered sulfonyl-containing heterocyclyl ring are -S(=O)₂- and/or N.

In a preferred example, in the ring B, the heteroatoms in the 6- to 12-membered sulfonyl-containing heterocyclyl ring are -S(=O)₂- and N.

In a preferred example, in the ring B, the 6- to 12-membered sulfonyl-containing heterocyclyl ring is connected to V through an N atom.

In a preferred example, in the ring B, the 6- to 12-membered sulfonyl-containing heterocyclyl ring is

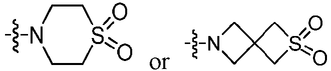

In a preferred example, in the ring C, the 6- to 12-membered sulfonyl-containing heterocyclyl ring is a 6- to 8-membered sulfonyl-containing heterocyclyl ring.

In a preferred example, in the ring C, the 6- to 12-membered sulfonyl-containing heterocyclyl ring is a monocyclic ring, a fused ring, a bridged ring, or a spiro ring.

In a preferred example, in the ring C, the 6- to 12-membered sulfonyl-containing heterocyclyl ring is a monocyclic ring or a spiro ring.

In a preferred example, in the ring C, the 6- to 12-membered sulfonyl-containing heterocyclyl ring is a saturated ring.

In a preferred example, in the ring C, the heteroatoms in the 6- to 12-membered sulfonyl-containing heterocyclyl ring are -S(=O)₂- and/or N.

In a preferred example, in the ring C, the heteroatoms in the 6- to 12-membered sulfonyl-containing heterocyclyl ring are -S(=O)₂- and N.

In a preferred example, in the ring C, the 6- to 12-membered sulfonyl-containing heterocyclyl ring is connected to V through an N atom.

In a preferred example, in the ring C, the 6- to 12-membered sulfonyl-containing heterocyclyl ring is

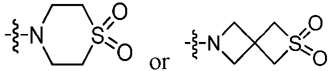

In a preferred example, in the R₁, the halogen is fluorine or chlorine.

In a preferred example, in the R₁, the C₁-C₆ alkyl is methyl or ethyl.

In a preferred example, in the R₁, the C₂-C₆ alkynyl is ethynyl or propynyl.

In a preferred example, in the R₂, the halogen is fluorine or chlorine.

In a preferred example, in the R₃, the C₁-C₆ alkyl is methyl or ethyl.

In a preferred example, in the R₃, the C₁-C₆ alkyl in the "-CO-C₁-C₆ alkyl" is methyl or ethyl.

In a preferred example, the 4- to 6-membered ring formed by R₁₁ and R₁₂ together with the N atom to which they are attached may be a 4- to 6-membered N-containing heterocycloalkyl ring or a 5- to 6-membered heteroaryl ring.

In a preferred example, W is selected from: -O-, -S-, -NH-; preferably, W is -O-.

In a preferred example, L is -CH₂-, -CH₂CH₂-, -CH₂CH₂CH₂-; preferably, L is-CH₂CH₂-.

In a preferred example, V is absent or -NH-.

In a preferred example, the ring A is a 7- to 15-membered monocyclic, fused, bridged, or spiro heterocyclyl ring;
preferably, the ring A comprises 1, 2, or 3 heteroatoms selected from N, O, or S; when there is more than one heteroatom, the heteroatoms are the same or different; preferably, the ring A comprises 1 N atom and 1 O atom.

In a preferred example, the ring A has a structure of 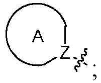 wherein Z represents C or N; preferably, Z is N; preferably, the ring A further comprises 1 O atom; preferably,

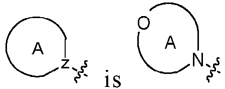

In a preferred example, the ring B is a 6- to 12-membered sulfonyl-containing monocyclic, fused, spiro, or bridged heterocyclyl ring; preferably, ring B is a 6- to 8-membered sulfonyl-containing monocyclic heterocyclyl ring; preferably, ring B is a 7- to 12-membered sulfonyl-containing fused, spiro, or bridged heterocyclyl ring.

In a preferred example, ring B is 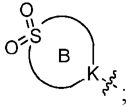 wherein K represents C or N.

In a preferred example, the diaryl compound, the tautomer, the stereoisomer, the solvate (such as hydrate), the pharmaceutically acceptable salt, or the prodrug thereof has a structure of formula Ia, Ib, or Ic: 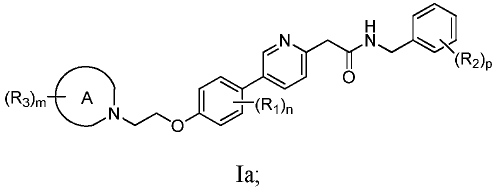 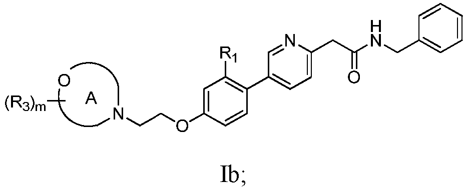 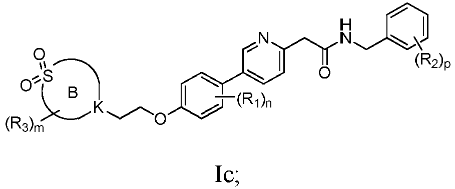
wherein K represents C or N;
ring A, ring B, m, n, p, R₁, R₂, and R₃ are as defined in the first aspect of the present disclosure.

In a preferred example, 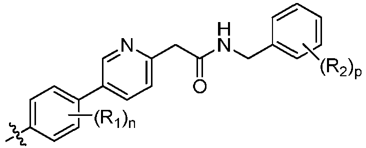 has a structure of

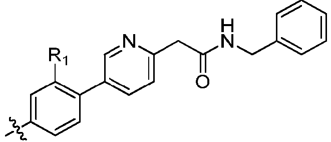

In a preferred example, the ring A is a 7- to 10-membered monocyclic heterocyclyl ring or a 7- to 12-membered fused, bridged, or spiro heterocyclyl ring.

In a preferred example, the ring A is selected from: 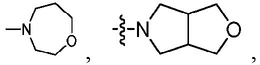 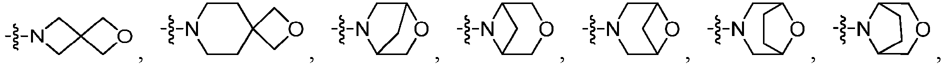 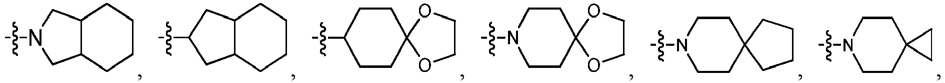 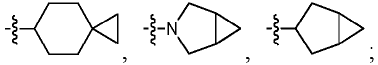 preferably,

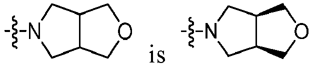

In a preferred example, R₁ and R₂ are each independently hydrogen or selected from: halogen, hydroxyl, amino, C₁-C₆ alkyl, -O-C₁-C₆ alkyl; the C₁-C₆ alkyl is optionally substituted by one or more than one halogen; R₃ is selected from: halogen, hydroxyl, amino, C₁-C₆ alkyl, -O-C₁-C₆ alkyl; the C₁-C₆ alkyl is optionally substituted by one or more than one halogen; and R₁ and R₂ are not hydrogen at the same time;
preferably, R₁, R₂, and R₃ are each independently selected from: halogen, C₁-C₆ alkyl; the C₁-C₆ alkyl is optionally substituted by one or more than one halogen;
more preferably, R₁, R₂, and R₃ are each independently selected from: fluorine, methyl, ethyl, propyl; the methyl, ethyl, and propyl are optionally substituted by one or more than one halogen;
preferably, the halogen is F.

In a preferred example, ring B is a 6- to 12-membered monocyclic, fused, spiro, or bridged heterocyclyl ring; preferably, ring B is a 6- to 8-membered monocyclic heterocyclyl ring; preferably, ring B is a 7- to 12-membered fused, spiro, or bridged heterocyclyl ring.

In a preferred example, the ring B is selected from:

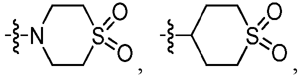

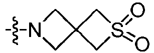

In a preferred example, R₁, R₂, and R₃ are each independently hydrogen or selected from: halogen, hydroxyl, amino, cyano, C₁-C₆ alkyl, -O-C₁-C₆ alkyl, -C(O)NR₁₁R₁₂; R₁₁ and R₁₂ are each independently hydrogen or C₁-C₆ alkyl;
the C₁-C₆ alkyl is optionally substituted by one or more than one halogen;
preferably, R₁, R₂, and R₃ are each independently selected from: halogen, C₁-C₆ alkyl; the C₁-C₆ alkyl is optionally substituted by one or more than one halogen;
more preferably, R₁, R₂, and R₃ are each independently selected from: fluorine, methyl, ethyl, propyl; the C₁-C₆ alkyl is optionally substituted by one or more than one fluorine; preferably, the halogen is F.

In a preferred example, n is 1.

In a preferred example, R₁ is hydrogen or selected from: halogen, hydroxyl, amino, cyano, C₁-C₆ alkyl, C₂-C₆ alkenyl, C₂-C₆ alkynyl;
preferably, R₁ is hydrogen or selected from: halogen, cyano, C₁-C₆ alkyl, C₂-C₆ alkynyl;
more preferably, R₁ is hydrogen or selected from: fluorine, chlorine, cyano, methyl,-C≡C-CH₃.

In a preferred example, R₂ is hydrogen.

In a preferred example, R₃ is hydrogen.

In a preferred example, the diaryl compound, the tautomer, the stereoisomer, the solvate (such as hydrate), the pharmaceutically acceptable salt, or the prodrug thereof has a structure of Id: 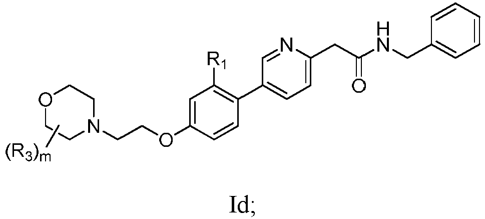
wherein m is 1 or 2; preferably, m is 1;
R₃ is hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₆ alkenyl, C₂-C₆ alkynyl, -C(O)NR₁₁R₁₂;
or R₁ and R₃ are each independently selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl; when there is more than one substituent, the substituents are the same or different.

In a preferred example, R₃ is hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₃ alkenyl, C₂-C₃ alkynyl,-C(O)NR₁₁R₁₂;
or R₁ and R₃ are each independently selected from: halogen, C₁-C₆ alkyl; the C₁-C₆ alkyl is optionally substituted by one or more than one substituent selected from the following: halogen, hydroxyl, amino, cyano, C₁-C₆ alkyl; when there is more than one substituent, the substituents are the same or different;
preferably, R₃ is hydrogen or selected from: halogen, methyl, ethyl, propyl; the methyl, ethyl, and propyl are optionally substituted by one or more than one halogen; R₁ is selected from: cyano, C₂-C₃ alkenyl, C₂-C₃ alkynyl; more preferably, R₃ is hydrogen; R₁ is selected from: cyano, C₂-C₃ alkenyl, C₂-C₃ alkynyl;
preferably, R₁ and R₃ are each independently selected from: halogen, methyl, ethyl, propyl; the methyl, ethyl, and propyl are optionally substituted by one or more than one halogen; preferably, the halogen is fluorine.

In a preferred example, the diaryl compound, the tautomer, the stereoisomer, the solvate (such as hydrate), the pharmaceutically acceptable salt, or the prodrug thereof has a structure of Ie or If: 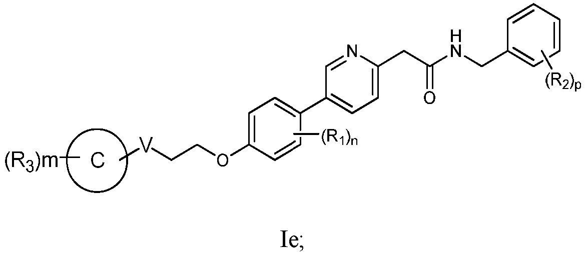 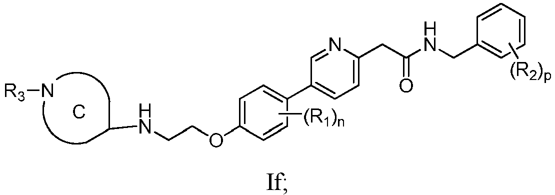
wherein ring C, R₁, R₂, n, and p are as defined in the first aspect of the present disclosure;
R₃ is C₁-C₆ alkyl, -CO-C₁-C₆ alkyl, -C(O)NR₁₁R₁₂, 3- to 6-membered cycloalkyl,-COO-C₁-C₆ alkyl, -COO-3- to 6-membered cycloalkyl, -CO-3- to 6-membered cycloalkyl,-S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
preferably, ring C is a 4- to 8-membered heterocyclyl ring; preferably, ring C is a 4-to 6-membered heterocyclyl ring; more preferably, ring C is a 4-membered ring;
preferably, R₃ is -CO-C₁-C₆ alkyl;
preferably, the R₃ is optionally substituted by one or more than one substituent selected from the following: halogen, 3- to 6-membered cycloalkyl;
more preferably, R₃ is -CO-C₁-C₃ alkyl-cyclopropyl, -CO-C₁-C₃ alkyl-cyclobutyl.

In a preferred embodiment, in the diaryl compound of formula I, Ia, Ib, Ic, Id, Ie, or If, R₁ is hydrogen or selected from: halogen, hydroxyl, amino, cyano, C₁-C₆ alkyl, C₂-C₆ alkenyl, C₂-C₆ alkynyl;
preferably, R₁ is hydrogen or selected from: halogen, cyano, C₁-C₆ alkyl, C₂-C₆ alkynyl;
more preferably, R₁ is hydrogen or selected from: fluorine, chlorine, cyano, methyl,-C≡C-CH₃.

In a preferred example, in the diaryl compound, the tautomer, the stereoisomer, the solvate (such as hydrate), the pharmaceutically acceptable salt, or the prodrug thereof, the diaryl compound is any one of the following compounds:

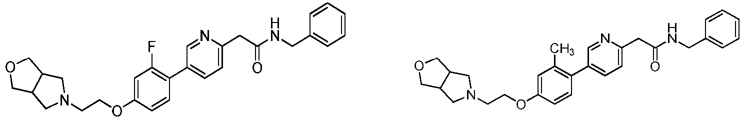

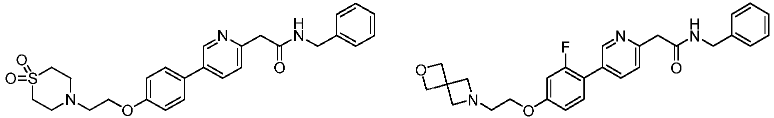

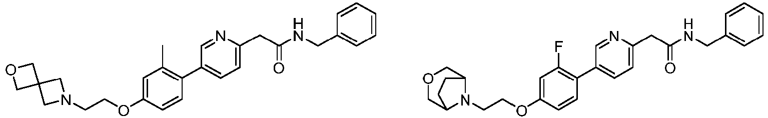

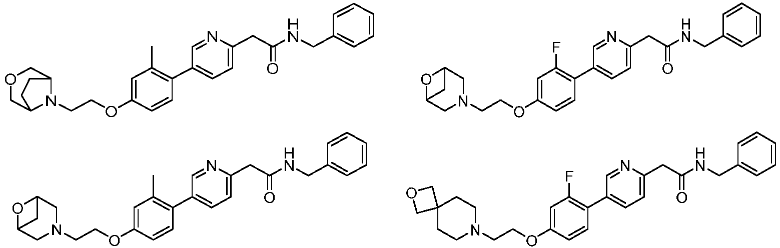

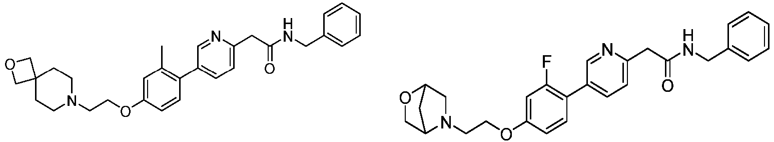

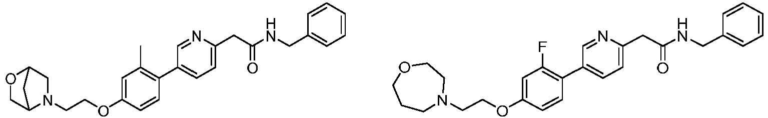

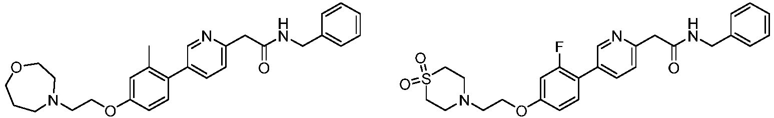

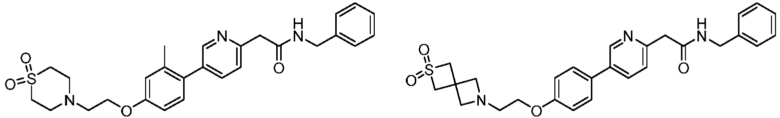

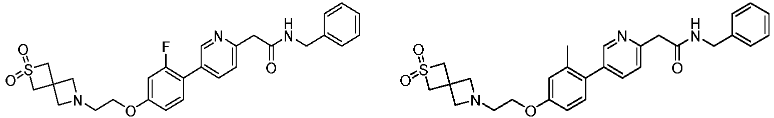

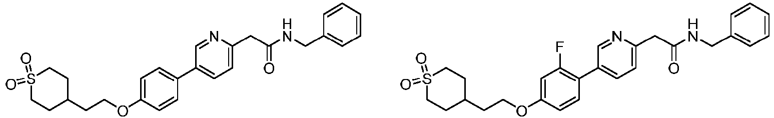

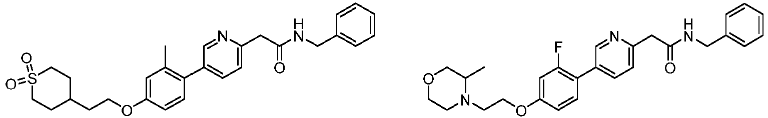

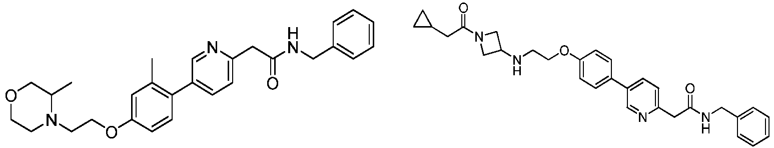

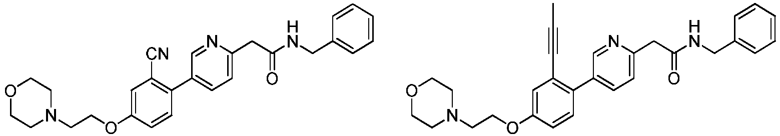

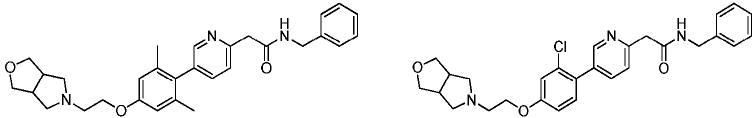

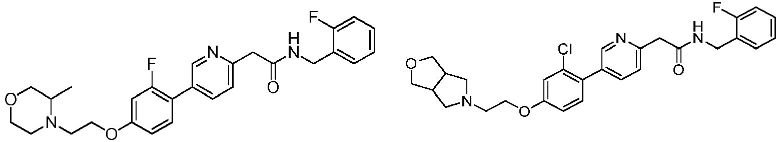

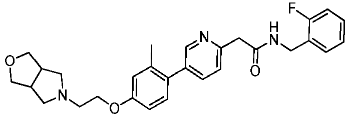

In a preferred example, in the diaryl compound, the tautomer, the stereoisomer, the solvate (such as hydrate), the pharmaceutically acceptable salt, or the prodrug thereof, the diaryl compound is any one of the following compounds: 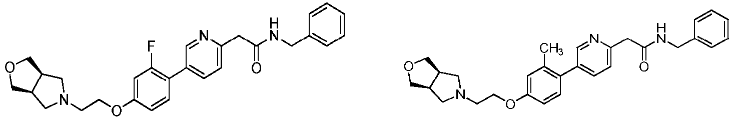 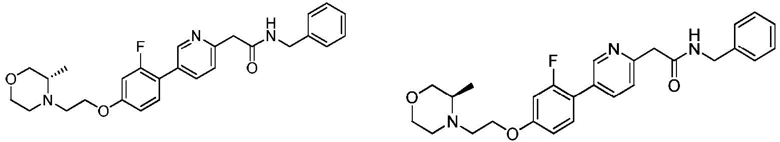 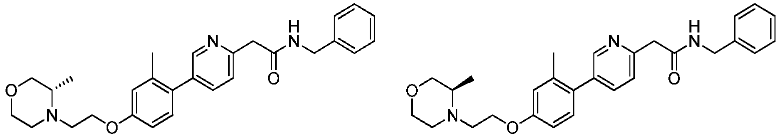 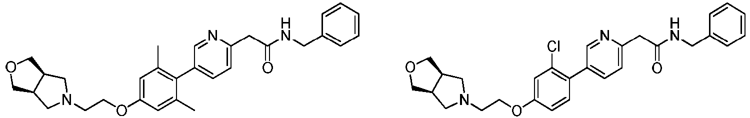 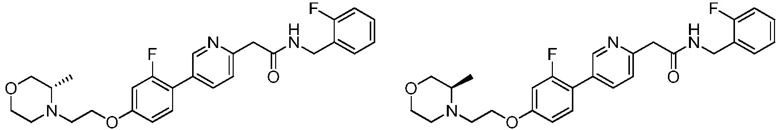 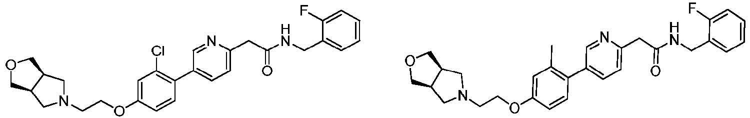
preferably, 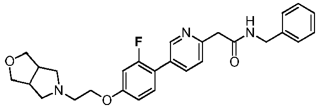 is 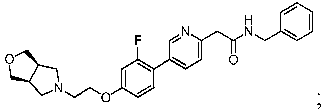
preferably, 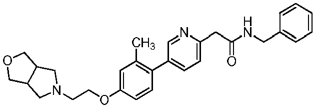 is

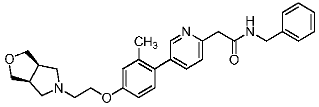

In a second aspect of the present disclosure, provided is a compound B having a structure of 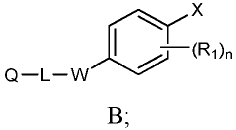
wherein Q, L, W, R₁, and n are as defined in the first aspect;
X is halogen or substituent G;
the substituent G is selected from: borate group, boronic acid group, alkyltin, trifluoromethanesulfonic acid group, methanesulfonic acid group, *p*-toluenesulfonic acid group;
preferably, the halogen is chlorine, bromine, or iodine.

In a preferred example, the compound B has the following structures: 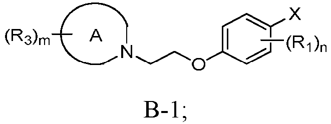 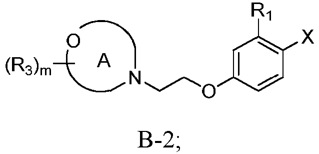 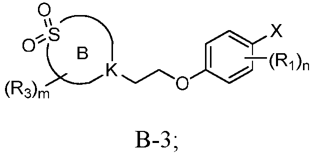 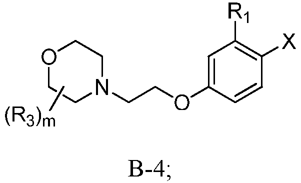 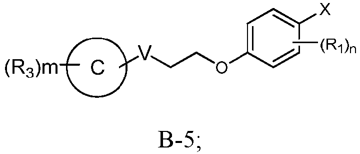 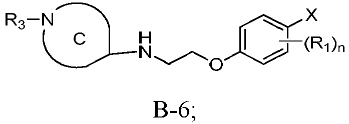
wherein K represents C or N;
ring A, ring B, m, n, p, R₁, R₂, and R₃ are as defined in the first aspect;
X is halogen or substituent G;

In a preferred example, the substituent G is selected from: borate group, boronic acid group, alkyltin, trifluoromethanesulfonic acid group, methanesulfonic acid group, *p-*toluenesulfonic acid group;
preferably, the halogen is chlorine, bromine, or iodine.

In a second aspect of the present disclosure, provided is an intermediate B having a structure of 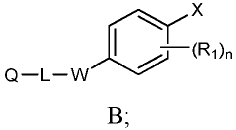
wherein Q, L, W, R₁, and n are as defined in the first aspect;
X is halogen or substituent G;
the substituent G is selected from: borate group, boronic acid group, alkyltin, trifluoromethanesulfonic acid group, methanesulfonic acid group, *p*-toluenesulfonic acid group;
preferably, the halogen is chlorine, bromine, or iodine.

In a preferred example, the intermediate B has the following structures: 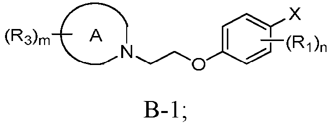 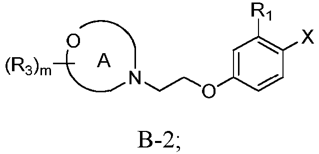 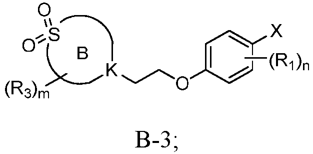 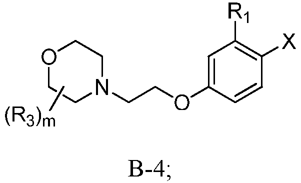 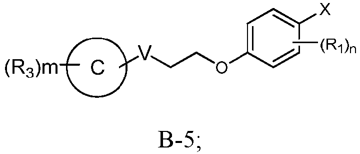 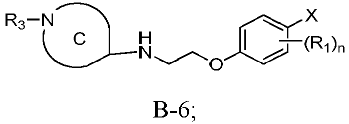
wherein K represents C or N;
ring A, ring B, m, n, p, R₁, R₂, and R₃ are as defined in the first aspect;
X is halogen or substituent G;

In a preferred example, the substituent G is selected from: borate group, boronic acid group, alkyltin, trifluoromethanesulfonic acid group, methanesulfonic acid group, *p-*toluenesulfonic acid group;
preferably, the halogen is chlorine, bromine, or iodine.

In a preferred example, the ring A is a 7- to 15-membered monocyclic, fused, bridged, or spiro heterocyclyl ring;
preferably, the ring A is a 7- to 10-membered monocyclic heterocyclyl ring or a 7- to 12-membered fused, bridged, or spiro heterocyclyl ring;
preferably, the ring A comprises 1 N atom and 1 O atom.

In a preferred embodiment, the borate group is selected from: bis(pinacolato)diboron, bis(catecholato)diboron, bis(3,3-dimethylpentane-2,4-glycolato)diboron, triethanolamine borate, trimethyl borate, triisopropyl borate, triethyl borate, tributyl borate, bis(neopentyl glycolato)diboron.

In a preferred example, ring B is a 6- to 12-membered monocyclic, fused, spiro, or bridged heterocyclyl ring; preferably, ring B is a 6- to 8-membered monocyclic heterocyclyl ring; preferably, ring B is a 7- to 12-membered fused, spiro, or bridged heterocyclyl ring.

In a preferred example, the alkyltin is butyltin, isopropyltin, or propyltin.

In a preferred example, R₁, R₂, and R₃ are each independently selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl; when there is more than one substituent, the substituents are the same or different.

In a preferred example, R₃ is hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₆ alkenyl, C₂-C₆ alkynyl,-C(O)NR₁₁R₁₂.
preferably, R₃ is hydrogen or selected from: halogen, methyl, ethyl, propyl; the methyl, ethyl, and propyl are optionally substituted by one or more than one halogen; R₁ is selected from: cyano, C₂-C₃ alkenyl, C₂-C₃ alkynyl;

In a preferred example, R₁, R₂, and R₃ are each independently selected from: halogen, C₁-C₆ alkyl; the C₁-C₆ alkyl is optionally substituted by one or more than one substituent selected from the following: halogen, hydroxyl, amino, cyano, C₁-C₆ alkyl; when there is more than one substituent, the substituents are the same or different;
preferably, R₁ and R₃ are each independently selected from: halogen, methyl, ethyl, propyl; the methyl, ethyl, and propyl are optionally substituted by one or more than one halogen; preferably, the halogen is fluorine.

In a preferred example, R₃ is C₁-C₆ alkyl, -CO-C₁-C₆ alkyl, -C(O)NR₁₁R₁₂, 3- to 6-membered cycloalkyl, -COO-C₁-C₆ alkyl, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂; wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl, 3- to 6-membered cycloalkyl; when there is more than one substituent, the substituents are the same or different.
preferably, ring C is a 4- to 8-membered heterocyclyl ring; preferably, ring C is a 4-to 6-membered heterocyclyl ring; more preferably, 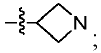
preferably, R₃ is -CO-C₁-C₆ alkyl; preferably, the R₃ is optionally substituted by one or more than one substituent selected from the following: halogen, 3- to 6-membered cycloalkyl;
more preferably, R₃ is -CO-C₁-C₃ alkyl-cyclopropyl, -CO-C₁-C₃ alkyl-cyclobutyl.

A compound having any one of the following structures:

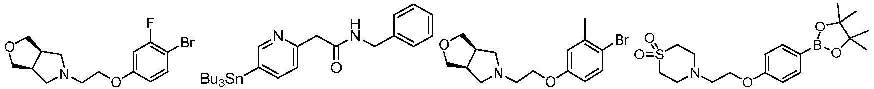

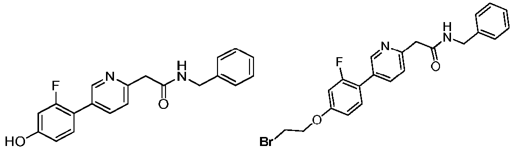

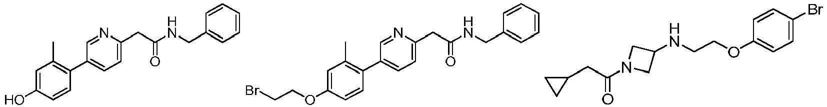

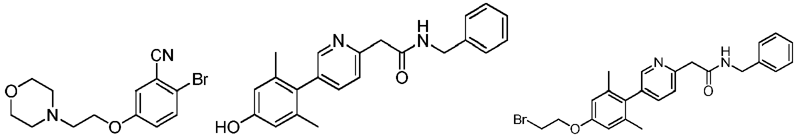

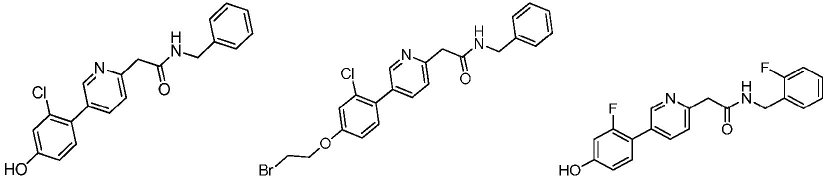

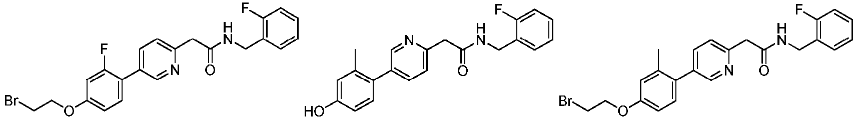

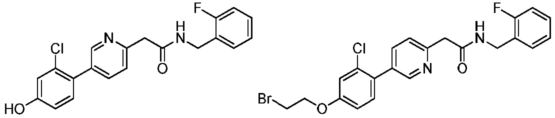

In a third aspect of the present disclosure, provided is a method for preparing the diaryl compound, the tautomer, the stereoisomer, the solvate (such as hydrate), the pharmaceutically acceptable salt, or the prodrug thereof as described in any one of the examples in the first aspect, comprising:
obtaining the diaryl compound by reacting the intermediate B as described in any one of the examples in the second aspect with an intermediate C under an alkaline condition 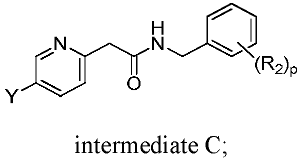
wherein R₂ and p are as defined in the first aspect;
Y is halogen or substituent G;
the substituent G is selected from: borate group, boronic acid group, alkyltin, trifluoromethanesulfonic acid group, methanesulfonic acid group, *p*-toluenesulfonic acid group;
when X in intermediate B is halogen, Y is G;
when X in intermediate B is G, X is halogen;
preferably, the halogen is chlorine, bromine, or iodine;
preferably, intermediate C has a structure of

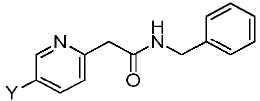

In a preferred embodiment, the borate group is selected from: bis(pinacolato)diboron, bis(catecholato)diboron, bis(3,3-dimethylpentane-2,4-glycolato)diboron, triethanolamine borate, trimethyl borate, triisopropyl borate, triethyl borate, tributyl borate, bis(neopentyl glycol ato)dib or on.

In a preferred example, the alkyltin is butyltin, isopropyltin, or propyltin.

In a preferred example, the reaction requires the protection of an inert gas, and the inert gas includes, but is not limited to: nitrogen, helium, neon, and argon.

In a preferred example, the reaction is carried out in the presence of a palladium catalyst; preferably, the palladium catalyst is selected from:

The reaction of each step of the present disclosure is preferably carried out in an inert solvent, and the inert solvent includes, but is not limited to: toluene, benzene, water, methanol, ethanol, isopropanol, ethylene glycol, N methylpyrrolidone, dimethyl sulfoxide, tetrahydrofuran, dichloromethane, trichloromethane, 1,2-dichloroethane, acetonitrile, *N,N-*dimethylformamide, *N,N*-dimethylacetamide, dioxane, or combinations thereof.

Preferably, the palladium catalyst is selected from the group consisting of: tris(dibenzylideneacetone)dipalladium (Pd₂(dba)₃), tetrakis(triphenylphosphine)palladium (Pd(PPh₃)₄), palladium acetate, palladium chloride, bis(triphenylphosphine)palladium(II) dichloride, palladium trifluoroacetate, bis(triphenylphosphinepalladium) acetate, [1,1'-bis(diphenylphosphino)ferrocene]dichloropalladium(II), bis(tri-o-tolylphosphine)palladium(II) dichloride, [1,2-bis(diphenylphosphino)ethane]dichloropalladium(II), or combinations thereof.

Preferably, the borate group is selected from the group consisting of: bis(pinacolato)diboron, bis(catecholato)diboron, bis(3,3-dimethylpentane-2,4-glycolato)diboron, triethanolamine borate, trimethyl borate, triisopropyl borate, triethyl borate, tributyl borate, bis(neopentyl glycolato)diboron.

In a fourth aspect of the present disclosure, provided is a pharmaceutical composition comprising the diaryl compound, the tautomer, the stereoisomer, the solvate (such as hydrate), the pharmaceutically acceptable salt, or the prodrug thereof as described in the first aspect.

Provided is a pharmaceutical composition comprising the diaryl compound, the tautomer, the stereoisomer, the solvate (such as hydrate), the pharmaceutically acceptable salt, or the prodrug thereof as described in the first aspect, and a pharmaceutically acceptable carrier and/or other active drug.

In a fifth aspect of the present disclosure, provided is a use of the diaryl compound, the tautomer, the stereoisomer, the solvate (such as hydrate), the pharmaceutically acceptable salt, or the prodrug thereof as described in the first aspect, or a use of the pharmaceutical composition as described in the fourth aspect, the use comprising:
1) inhibiting tubulin polymerization and/or Src kinase;
2) preventing and/or treating a disease related to tubulin polymerization and/or Src kinase;
3) preparing a tubulin polymerization and/or Src kinase inhibitor;
4) preparing a drug, pharmaceutical composition, or formulation for preventing and/or treating a disease related to tubulin polymerization and/or Src kinase.

Preferably, the use comprises: inhibiting tubulin polymerization; and/or, preventing and/or treating a disease mediated by tubulin polymerization; and/or, preparing a tubulin polymerization inhibitor, and/or a drug, pharmaceutical composition, or formulation for preventing and/or treating a disease mediated by tubulin polymerization.

Preferably, the use comprises: inhibiting Src kinase; and/or, preventing and/or treating a disease mediated by Src kinase; and/or, preparing a Src kinase inhibitor; and/or, preparing a drug, pharmaceutical composition, or formulation for preventing and/or treating a disease mediated by Src kinase.

Preferably, the drug is an external preparation.

Preferably, the drug is transdermally administered.

Preferably, the disease related to tubulin polymerization and/or Src kinase comprises tumor and skin disease.

In a sixth aspect of the present disclosure, provided is a use of the diaryl compound, the tautomer, the stereoisomer, the solvate (such as hydrate), the pharmaceutically acceptable salt, or the prodrug thereof as described in the first aspect in the preparation of a drug for the treatment of tumor and/or skin disease; or a use of the pharmaceutical composition as described in the fourth aspect in the preparation of a drug for the treatment of tumor and/or skin disease.

Preferably, the drug is an external preparation.

Preferably, the drug is transdermally administered.

In a seventh aspect of the present disclosure, provided is a use of at least one of the diaryl compound, the tautomer, the stereoisomer, the solvate (such as hydrate), the pharmaceutically acceptable salt, or the prodrug thereof as described in the first aspect of the present disclosure or the pharmaceutical composition as described in the fourth aspect of the present disclosure for treating or preventing tumor and/or skin disease.

In a preferred embodiment, the tumor comprises: solid tumor, sarcoma, and hematological cancer;

Preferably, the tumor comprises: breast cancer, ovarian cancer, prostate cancer, cervical cancer, testicular cancer, colon cancer, colorectal cancer, liver cancer, non-small cell lung cancer, squamous cell carcinoma (such as cutaneous squamous cell carcinoma), small cell lung cancer, gastric cancer, gastrointestinal stromal tumor, pancreatic cancer, bladder cancer, germ cell tumor, mast cell tumor, mastocytosis, glioblastoma, neuroblastoma, astrocytoma, melanoma, B-cell lymphoma, T-cell lymphoma, slowly progressive lymphoma, Hodgkin lymphoma, non-Hodgkin lymphoma, acute myeloid leukemia, acute lymphoblastic leukemia, chronic myeloid leukemia, chronic lymphocytic leukemia, myeloma, and/or myelodysplastic syndrome.

In a preferred embodiment, the skin disease comprises: actinic keratosis, psoriasis, atopic dermatitis, psoriasis, vitiligo, roseola, and/or systemic lupus erythematosus.

In an eighth aspect of the present disclosure, provided is a method for inhibiting Src kinase, or preventing and/or treating a disease related to (or mediated by) Src kinase, comprising the steps of: administering to a subject in need thereof the compound, the tautomer, the stereoisomer, the solvate (such as hydrate), the pharmaceutically acceptable salt, or the prodrug thereof as described in the first aspect of the present disclosure, or the pharmaceutical composition as described in the fourth aspect of the present disclosure.

In a ninth aspect of the present disclosure, provided is a method for inhibiting tubulin, or preventing and/or treating a disease related to (or mediated by) tubulin, comprising the steps of: administering to a subject in need thereof the compound, the tautomer, the stereoisomer, the solvate (such as hydrate), the pharmaceutically acceptable salt, or the prodrug thereof as described in the first aspect of the present disclosure, or the pharmaceutical composition as described in the fourth aspect of the present disclosure.

The additional aspects and advantages of the present disclosure will be partly given in the following description, and part of them will become apparent from the following description, or can be understood through the implementation of the present disclosure.

### Term definitions and explanations

Unless otherwise specified, the definitions of groups and terms described in the description and claims include definitions thereof as examples, exemplary definitions, preferred definitions, definitions recorded in tables, and definitions of specific compounds in the examples, *etc.,* which can be arbitrarily combined and integrated with each other. Such combined and integrated definitions of groups and compound structures should fall within the scope of the description of the present disclosure.

Unless otherwise defined, all scientific and technological terms of the present disclosure have the same meanings as those commonly understood by those skilled in the art to which the subject matter of the claims belongs. Unless otherwise specified, all patents, patent applications, and published materials cited in the present disclosure are incorporated herein by reference in their entirety. If a term has multiple definitions in the present disclosure, the definitions in this section shall prevail.

It should be understood that the above brief description and the following detailed description are exemplary and for explanatory purposes only, and do not limit the subject matter of the present disclosure in any way. In the present disclosure, the use of the singular also includes the plural unless specifically stated otherwise. It must be noted that the singular form used in the present description and claims includes the plural form of the object referred to unless clearly stated otherwise. It should also be noted that the use of "or" and "alternatively" indicates "and/or" unless otherwise specified. Furthermore, the use of the term "comprising" as well as other forms, such as "comprises", "includes", and "contains", is not limiting.

Definitions of standard chemical terms can be found in references (including Carey and Sundberg "ADVANCED ORGANIC CHEMISTRY 4THED." Vols. A (2000) and B (2001), Plenum Press, New York). Unless otherwise specified, conventional methods in the technical scope of the art, such as mass spectrometry, NMR, IR, and UV/Vis spectroscopy, and pharmacological methods are used. Unless specific definitions are provided, the terms used herein in the relevant descriptions of analytical chemistry, synthetic organic chemistry, and pharmaceuticals and medicinal chemistry are known in the art. Standard techniques can be used in chemical synthesis, chemical analysis, drug preparation, formulation, and delivery, and treatment of patients. For example, reaction and purification can be carried out according to the manufacturer's instructions for use of the kit, or in a manner known in the art, or the description of the present disclosure. Generally, the above techniques and methods can be implemented according to the descriptions in a number of summary and more specific documents cited and discussed in the present disclosure according to conventional methods well-known in the art. In the present description, groups and substituents thereof can be selected by those skilled in the art to provide stable structural moieties and compounds.

When a substituent is described by a conventional chemical formula written from left to right, the substituent also includes the chemically equivalent substituent obtained when the structural formula is written from right to left. For example, CH₂O is equivalent to OCH₂. As used herein, 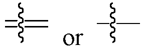 indicates the connection site of the group.

The section headings used herein are for the purpose of organizing the article only and should not be construed as limiting the subject matter described. All documents, or portions of documents, cited in the present disclosure, including but not limited to patents, patent applications, articles, books, manuals, and treatises, are incorporated herein by reference in their entirety.

In addition to the foregoing, when used in the description and claims of the present disclosure, the following terms have the meanings shown below unless otherwise specified.

When the numerical range described in the description and claims of the present disclosure is understood as "integers", it should be understood as recording the two endpoints of the range and all integers in the range. For example, an "integer from 1 to 6" should be understood as recording every integer of 1, 2, 3, 4, 5, and 6.

In the present disclosure, the term "halogen" alone or as part of another substituent refers to fluorine, chlorine, bromine, iodine.

"Sulfonyl" alone or as part of another substituent refers to a 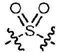 group.

As used herein, the term "alkyl" alone or as part of another substituent refers to a linear or branched hydrocarbon chain group consisting only of carbon atoms and hydrogen atoms, free of unsaturated bonds, having, for example, 1 to 6 carbon atoms, and connected to the rest of the molecule by a single bond. Examples of alkyl include, but are not limited to, methyl, ethyl, *n*-propyl, isopropyl, *n*-butyl, isobutyl, *tert*-butyl, pentyl, isopentyl, neopentyl, and hexyl. Alkyl may also be an isotopic isomer of naturally abundant alkyl rich in isotopes of carbon and/or hydrogen (*i.e*., deuterium or tritium).

As used herein, the term "alkenyl" refers to an unbranched or branched monovalent hydrocarbon chain that contains one or more than one carbon-carbon double bond.

As used herein, the term "alkynyl" refers to an unbranched or branched monovalent hydrocarbon chain that contains one or more than one carbon-carbon triple bond.

The term "C₁-C₆ alkyl", alone or as part of another substituent, should be understood to mean a linear or branched saturated monovalent hydrocarbon radical having 1, 2, 3, 4, 5, or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof. In particular, the group has 1, 2 or 3 carbon atoms ("C₁-C₃ alkyl"), such as methyl, ethyl, n-propyl, or isopropyl.

The term "alkylene" alone or as part of another substituent refers to a saturated divalent hydrocarbon group obtained by removing two hydrogen atoms from a saturated linear or branched hydrocarbon group. Examples of alkylene groups include methylene (-CH₂-), ethylene (including -CH₂CH₂- or -CH(CH₃)-), isopropylene (including -CH(CH₃)CH₂- or-C(CH₃)₂-), *etc.*

The term "cycloalkyl" or "carbocyclyl" alone or as part of another substituent refers to a cyclic alkyl group. The term "m- to n-membered cycloalkyl" or "Cₘ-Cₙ cycloalkyl" should be understood to mean a saturated, unsaturated, or partially saturated carbocyclic ring having m to n atoms. For example, "3- to 15-membered cycloalkyl" or "C₃-C₁₅ cycloalkyl" refers to a cyclic alkyl group containing 3 to 15, 3 to 9, 3 to 6, or 3 to 5 carbon atoms, which may contain 1 to 4 rings. "5- to 8-membered cycloalkyl" contains 5 to 8 carbon atoms. It includes a monocyclic, bicyclic, tricyclic, spiro, or bridged ring. Examples of unsubstituted cycloalkyl include, but are not limited to, cyclopropyl, cyclobutyl, cyclopentyl, cyclohexyl, and adamantyl, or bicyclic hydrocarbyl such as decahydronaphthalene ring. Cycloalkyl may be substituted by one or more than one substituent. In some embodiments, cycloalkyl may be aryl- or heteroaryl-fused cycloalkyl. The term "C₃-C₆ cycloalkyl" should be understood to mean a saturated monovalent monocyclic or bicyclic hydrocarbon ring having 3 to 6 carbon atoms, including fused or bridged polycyclic systems. For example, cyclopropyl, cyclobutyl, cyclopentyl, cyclohexyl.

The term "haloalkyl" alone or as part of another substituent refers to a branched and linear saturated aliphatic hydrocarbon group having a specific number of carbon atoms and substituted by one or more than one halogen (e.g., -CvFw, where v = 1 to 3 and w = 1 to (2v + 1)). Examples of haloalkyl include, but are not limited to, trifluoromethyl, trichloromethyl, pentafluoroethyl, pentachloroethyl, 2,2,2-trifluoroethyl, heptafluoropropyl, and heptachloropropyl.

"m- to n-membered heterocyclyl", alone or as part of another substituent, should be understood to mean a saturated, unsaturated, or partially saturated monocyclic ring, fused ring (including bicyclic ring and tricyclic ring), spiro ring, or bridged ring having m to n atoms, preferably a saturated heterocyclic ring.

For example, "7- to 15-membered heterocyclyl" should be understood to mean a saturated, unsaturated, or partially saturated monocyclic ring, fused ring (including bicyclic ring and tricyclic ring), spiro ring, or bridged ring having 7 to 15 atoms, preferably a saturated heterocyclic ring. Herein, 1, 2, 3, 4, or 5 ring atoms are selected from N, O, and S. It should be understood that when the total number of S and O atoms in the heterocyclyl exceeds 1, these heteroatoms are not adjacent to each other. If the heterocyclyl is monocyclic, it must not be aromatic. Examples of heterocyclyl include, but are not limited to, piperidinyl, *N-*acetylpiperidinyl, *N*-methyl piperidinyl, *N*-formylpiperazinyl, *N*-methylsulfonylpiperazinyl, homopiperazinyl, piperazinyl, azetidinyl, oxetanyl, morpholinyl, tetrahydroisoquinolinyl, tetrahydroquinolinyl, dihydroindolyl, tetrahydropyranyl, dihydro-2*H*-pyranyl, tetrahydrofuryl, tetrahydrothiopyranyl, tetrahydrothiopyran-1-oxide, tetrahydrothiopyran-1,1-dioxide, 1*H-*pyridin-2-one, and 2,5-dioxoimidazolidinyl. "6- to 12-membered heterocyclyl" refers to a saturated, unsaturated, or partially saturated monocyclic ring, fused ring (including bicyclic ring and tricyclic ring), spiro ring, or bridged ring having 6 to 12 atoms.

The term "fused ring" refers to a cyclic hydrocarbon in which any two rings in a compound share two carbon atoms that are directly connected, and can be divided into bicyclic hydrocarbon, tricyclic hydrocarbon, tetracyclic hydrocarbon, *etc.* according to the number of constituent rings. Non-limiting examples include:

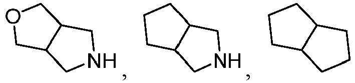

The term "spiro ring" refers to a polycyclic group that shares one carbon atom (called spiro atom) between monocyclic rings, which may contain one or more than one double bond, but none of the rings has a fully conjugated π-electron system. It is preferably 6- to 12-membered, more preferably 7- to 8-membered. Spirocycloalkyl can be divided into monospirocycloalkyl, bispirocycloalkyl, or polyspirocycloalkyl according to the number of spiro atoms shared between rings, preferably monospirocycloalkyl and bispirocycloalkyl. It is more preferably 4-membered/4-membered, 4-membered/5-membered, 4-membered/6-membered, 5-membered/5-membered, or 5-membered/6-membered monospirocycloalkyl. Non-limiting examples of spirocycloalkyl include:

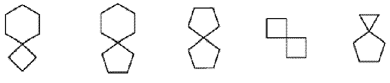

Spirocycloalkyl in which monospirocycloalkyl shares a spiro atom with heterocycloalkyl is also included. Non-limiting examples include:

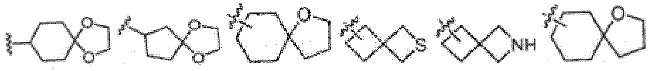

The term "bridged ring" refers to a cyclic hydrocarbon in which any two rings in a compound share two carbon atoms that are not directly connected, and can be divided into bicyclic hydrocarbon, tricyclic hydrocarbon, tetracyclic hydrocarbon, *etc.* according to the number of constituent rings. Non-limiting examples include:

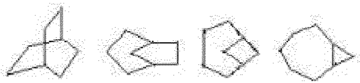

"C₂-C₆ alkenyl", alone or as part of another substituent, should be understood to mean a linear or branched monovalent hydrocarbon group containing one or more than one double bond and having 2, 3, 4, 5, or 6 carbon atoms, preferably for example, having 2 or 3 carbon atoms (*i.e.,* C₂-C₃ alkenyl). It should be understood that in the case where the alkenyl contains more than one double bond, the double bonds may be separated from each other or conjugated. The alkenyl is, for example, vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl.

The term "C₂-C₆ alkynyl", alone or as part of another substituent, should be understood to mean a linear or branched monovalent hydrocarbon group containing one or more than one triple bond and having 2, 3, 4, 5, or 6 carbon atoms, preferably for example, having 2 or 3 carbon atoms ("C₂-C₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl. In particular, the alkynyl is ethynyl, prop-1-ynyl, or prop-2-ynyl.

In examples of the present disclosure, protons may occupy two or more positions in the heterocyclic system in cyclic form, for example, 1*H*- and 3*H*-imidazole, 1*H-,* 2*H-,* and 4*H-*1,2,4-triazole, 1*H*- and 2*H*-isoindole, and 1*H*- and 2*H*-pyrazole. Tautomeric forms may be in equilibrium or sterically fixed to one form by appropriate substitutions. For example:

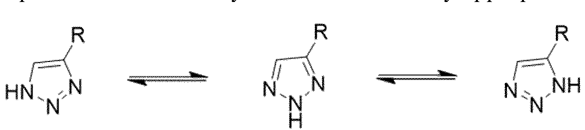

Due to resonance, the hydrogen of the nitrogen on triazole can be on any of the three nitrogens, so the naming will be different, but these three forms actually represent the same compound.

The compounds provided herein, including intermediates useful in the preparation of the compounds provided herein, contain reactive functional groups (such as, but not limited to, carboxyl, hydroxyl, and amino moieties), and also include protected derivatives thereof. "Protected derivatives" are those compounds in which one or more than one reactive site is blocked by one or more than one protecting group (also referred to as protective group). Suitable carboxyl protecting groups include benzyl, *tert*-butyl, *etc.,* as well as isotopes, *etc.* Suitable amino and amido protecting groups include acetyl, trifluoroacetyl, *tert-*butoxycarbonyl, benzyloxycarbonyl, *etc.* Suitable hydroxyl protecting groups include benzyl, *etc.* Other suitable protecting groups are well known to those skilled in the art.

In the present disclosure, "optional" or "optionally" means that the subsequently described event or circumstance may or may not occur, and the description includes both the occurrence and non-occurrence of the event or circumstance. For example, "optionally substituted aryl" means that the aryl is substituted or unsubstituted, and the description includes both substituted and unsubstituted aryl.

In the present disclosure, the term "salt" or "pharmaceutically acceptable salt" includes a pharmaceutically acceptable acid addition salt and a pharmaceutically acceptable base addition salt. The term "pharmaceutically acceptable" refers to that for those compounds, materials, compositions and/or dosage forms, they are suitable for use in contact with the tissues of human and animal without excess toxicity, irritation, allergic reactions, or other problems or complications within the scope of reliable medical judgment, and are commensurate with a reasonable benefit/risk ratio.

"Pharmaceutically acceptable acid addition salt" refers to a salt which retains the biological effectiveness of the free base without other side effects and is formed with an inorganic or organic acid. "Pharmaceutically acceptable base addition salt" refers to a salt which retains the biological effectiveness of the free acid without other side effects and is formed with an inorganic or organic base. In addition to the pharmaceutically acceptable salts, other salts may be adopted in the present disclosure. The other salts can serve as intermediates in the purification of compounds or in the preparation of other pharmaceutically acceptable salts or can be used for identifying, characterizing, or purifying the compounds of the present disclosure.

The term "amine salt" refers to the product obtained by neutralizing alkyl primary amine, secondary amine or tertiary amine with an acid. The acid includes the inorganic acid or the organic acid as described in the present disclosure.

The term "stereoisomer" refers to an isomer produced by a different spatial arrangement of atoms in the molecule, including *cis-trans* isomers, enantiomers, diastereomers, and conformational isomers.

Depending on the selected raw materials and methods, the compounds of the present disclosure may exist in the form of one of the possible isomers or a mixture thereof, for example, as pure optical isomers, or as a mixture of isomers such as a mixture of racemic isomer and diastereoisomer, depending on the number of asymmetric carbon atoms. When describing optically active compounds, the prefixes *D* and *L* or *R* and *S* are used to denote the absolute configurations of the molecule with respect to chiral center(s) in the molecule. The prefixes *D* and *L* or (+) and (-) are symbols used to specify a rotation of plane-polarized light caused by a compound, where (-) or *L* indicates that the compound is levorotatory. The prefix (+) or *D* indicates that the compound is dextrorotatory.

When the bond with a chiral carbon in the formula of the present disclosure is depicted in a straight line, it should be understood that the two configurations (*R*) and (*S*) of the chiral carbon and both the resulting enantiomerically pure compound and mixture are included in the scope defined by the general formula. The graphical representation of the racemically or enantiomerically pure compound herein is from Maehr, J. Chem. Ed. 1985, 62: 114-120. The absolute configuration of a stereocenter is represented by wedge-shaped bonds and dashed-line bonds.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. Prototropic tautomer is resulted from a migration of covalently bonded hydrogen atoms between two atoms. The tautomer generally exists in an equilibrium form, and when trying to separate a single tautomer, a mixture is usually produced, the physical and chemical properties of which are consistent with the mixture of compounds. The position of equilibrium depends on the intramolecular chemical properties. For example, for many aliphatic aldehydes and ketones, such as acetaldehyde, the ketonic form is dominant; and for phenols, the enol form is dominant. All tautomeric forms of the compounds are included in the present disclosure.

The term "pharmaceutical composition" of the present disclosure refers to a formulation of the compound of the present disclosure with a medium generally accepted in the art for delivering a biologically active compound to a mammal (*e.g*., a human). The medium includes a pharmaceutically acceptable carrier. The purpose of the pharmaceutical composition is to promote the administration to an organism, facilitate the absorption of the active ingredient and thus exert its biological activity.

In the present disclosure, "pharmaceutically acceptable carrier" includes, but is not limited to, any acceptable adjuvants, carriers, excipients, glidants, sweeteners, diluents, preservatives, dyes/coloring agents, flavoring agents, surfactants, wetting agents, dispersants, suspending agents, stabilizers, isotonic agents, solvents, or emulsifiers for humans or livestocks as licensed by relevant governmental administrations.

The term "solvate" refers to the compound of the present disclosure or a salt thereof including a stoichiometric or non-stoichiometric solvent bonded through an intermolecular non-covalent force. When the solvent is water, the solvate is a hydrate.

The term "prodrug" can be converted into the compound of the present disclosure having biological activity under physiological conditions or through solvolysis. The prodrug of the present disclosure is prepared by modifying the functional groups in the compound, and the modification can be removed by conventional operations or *in vivo,* so as to obtain the parent compound. The prodrug includes a compound formed by attaching a hydroxyl or amino group in the compound of the present disclosure to any group. When the prodrug of the compound of the present disclosure is administered to a mammal individual, the prodrug is dissociated to form a free hydroxyl group and a free amino group respectively.

The compound of the present disclosure may contain an unnatural proportion of atomic isotope at one or more than one atom(s) that constitute the compound. For example, the compound can be labeled with a radioactive isotope, such as deuterium (²H), tritium (³H), iodine -125(¹²⁵I), or C-14 (¹⁴C). All isotopic variations of the compound of the present disclosure, whether radioactive or not, are encompassed within the scope of the present disclosure.

The term "excipient" refers to a pharmaceutically acceptable inert ingredient. Examples of categories of the term "excipient" include, but are not limited to, binders, disintegrants, lubricants, glidants, stabilizers, fillers, diluents, *etc.* Excipients can enhance operation properties of the pharmaceutical formulation, *i.e.,* allowing the formulation to be more suitable for direct compression by increasing fluidity and/or adhesion.

As used herein, the term "treatment" and other similar synonyms include the following meanings:
(i) preventing the occurrence of a disease or condition in mammals, particularly when such mammals are susceptible to the disease or condition but have not been diagnosed as having the disease or condition;
(ii) inhibiting the disease or condition, *i.e.,* restraining its development;
(iii) ameliorating the disease or condition, *i.e.,* causing the disease or condition to subside; or
(iv) alleviating the symptoms caused by the disease or condition.

For the reaction of each step, the reaction temperature can be appropriately selected according to the solvent, starting material, reagent, *etc.,* and the reaction time can also be appropriately selected according to the reaction temperature, solvent, starting material, reagent, *etc.* After the reaction of each step, the target compound can be separated and purified from the reaction system by common methods, such as filtration, extraction, recrystallization, washing, silica gel column chromatography, and other methods. Without affecting the next reaction step, the target compound can also be directly used in the next reaction step without separation and purification.

The above preferred conditions can be combined arbitrarily to obtain preferred embodiments of the present disclosure without violating common knowledge in the art.

The reagents and raw materials used in the present disclosure are all commercially available.

The positive progressive effect of the present disclosure is that after extensive and in-depth research, the inventors have unexpectedly developed a diaryl compound as a tubulin/Src dual target inhibitor, a preparation method therefor, and a use thereof. The diaryl compound has a structure of formula I as described in the present disclosure. The diaryl compound can be used as a dual-target inhibitor against tubulin and Src kinase, and can also be used as a mono-target inhibitor against tubulin or Src kinase. The compound of the present disclosure can significantly inhibit the polymerization of tubulin monomers and inhibit cell proliferation. It has good pharmacokinetic properties for topical administration to the skin and has good druggability. It can be used for preparing skin external preparations with the advantages of fast metabolism and low side effects.

### BRIEF DESCRIPTION OF THE DRAWINGS

Figure 1 shows a polymerization curve of tubulin monomer by compounds in test example 1.
Figure 2 shows the results of inhibition of Src signaling pathway by compounds.

### DETAILED DESCRIPTION OF THE PREFERRED EMBODIMENT

The present disclosure is further illustrated below in conjunction with specific examples. It should be understood that the following description is only the most preferred embodiment of the present disclosure and should not be construed as limiting the scope of protection of the present disclosure. On the basis of a full understanding of the present disclosure, the experimental methods without indication of specific conditions in the following examples shall be implemented usually in accordance with conventional conditions or the conditions suggested by the manufacturer. Those skilled in the art can make non-essential modifications to the technical solutions of the present disclosure, and such modifications should be considered to be included in the scope of protection of the present disclosure.

### Intermediate A1: Preparation of N-benzyl-2-(5-bromopyridin-2-yl)acetamide

The synthetic route is as follows:

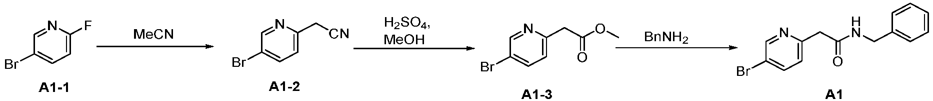

### Step 1: Preparation of 2-(5-bromopyridin-2-yl)acetonitrile

Acetonitrile (19.6 g, 477 mmol) was added to anhydrous tetrahydrofuran (500 mL) at room temperature, cooled to -78°C, and n-butyllithium (170 mL, 426 mmol) was slowly added dropwise thereto. The reaction mixture was stirred at -78°C for 1 hour, and then 5-bromo-2-fluoropyridine (30 g, 170 mmol) in tetrahydrofuran (100 mL) was added dropwise thereto. After the dropwise addition was completed, the reaction mixture was slowly warmed to room temperature and stirred for another 2 hours. After the completion of the reaction was detected by TLC, the reaction mixture was poured into saturated ammonium chloride solution/ethyl acetate (300 mL/300 mL), and the phases were separated. The aqueous phase was extracted with ethyl acetate (200 mL × 3), and the organic phases were combined, dried over anhydrous sodium sulfate, filtered, concentrated, and the residue was purified by a thin-layer silica gel plate to obtain compound A1-2 (22.5 g, yield: 67%).

### Step 2: Preparation of methyl 2-(5-bromopyridin-2-yl)acetate

Compound A1-2 (30 g, 152 mmol) was added to anhydrous methanol (200 mL) at room temperature, and concentrated sulfuric acid (25 mL, 457 mmol) was slowly added dropwise thereto, and then the reaction mixture was heated to reflux and stirred for 24 hours. After the completion of the reaction was detected by TLC, the reaction mixture was concentrated under reduced pressure to remove methanol, and the residue was dissolved in dichloromethane (500 mL). The organic phase was sequentially washed with water (200 mL), saturated sodium bicarbonate solution (200 mL), and saturated brine (200 mL), dried over anhydrous sodium sulfate, filtered, concentrated, and the residue was purified by a thin-layer silica gel plate to obtain compound A1-3 (31 g, yield: 88%).

### Step 3: Preparation ofN-benzyl-2-(5-bromopyridin-2-yl)acetamide

Benzylamine (39.1 g, 365 mmol) was added to compound A1-3 (28 g, 122 mmol) at room temperature, and then the reaction mixture was heated to 125°C and stirred for 15 hours. After the completion of the reaction was detected by TLC, the reaction mixture was cooled to room temperature, and ethyl acetate/petroleum ether (100 mL/100 mL) was slowly added thereto under stirring. The precipitated solid was filtered to obtain compound A1 (yellow solid, 28.5 g, yield: 77%).

¹H NMR (400 MHz, DMSO-d₆): δ 8.66 - 8.53 (m, 2H), 7.97 (dd, *J* = 8.3, 2.5 Hz, 1H), 7.38 - 7.19 (m, 6H), 4.26 (d, *J =* 5.9 Hz, 2H), 3.66 (s, 2H).

LC-MS, M/Z (ESI): 306.9 [M+H]⁺.

### Intermediate A2: Preparation of N benzyl-2-(5-(tributylstannyl)pyridin-2-yl)acetamide

The synthetic route is as follows:

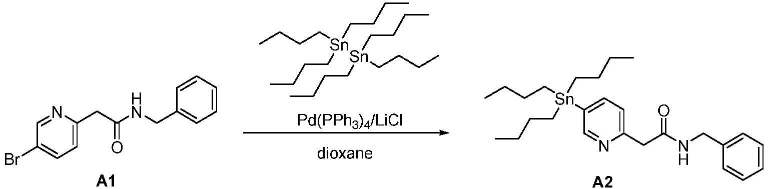

Intermediate A1 (15 g, 49.2 mmol) was added to 1,4-dioxane (300 mL) at room temperature, then lithium chloride (6.2 g, 147 mmol) was added thereto, and 1,1,1,2,2,2-hexabutyldistannane (29.8 mL, 59.0 mmol) and tetrakis(triphenylphosphine)palladium (2.84 g, 2.46 mmol) were added thereto under a nitrogen atmosphere, and then the reaction mixture was heated to 110°C and stirred for 15 hours. After the completion of the reaction was detected by TLC, the reaction mixture was cooled to room temperature, concentrated under reduced pressure to remove the solvent, diluted with water (500 mL), and extracted with ethyl acetate (200 mL × 3), and the phases were separated. The organic phases were combined, dried over anhydrous sodium sulfate, filtered, concentrated, and the residue was purified by a thin-layer silica gel plate (petroleum ether: ethyl acetate (V/V) = 5:1) to obtain intermediate A2 (10.5 g, yield: 41.5%).

¹H NMR (400 MHz, CDCl₃): δ 8.01 (s, 3H), 7.71 (dd, *J =* 7.5, 1.6 Hz, 1H), 7.31-7.26 (m, 2H), 7.24 - 7.20 (m, 3H), 4.47 (d, *J =* 5.8 Hz, 2H), 3.74 (s, 2H), 1.56 - 1.47 (m, 5H), 1.31 (ddd, *J =* 22.6, 14.9, 7.3 Hz, 7H), 1.08 (dd, *J =* 9.5, 6.8 Hz, 6H), 0.87 (t, *J =* 7.3 Hz, 9H).

LC-MS, M/Z (ESI): 517.3 [M+H]⁺.

### Example 1: Preparation of compound 1-1

The synthetic route is as follows:

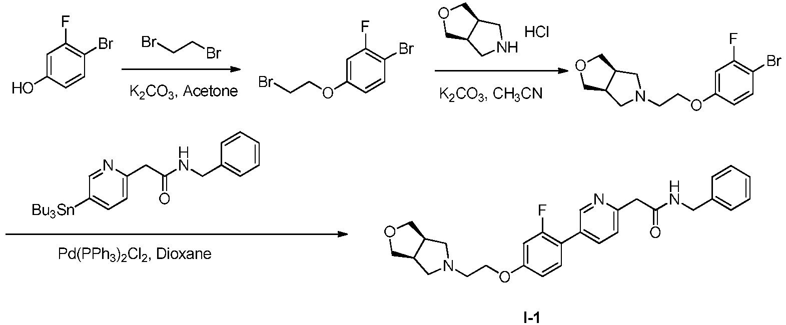

### Step 1: Preparation of 1-bromo-4-(2-bromoethoxy)-2-fluorobenzene

4-Bromo-3-fluorophenol (1 g, 5.24 mmol), 1,2-dibromoethane (5.90 g, 31.4 mmol), and potassium carbonate (4.41 g, 31.9 mmol) were dissolved in acetone (30 mL), and the reaction mixture was heated to 80°C and reacted for 18 hours. The reaction mixture was cooled to room temperature, filtered, the filtrate was concentrated, and the residue was separated by column chromatography (petroleum ether: ethyl acetate (V/V) = 30: 1) to obtain 1-bromo-4-(2-bromoethoxy)-2-fluorobenzene (1.3 g, colorless oil, yield: 83%).

### Step 2: Preparation of (3aR,6aS)-S-(2-(4-bromo-3-fluorophenoxy)ethyl)hexahydro-1H-furo[3,4-c]pyrrole

1-Bromo-4-(2-bromoethoxy)-2-fluorobenzene (1.3 g, 4.36 mmol) was dissolved in dry acetonitrile (30 mL), then (3a*R*,6a*S*)-hexahydro-1*H*-furo[3,4-*c*]pyrrole hydrochloride (718 mg, 4.80 mmol) and potassium carbonate (1.809 g, 13.09 mmol) were added thereto, and the reaction mixture was heated to 80°C and reacted for 20 hours. The reaction mixture was cooled to room temperature, filtered, the filtrate was concentrated, and the residue was purified by silica gel column chromatography (petroleum ether: ethyl acetate (V/V) = 1:1) to obtain (3a*R*,6a*S*)-5-(2-(4-bromo-3-fluorophenoxy)ethyl)hexahydro-1*H*-furo[3,4-*c*]pyrrole (1.1 g, yellow oil, yield: 76%).

LC-MS, M/Z (ESI): 330.0 [M+H]⁺.

### Step 3: Preparation of N-benzyl-2-(5-(2-fluoro-4-(2-((3aR,6aS)-tetrahydro-1H-furo[3,4-c]pyrrol-5(3H)-yl)ethoxy)phenyl)pyridin-2-yl)acetamide (I-1)

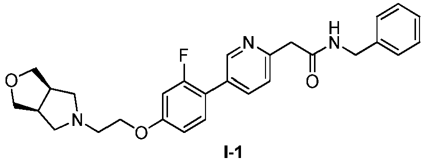

(3a*R*,6a*S*)-5-(2-(4-Bromo-3-fluorophenoxy)ethyl)hexahydro-1*H*-furo[3,4-*c*]pyrrole (150 mg, 0.454 mmol) was dissolved in dry 1,4-dioxane (5 mL), then *N*-benzyl-2-(5-(tributylstannyl)pyridin-2-yl)acetamide (281 mg, 0.545 mmol) and bis(triphenylphosphine)palladium(II) dichloride (31.9 mg, 0.045 mmol) were added thereto. The reaction system was replaced with argon three times, and heated to 100°C and reacted for 4 hours under an argon atmosphere. The reaction mixture was cooled to room temperature, diluted with water (50 mL), and extracted with dichloromethane (50 mL × 3). The organic phases were combined, dried over anhydrous sodium sulfate, concentrated, and the residue was separated by a silica gel plate (ethyl acetate: methanol (V/V) = 20:1, NH₃·H₂O) to obtain *N-*benzyl-2-(5-(2-fluoro-4-(2-((3a*R*,6a*S*)-tetrahydro-1*H*-furo[3,4-*c*]pyrrol-5(3*H*)-yl)ethoxy)phenyl)pyridin-2-yl)acetamide (I-1) (25 mg, yield: 11.57%).

¹H NMR (400 MHz, DMSO-d₆) δ 8.60 (br, 2H), 7.86 (m, 1H), 7.49 - 6.92 (m, 9H), 4.30 (m, 2H), 4.12 (m, 2H), 3.72 (m, 4H), 3.39 (m, 2H), 2.75 - 2.68 (m, 6H), 2.39 (m, 2H).

LC-MS, M/Z (ESI): 476.2 [M+H]⁺.

### Example 2: Preparation of compound I-2

The synthetic route is as follows:

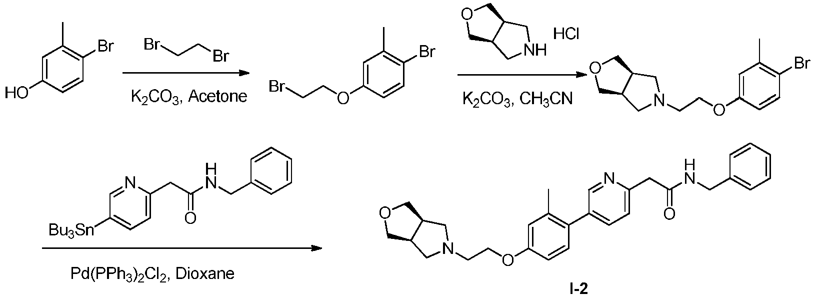

### Step 1: Synthesis of 1-bromo-4-(2-bromoethoxy)-2-methylbenzene

4-Bromo-3-methylphenol (1 g, 5.37 mmol), 1,2-dibromoethane (5.99 g, 32.2 mmol), and potassium carbonate (4.45 g, 32.2 mmol) were dissolved in acetone (30 mL), and the reaction mixture was heated to 80°C, refluxed, and reacted for 18 hours. The reaction mixture was cooled to room temperature, filtered, the filtrate was concentrated, and the residue was separated by column chromatography (petroleum ether: ethyl acetate (V/V) = 30:1) to obtain 1-bromo-4-(2-bromoethoxy)-2-methylbenzene (1.3 g, colorless oil, yield: 82.8%).

### Step 2: (3aR,6aS)-5-(2-(4-bromo-3-methylphenoxy)ethyl)hexahydro-1H-furo[3,4-c]pyrrole

1-Bromo-4-(2-bromoethoxy)-2-methylbenzene (1.3 g, 4.45 mmol) was dissolved in dry acetonitrile (30 mL), then (3a*R*,6a*S*)-hexahydro-1*H*-furo[3,4-*c*]pyrrole hydrochloride (730 mg, 4.90 mmol) and potassium carbonate (1.844 g, 13.36 mmol) were added thereto, and the reaction mixture was heated to 80°C and reacted for 20 hours. The reaction mixture was cooled to room temperature, filtered, the filtrate was concentrated, and the residue was purified by silica gel column chromatography (petroleum ether: ethyl acetate (V/V) = 1:1) to obtain (3a*R*,6a*S*)-5-(2-(4-bromo-3-methylphenoxy)ethyl)hexahydro-1*H*-furo[3,4-*c*]pyrrole (1.0 g, yellow oil, yield: 69.1%)

LC-MS, M/Z (ESI): 326.0 [M+H]⁺.

### Step 3: N-benzyl-2-(5-(2-methyl-4-(2-((3aR,6aS)-tetrahydro-1H-furo[3,4-c]pyrrol-5(3H)-yl)ethoxy)phenyl)pyridin-2-yl)acetamide (I-2)

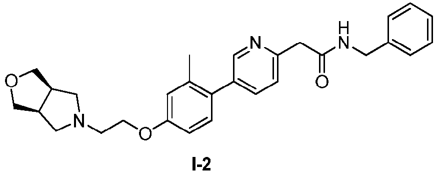

(3a*R*,6a*S*)-5-(2-(4-Bromo-3-methylphenoxy)ethyl)hexahydro-1*H*-furo[3,4-*c*]pyrrole (150 mg, 0.460 mmol) was dissolved in dry 1,4-dioxane (5 mL), then *N*-benzyl-2-(5-(tributylstannyl)pyridin-2-yl)acetamide (355 mg, 0.690 mmol) and bis(triphenylphosphine)palladium(II) dichloride (32.3 mg, 0.046 mmol) were added thereto. The reaction system was replaced with argon three times, and heated to 100°C and reacted for 4 hours under an argon atmosphere. The reaction mixture was cooled to room temperature, diluted with water (50 mL), and extracted with dichloromethane (50 mL × 3). The organic phases were combined, dried over anhydrous sodium sulfate, concentrated, and the residue was separated by a silica gel plate (ethyl acetate: methanol (V/V) = 20:1, NH₃·H₂O) to obtain *N-*benzyl-2-(5-(2-methyl-4-(2-((3a*R*,6a*S*)-tetrahydro-1*H*-furo[3,4-*c*]pyrrol-5(3*H*)-yl)ethoxy)phenyl)pyridin-2-yl)acetamide (I-2) (20 mg, yield: 9.22%).

¹H NMR (400 MHz, DMSO-d₆): δ 8.62 (t, 1H), 8.41(d, 1H), 7.70 - 6.83 (m, 10H), 4.30 (d, 2H), 4.07 (t, 2H), 3.78 - 3.62 (m, 4H), 3.39 - 3.37 (m, 2H), 2.75 - 2.61 (m, 6H), 2.39 - 2.37 (m, 2H), 2.21 (s, 3H).

LC-MS, M/Z (ESI): 472.2 [M+H]⁺.

### Example 3: Preparation of compound 1-3

The synthetic route is as follows:

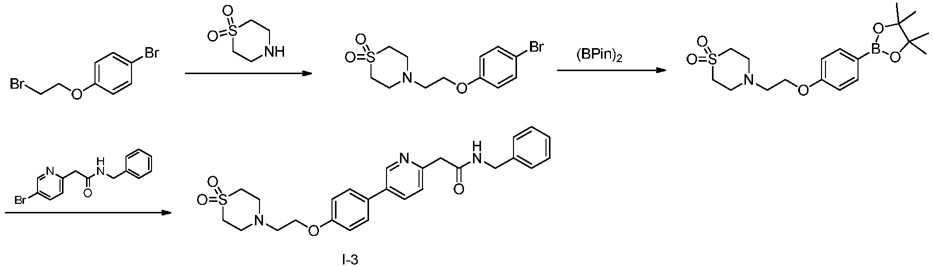

### Step 1: Preparation of 4-(2-(4-bromophenoxy)ethyl)thiomorpholine 1,1-dioxide

1-Bromo-4-(2-bromoethoxy)benzene (2.8 g, 10.0 mmol), K₂CO₃ 4.14 g, 30 mmol), and thiomorpholine 1,1-dioxide (1.5 g, 11.0 mmol) were added to acetonitrile (30 mL) at room temperature, and the reaction mixture was stirred at 85°C for 12 hours under a nitrogen atmosphere. After the reaction was completed, the reaction mixture was diluted with water (100 mL) and extracted with ethyl acetate (30 mL × 3). The phases were separated, and the organic phases were combined. The organic phase was dried over anhydrous sodium sulfate, filtered, and concentrated. The residue was purified by a thin-layer silica gel plate (petroleum ether: ethyl acetate (V/V) = 2:3) to obtain 4-(2-(4-bromophenoxy)ethyl)thiomorpholine 1,1-dioxide (2.5 g, white solid, yield: 75%).

### Step 2: Preparation of 4-(2-(4-(4,4,5,5-tetramethyl-1,3,2-dioxaborolan-2-yl)phenoxy)ethyl)thiomorpholine 1,1-dioxide

Compounds 4-(2-(4-bromophenoxy)ethyl)thiomorpholine 1,1-dioxide (2.0 g, 6.0 mmol), bis(pinacolato)diboron (2.28 g, 9.0 mmol), potassium acetate (1.76 g, 18.0 mmol), and [1,1'-bis(diphenylphosphino)ferrocene]dichloropalladium(II) (0.4 g, 0.6 mmol) were added to 1,4-dioxane (20 mL). The system was replaced with nitrogen, and the reaction mixture was heated to 85°C and stirred for 10 hours. The reaction mixture was diluted with water (50 mL) and extracted with ethyl acetate (30 mL × 3). The phases were separated, and the organic phases were combined. The organic phase was dried over anhydrous sodium sulfate, filtered, and concentrated. The residue was purified by a thin-layer silica gel plate (petroleum ether: ethyl acetate (V/V) = 1:2) to obtain 4-(2-(4-(4,4,5,5-tetramethyl-1,3,2-dioxaborolan-2-yl)phenoxy)ethyl)thiomorpholine 1,1-dioxide (1.1 g, white solid, yield: 70%).

### Step 3: Preparation of N-benzyl-2-(5-(4-(2-(1,1-dioxidothiomorpholino)ethoxy)phenyl)pyridin-2-yl)acetamide

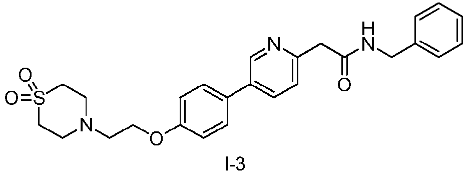

4-(2-(4-(4,4,5,5-Tetramethyl-1,3,2-dioxaborolan-2-yl)phenoxy)ethyl)thiomorpholine 1,1-dioxide (300 mg, 0.79 mmol), N benzyl-2-(5-bromopyridin-2-yl)acetamide (287 mg, 0.95 mmol), tetrakis(triphenylphosphine)palladium (80 mg, 0.071 mmol), and sodium carbonate 254 mg, 2.40 mmol) were added to 1,2-dimethoxyethane 3 mL) and water (0.5 mL), and the reaction mixture was heated to 100°C and stirred for 12 hours. The reaction mixture was diluted with water (20 mL) and extracted with ethyl acetate (10 mL × 3). The phases were separated, and the organic phases were combined. The organic phase was dried over anhydrous sodium sulfate, filtered, and concentrated. The residue was purified by a thin-layer silica gel plate (petroleum ether: ethyl acetate (V/V) = 1:2), and then purified by preparative liquid chromatography to obtain product *N*-benzyl-2-(5-(4-(2-(1,1-dioxidothiomorpholino)ethoxy)phenyl)pyridin-2-yl)acetamide (I-3) (70 mg, yield: 46.4%).

¹H NMR (400 MHz, CDCl₃) δ 8.70 (d, 1H), 7.81 (dd, 1H), 7.59 (s, 1H), 7.50 (t, 2H), 7.35 - 7.30 (m, 2H), 7.25 (d, 4H), 6.99 (d, 2H), 4.49 (d, 2H), 4.14 (t, 2H), 3.82 (s, 2H), 3.22-3.14 (m, 4H), 3.12 - 3.05 (m, 4H), 3.02 (t, 2H).

LC-MS, M/Z (ESI): 480.2 [M+H]⁺.

### Example 4: Preparation of compound 1-4

The synthetic route is as follows:

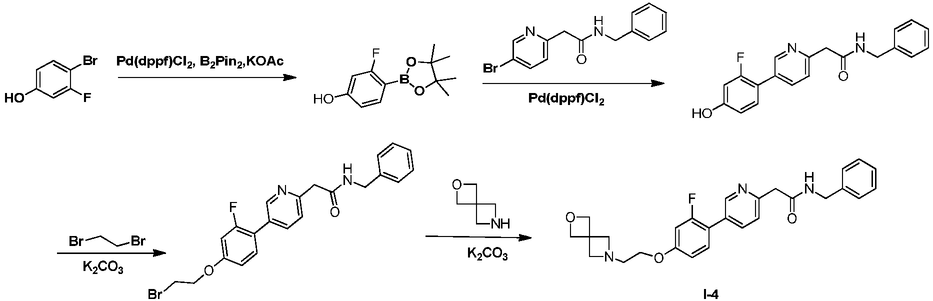

### Step 1: Preparation of 3-fluoro-4-(4,4,5,5-tetramethyl-1,3,2-dioxaborolan-2-yl)phenol

Compounds 4-bromo-3-fluorophenol (5.0 g, 24.10 mmol), KOAc (9.99 g, 72.3 mmol), bis(pinacolato)diboron (12.24 g, 48.2 mmol), and [1,1'-bis(diphenylphosphino)ferrocene]dichloropalladium(II) (1.76 g, 2.41 mmol) were added to dioxane (80 mL) at room temperature, and the reaction mixture was stirred at 85°C for 12 hours under a nitrogen atmosphere. After the reaction was completed, the reaction mixture was diluted with water (100 mL) and extracted with ethyl acetate (50 mL × 3), and the phases were separated. The organic phases were combined, dried over anhydrous sodium sulfate, filtered, concentrated, and the residue was purified by a thin-layer silica gel plate (petroleum ether: ethyl acetate (V/V) = 2:3) to obtain compound 3-fluoro-4-(4,4,5,5-tetramethyl-1,3,2-dioxaborolan-2-yl)phenol (3.8 g, white solid, yield: 60.1%).

### Step 2: Preparation of N-benzyl-2-(5-(2-fluoro-4-hydroxyphenyl)pylidin-2-yl)acetamide

Compounds 3-fluoro-4-(4,4,5,5-tetramethyl-1,3,2-dioxaborolan-2-yl)phenol (2.1 g, 8.25 mmol), *N*-benzyl-2-(5-bromopyridin-2-yl)acetamide (2.77 g, 9.08 mmol), potassium carbonate (3.42 g, 24.75 mmol), and [1,1'-bis(diphenylphosphino)ferrocene]dichloropalladium(II) (0.065 g, 0.089 mmol) were added to dioxane (30 mL). The system was replaced with nitrogen, and the reaction mixture was heated to 85°C and stirred for 10 hours. The reaction mixture was diluted with water (100 mL) and extracted with ethyl acetate (30 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 3:2) to obtain compound *N*-benzyl-2-(5-(2-fluoro-4-hydroxyphenyl)pyridin-2-yl)acetamide (2.6 g, white solid, yield: 42.4%).

LC-MS, M/Z (ESI): 337.3 [M+H]⁺.

### Step 3: Preparation of N-benzyl-2-(5-(4-(2-bromoethoxy)-2-fluorophenyl)pyridin-2-yl)acetamide

*N-*Benzyl-2-(5-(2-fluoro-4-hydroxyphenyl)pyridin-2-yl)acetamide (0.14 g, 0.416 mmol), 1,2-dibromoethane (0.469 g, 2.497 mmol), and potassium carbonate (0.345 g, 2.497 mmol) were placed in acetonitrile (3 mL), and the reaction mixture was heated to 85°C and stirred for 12 hours. The reaction mixture was diluted with water (5 mL) and extracted with ethyl acetate (5 mL × 3). The phases were separated, and the organic phases were combined. The organic phase was dried over anhydrous sodium sulfate, filtered, and concentrated. The residue was purified by column chromatography (petroleum ether: ethyl acetate (V/V) = 3:1) to obtain compound *N-*benzyl-2-(5-(4-(2-bromoethoxy)-2-fluorophenyl)pyridin-2-yl)acetamide (0.14 g, white solid, yield: 76%).

### Step 4: Preparation of 2-(5-(4-(2-(2-oxa-6-azaspiro[3.3]heptan-6-yl)ethoxy)-2-fluorophenyl)pyridin-2-yl)-N-benzylacetamide (I-4)

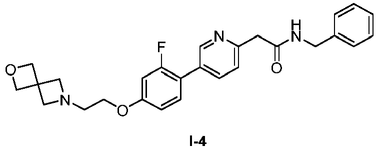

*N-*Benzyl-2-(5-(4-(2-bromoethoxy)-2-fluorophenyl)pyridin-2-yl)acetamide (0.14 g, 0.316 mmol), 2-oxa-6-azaspiro[3.3]heptane (0.063 g, 0.632 mmol), and potassium carbonate (0.131 g, 0.947 mmol) were placed in acetonitrile (3 mL), and the reaction mixture was heated to 85°C and stirred for 12 hours. The reaction mixture was diluted with water (5 mL) and extracted with ethyl acetate (5 mL × 3). The phases were separated, and the organic phases were combined. The organic phase was dried over anhydrous sodium sulfate, filtered, concentrated, and the residue was purified by preparative high-performance liquid chromatography to obtain compound 2-(5-(4-(2-(2-oxa-6-azaspiro[3.3]heptan-6-yl)ethoxy)-2-fluorophenyl)pyridin-2-yl)-*N*-benzylacetamide (I-4) (8.6 mg, yield: 5.9%).

¹H NMR (400 MHz,CDCl₃) δ 8.64 (s, 1H), 7.80 (d, 1H), 7.67 (s, 1H), 7.33 (dd, 2H), 7.30 (d, 1H), 7.28 (s, 1H), 7.24 (dt, 3H), 6.75 (m, 2H), 4.75 (s, 4H), 4.47 (t, 2H), 3.98 (t, 2H), 3.82 (s, 2H), 3.49 (s, 4H), 2.80 (t, 2H).

LC-MS, M/Z (ESI): 462.1 [M+H]⁺.

### Example 5: Preparation of compound 1-5

The synthetic route is as follows:

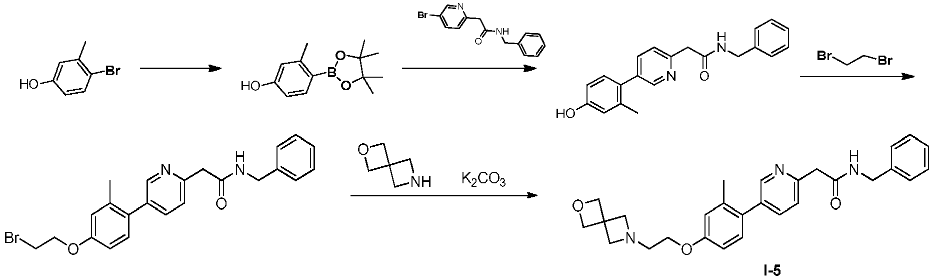

### Step 1: Preparation of 3-methyl-4-(4,4,5,5-tetramethyl-1,3,2-dioxaborolan-2-yl)phenol

Compounds 4-bromo-3-methylphenol (5 g, 26.7 mmol) and bis(pinacolato)diboron (10.2 g, 40.1 mmol) were added to 1,4-dioxane (70 mL) at room temperature, then potassium acetate (6.56 g, 66.8 mmol) was added thereto, and [1,1'-bis(diphenylphosphino)ferrocene]dichloropalladium(II) (950 mg, 1.3 mmol) was added thereto under a nitrogen atmosphere. The reaction mixture was heated to 95°C and stirred for 6 hours. The reaction mixture was cooled to room temperature, diluted with water (50 mL), and extracted with ethyl acetate (50 mL × 3), and the phases were separated. The organic phases were combined, dried over anhydrous sodium sulfate, filtered, concentrated, and the residue was purified by a thin-layer silica gel plate (petroleum ether: ethyl acetate (V/V) = 5:1) to obtain compound 2-methyl-4-(4-(4,4,5,5-tetramethyl-1,3,2-dioxaborolan-2-yl)phenoxy)butan-2-ol (6.1 g, yield: 97%).

### Step 2: Preparation of N-benzyl-2-(5-(4-hydroxy-2-methylphenyl)pyridin-2-yl)acetamide

Compounds 3-methyl-4-(4,4,5,5-tetramethyl-1,3,2-dioxaborolan-2-yl)phenol (1.15 g, 4.92 mmol) and *N*-benzyl-2-(5-bromopyridin-2-yl)acetamide (1.0 g, 3.33 mmol) were added to 1,4-dioxane/water (10 mL/1 mL) at room temperature, then potassium fluoride (0.760 g, 13.1 mmol) was added thereto, and [1,1'-bis(diphenylphosphino)ferrocene]dichloropalladium(II) (117 mg, 0.16 mmol) was added thereto under a nitrogen atmosphere. The reaction mixture was heated to 90°C and stirred for 10 hours. The reaction mixture was cooled to room temperature, diluted with water (50 mL), and extracted with ethyl acetate (20 mL × 3), and the phases were separated. The organic phases were combined, dried over anhydrous sodium sulfate, filtered, concentrated, and the residue was purified by column chromatography on silica gel (CH₂Cl₂: MeOH (V/V) = 95:5) to obtain compound N-benzyl-2-(5-(4-(3-hydroxy-3-methylbutoxy)phenyl)pyridin-2-yl)acetamide (850 mg, yield: 78%).

### Step 3: Preparation of N-benzyl-2-(5-(4-(2-bromoethoxy)-2-methylphenyl)pyridin-2-yl)acetamide

*N-*Benzyl-2-(5-(2-methyl-4-hydroxyphenyl)pyridin-2-yl)acetamide (0.6 g, 1.8 mmol) and potassium carbonate (1.5 g, 10.7 mmol) were added to anhydrous acetonitrile (15 mL) at room temperature, heated to 80°C and stirred for 1 hour, then 1,2-dibromoethane (2.01 g, 10.7 mmol) was added thereto, and the reaction mixture was stirred at 80°C for another 10 hours. The reaction mixture was cooled to room temperature, and concentrated to remove the solvent to obtain a crude product, which was purified by silica gel column chromatography (DCM: MeOH (V/V) = 95:5) to obtain compound *N-*benzyl-2-(5-(4-(2-bromoethoxy)-2-fluorophenyl)pyridin-2-yl)acetamide (0.55 g, yield: 69%).

### Step 4: Preparation of 2-(5-(4-(2-(2-oxa-6-azaspiro[3.3]heptan-6-yl)ethoxy)-2-methylphenyl)pyridin-2-yl)-N-benzylacetamide (I-5)

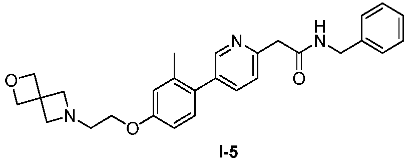

In a 100.0 mL single-necked flask, *N-*benzyl-2-(5-(4-(2-bromoethoxy)-2-fluorophenyl)pyridin-2-yl)acetamide (500.0 mg, 1.14 mmol) was dissolved in acetonitrile (5.0 mL), then potassium carbonate (786.4 mg, 5.69 mmol) was added thereto. After 2-oxa-6-azaspiro[3.3]heptane (124.1 mg, 1.25 mmol) was added dropwise thereto, the system was heated to 85°C and stirred for 16 hours. After the reaction was completed, the reaction mixture was filtered, and the mother liquor was subjected to rotary evaporation until dryness. The residue was purified by silica gel column chromatography (petroleum ether: ethyl acetate (V/V) = 20:1 to 1:1) to obtain 2-(5-(4-(2-(2-oxa-6-azaspiro[3.3]heptan-6-yl)ethoxy)-2-methylphenyl)pyridin-2-yl)-*N-*benzylacetamide (340.0 mg, yield: 65.3%).

¹H NMR (400 MHz, DMSO-d₆) δ 8.64 (t, 1H), 8.42 (d, 1H), 7.70 (dd, 1H), 7.39 (d, 1H), 7.36 - 7.19 (m, 5H), 7.14 (d, 1H), 6.88 (d, 1H), 6.83 (dd, 1H), 4.60 (s, 4H), 4.31 (d, 2H), 3.93 (t, 2H), 3.73 (s, 2H), 3.35 (s, 4H), 2.68 (t, 2H), 2.22 (s, 3H).

LC-MS, M/Z (ESI): 458.05 [M+H]⁺.

The following compounds were prepared by referring to the preparation of other compounds of the present disclosure.

| **Compound No.** | **Structural formula/naming** | **Compound mass spectrum** |
|---|---|---|
| | | LC-MS, M/Z (ESI) |
| **I-6** | 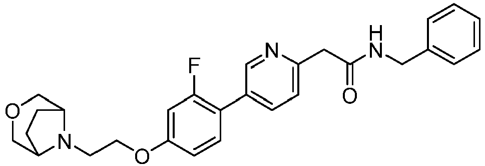 2-(5-(4-(2-(3-oxa-8-azabicyclo[3.2.1]octan-8-yl)ethoxy)-2-fluorophenyl)pyridin-2-yl)-*N-*benzylacetamide | 476.2 |
| **I-7** | 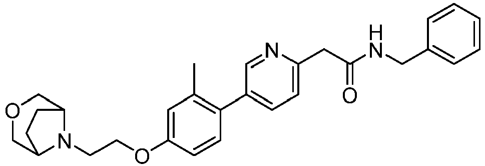 2-(5-(4-(2-(3-oxa-8-azabicyclo[3.2.1]octan-8-yl)ethoxy)-2-methylphenyl)pyridin-2-yl)-*N-*benzylacetamide | 472.3 |
| **I-8** | 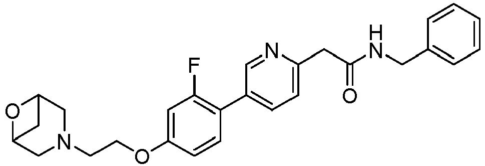 2-(5-(4-(2-(6-oxa-3-azabicyclo[3.1.1]heptan-3-yl)ethoxy)-2-fluorophenyl)pyridin-2-yl)-*N-*benzylacetamide | 462.2 |
| **I-9** | 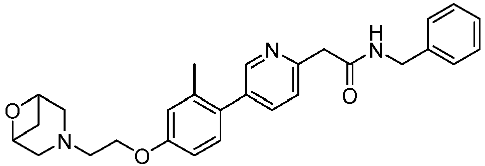 2-(5-(4-(2-(6-oxa-3-azabicyclo[3.1.1]heptan-3-yl)ethoxy)-2-methylphenyl)pyridin-2-yl)-*N-*benzylacetamide | 458.2 |
| **I-10** | 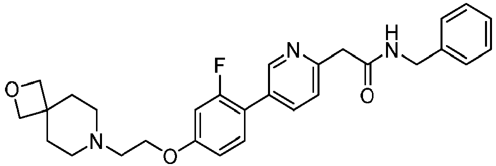 2-(5-(4-(2-(2-oxa-7-azaspiro[3.5]nonan-7-yl)ethoxy)-2-fluorophenyl)pyridin-2-yl)-*N-*benzylacetamide | 490.3 |
| **I-11** | 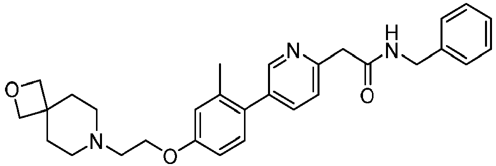 2-(5-(4-(2-(2-oxa-7-azaspiro[3.5]nonan-7-yl)ethoxy)-2-methylphenyl)pyridin-2-yl)-*N-*benzylacetamide | 486.3 |
| **I-12** | 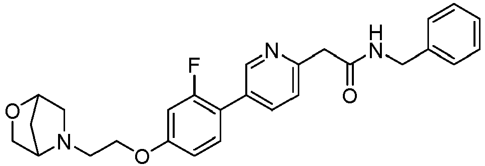 2-(5-(4-(2-(2-oxa-5-azabicyclo[2.2.1]heptan-5-yl)ethoxy)-2-fluorophenyl)pyridin-2-yl)-*N-*benzylacetamide | 462.2 |
| **I-13** | 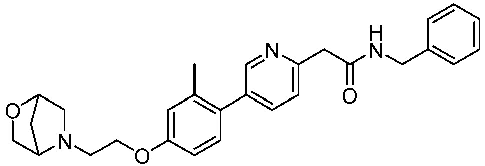 2-(5-(4-(2-(2-oxa-5-azabicyclo[2.2.1]heptan-5-yl)ethoxy)-2-methylphenyl)pyridin-2-yl)-*N-*benzylacetamide | 458.2 |
| **I-14** | 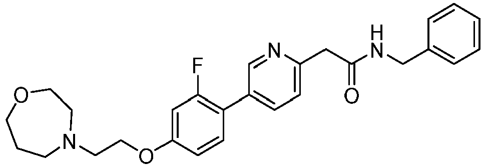 2-(5-(4-(2-(1,4-oxazepan-4-yl)ethoxy)-2-fluorophenyl)pyridin-2-yl)-*N*-benzylacetamide | 464.2 |
| **I-15** | 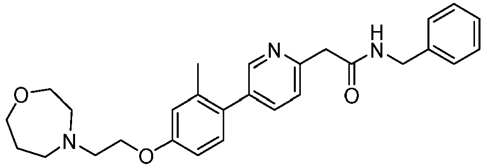 2-(5-(4-(2-(1,4-oxazepan-4-yl)ethoxy)-2-methylphenyl)pyridin-2-yl)-N benzylacetamide | 460.3 |
| **I-16** | 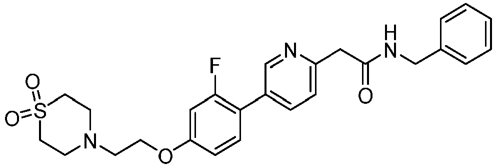 *N*-benzyl-2-(5-(4-(2-(1,1-dioxidothiomorpholino)ethoxy)-2-fluorophenyl)pyridin-2-yl)acetamide | 498.2 |
| **I-17** | 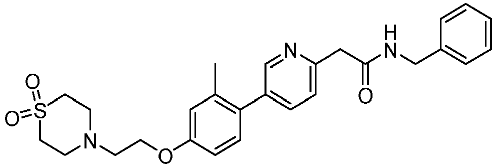 *N*-benzyl-2-(5-(4-(2-(1,1-dioxidothiomorpholino)ethoxy)-2-methylphenyl)pyridin-2-yl)acetamide | 494.2 |
| **I-19** | 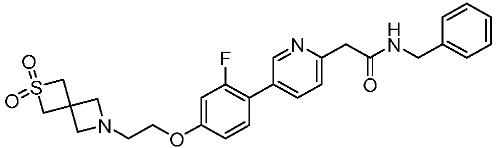 *N-*benzyl-2-(5-(4-(2-(2,2-dioxido-2-thia-6-azaspiro[3.3]heptan-6-yl)ethoxy)-2-fluorophenyl)pyridin-2-yl)acetamide | 510.2 |
| **I-20** | 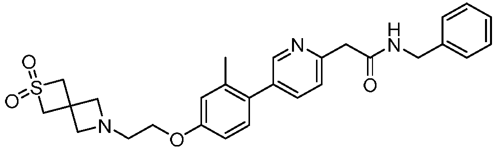 *N*-benzyl-2-(5-(4-(2-(2,2-dioxido-2-thia-6-azaspiro[3.3]heptan-6-yl)ethoxy)-2-methylphenyl)pyridin-2-yl)acetamide | 506.2 |
| **I-21** | 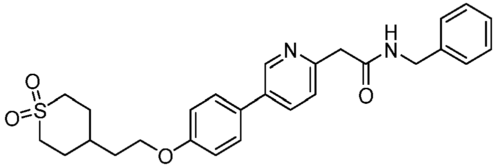 *N*-benzyl-2-(5-(4-(2-(1,1-dioxidotetrahydro-2*H-*thiopyran-4-yl)ethoxy)phenyl)pyridin-2-yl)acetamide | 479.2 |
| **I-22** | 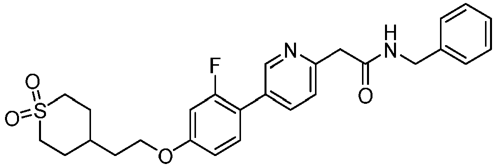 *N*-benzyl-2-(5-(4-(2-(1,1-dioxidotetrahydro-2*H-*thiopyran-4-yl) ethoxy)-2-fluorophenyl)pyridin-2-yl)acetamide | 497.2 |
| **I-23** | 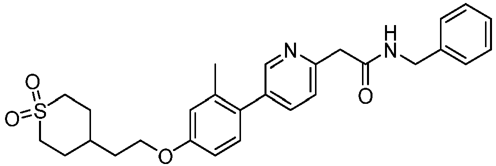 *N*-benzyl-2-(5-(4-(2-(1,1-dioxidotetrahydro-2*H-*thiopyran-4-yl)ethoxy)-2-methylphenyl)pyridin-2-yl)acetamide | 493.2 |
| **I1-25** | 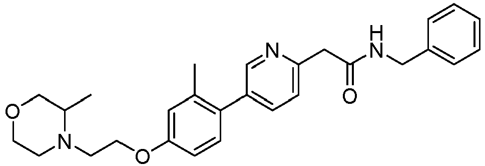 *N*-benzyl-2-(5-(2-methyl-4-(2-(3-methylmorpholino)ethoxy)phenyl)pyridin-2-yl)acetamide | 460.3 |

### Example 18: Preparation of compound I-18

The synthetic route is as follows:

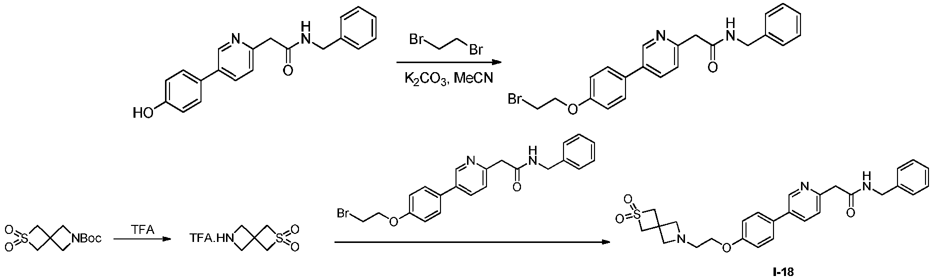

### Step 1: Preparation of N-benzyl-2-(5-(4-(2-bromoethoxy)phenyl)pyridin-2-yl)acetamide

*N-*Benzyl-2-(5-(4-hydroxyphenyl)pyridin-2-yl)acetamide (0.5 g, 1.56 mmol), 1,2-dibromoethane (1.75 g, 9.35 mmol), and potassium carbonate (1.29 g, 9.35 mmol) were placed in acetonitrile (6 mL), and the reaction mixture was heated to 85°C and stirred for 12 hours. The reaction mixture was diluted with water (10 mL) and extracted with ethyl acetate (5 mL × 3). The phases were separated, and the organic phases were combined. The organic phase was dried over anhydrous sodium sulfate, filtered, and concentrated. The residue was purified by column chromatography (petroleum ether: ethyl acetate (V/V) = 3:1) to obtain compound N-benzyl-2-(5-(4-(2-bromoethoxy)phenyl)pyridin-2-yl)acetamide (0.4 g, white solid, yield: 60.0%).

### Step 2: Preparation of 2-thia-6-azaspiro[3.3]heptane-2,2-dioxide

*tert*-Butyl 2-thia-6-azaspiro[3.3]heptane-6-carboxylate 2,2-dioxide (0.2 g, 0.81 mmol) was added to anhydrous dichloromethane (2 mL) at room temperature, then trifluoroacetic acid (2 mL) was added thereto, and the reaction mixture was stirred at room temperature for 2 hours. After the completion of the reaction was detected by TLC, the reaction mixture was concentrated to remove the solvent to obtain the crude product 2-thia-6-azaspiro[3.3]heptane-2,2-dioxide trifluoroacetate (0.21 g, yield: 100%), which was directly used in the next step.

### Step 3: Preparation of N-benzyl-2-(5-(4-(2-(2,2-dioxido-2-thia-6-azaspiro[3.3]heptan-6-yl)ethoxy)phenyl)pyridin-2-yl)acetamide (1-18)

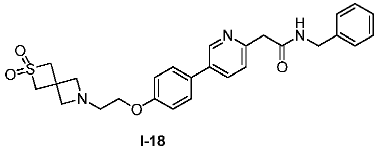

*N*-Benzyl-2-(5-(4-(2-bromoethoxy)phenyl)pyridin-2-yl)acetamide (0.1 g, 0.23 mmol) was added to DMF (2 mL) at room temperature, then triethylamine (91 mg, 0.9 mmol), potassium iodide (150 mg, 0.9 mmol), and the crude product 2-thia-6-azaspiro[3.3]heptane-2,2-dioxide trifluoroacetate (120 mg, 0.47 mmol) were added thereto, and the reaction mixture was stirred at 60°C for 8 hours. After the completion of the reaction was detected by TLC, the reaction mixture was purified by preparative high-performance liquid chromatography to obtain compound *N*-benzyl-2-(5-(4-(2-(2,2-dioxido-2-thia-6-azaspiro[3.3]heptan-6-yl)ethoxy)phenyl)pyridin-2-yl)acetamide (68.1 mg, yield: 59%).

¹H NMR (400 MHz, DMSO-d₆) δ 8.74 (d, 1H), 8.61 (t, 1H), 7.96 (dd, 1H), 7.64 (d, 2H), 7.39 (d, 1H), 7.34 - 7.20 (m, 5H), 7.02 (d, 2H), 4.37 - 4.25 (m, 6H), 3.99 (t, 2H), 3.70 (s, 2H), 3.42 (s, 4H), 2.77 (t, 2H).

LC-MS, M/Z (ESI): 492.2 [M+H]⁺.

### Example 24: Preparation of compounds I-24A and I-24B

The synthetic route is as follows:

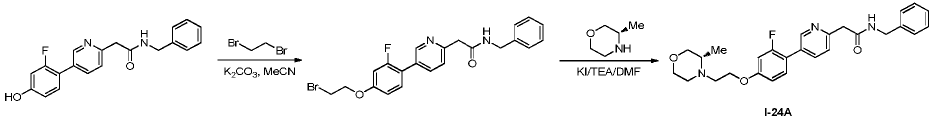

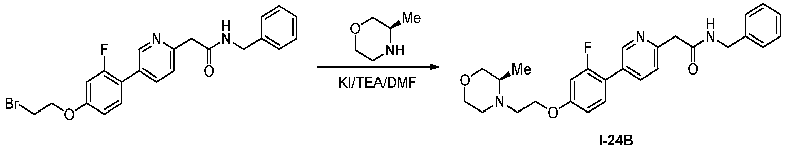

### Step 1: Preparation of N-benzyl-2-(5-(4-(2-bromoethoxy)-2-fluorophenyl)pyridin-2-yl)acetamide

*N-*Benzyl-2-(5-(2-fluoro-4-hydroxyphenyl)pyridin-2-yl)acetamide (0.6 g, 1.8 mmol) and potassium carbonate (1.5 g, 10.7 mmol) were added to anhydrous acetonitrile (15 mL) at room temperature, heated to 80°C and stirred for 1 hour, then 1,2-dibromoethane (2.01 g, 10.7 mmol) was added thereto, and the reaction mixture was stirred at 80°C for another 10 hours. After the completion of the reaction was detected by TLC, the reaction mixture was cooled to room temperature, concentrated by rotary evaporation to remove the solvent to obtain a crude product, which was purified by silica gel column chromatography to obtain compound *N-*benzyl-2-(5-(4-(2-bromoethoxy)-2-fluorophenyl)pyridin-2-yl)acetamide (0.55 g, yield: 69%).

### Step 2: Preparation of (S)-N-benzyl-2-(5-(2-fluoro-4-(2-(3-methylmorpholino)ethoxy)phenyl)pyridin-2-yl)acetamide

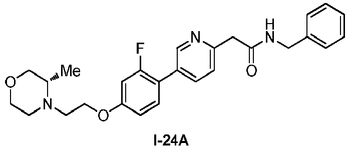

*N-*Benzyl-2-(5-(4-(2-bromoethoxy)-2-fluorophenyl)pyridin-2-yl)acetamide (0.1 g, 0.23 mmol) was added to *N*,*N*-dimethylformamide (2 mL) at room temperature, then triethylamine (91 mg, 0.9 mmol), potassium iodide (150 mg, 0.9 mmol), and (*S*)-3-methylmorpholine (34 mg, 0.34 mmol) were added thereto, and the reaction mixture was stirred at 60°C for 8 hours. The reaction mixture was purified by preparative high-performance liquid chromatography to obtain compound (*S*)-*N*-benzyl-2-(5-(2-fluoro-4-(2-(3-methylmorpholino)ethoxy)phenyl)pyridin-2-yl)acetamide (I-24A) (18.9 mg, yield: 18%).

¹H NMR (400 MHz, DMSO-d₆) δ 8.68 - 8.56 (m, 2H), 7.86 (d, 1H), 7.50 (t, 1H), 7.43(d, 1H), 7.36 - 7.19 (m, 5H), 7.03 - 6.88 (m, 2H), 4.30 (d, 2H), 4.13 (t, 2H), 3.72 (s, 2H), 3.70 - 3.64 (m, 1H), 3.59 (dd, 1H), 3.52 - 3.43 (m, 1H), 3.06 (ddd, 2H), 2.80 (dt, 1H), 2.68 - 2.56 (m, 1H), 2.47 - 2.34 (m, 2H), 0.93 (t, 3H).

LC-MS, M/Z (ESI): 464.3 [M+H]⁺.

### Step 3: Preparation of (R)-N-benzyl-2-(5-(2-fluoro-4-(2-(3-methylmorpholino)ethoxy)phenyl)pyridin-2-yl)acetamide

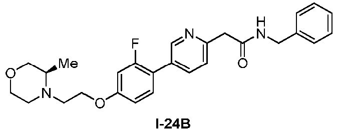

*N-*Benzyl-2-(5-(4-(2-bromoethoxy)-2-fluorophenyl)pyridin-2-yl)acetamide (0.1 g, 0.23 mmol) was added to DMF (2 mL) at room temperature, then triethylamine (91 mg, 0.9 mmol), potassium iodide (150 mg, 0.9 mmol), and (*R*)-3-methylmorpholine (34 mg, 0.34 mmol) were added thereto, and the reaction mixture was stirred at 60°C for 8 hours. After the completion of the reaction was detected by TLC, the reaction mixture was purified by preparative high-performance liquid chromatography to obtain compound (*R*)-*N*-benzyl-2-(5-(2-fluoro-4-(2-(3-methylmorpholino)ethoxy)phenyl)pyridin-2-yl)acetamide (I-24B) (19.9 mg, yield: 19%).

¹H NMR (400 MHz, DMSO-d₆) δ 8.63 (dd, *J=* 11.8, 5.6 Hz, 2H), 7.86 (d, *J=* 8.1 Hz, 1H), 7.50 (t, *J =* 9.0Hz, 1H), 7.43 (d, *J =* 8.1 Hz, 1H), 7.35 - 7.20 (m, 5H), 6.99 (dd, *J =* 12.9, 2.2 Hz, 1H), 6.92 (dd, *J =* 8.6,2.1 Hz, 1H), 4.30 (d, *J =* 5.9 Hz, 2H), 4.13 (t, *J =* 5.9 Hz, 2H), 3.73 (s, 2H), 3.67 (d, *J =* 11.1 Hz, 1H),3.59 (dd, *J =* 11.0, 2.9 Hz, 1H), 3.47 (td, *J =* 11.7, 5.6 Hz, 1H), 3.14 - 3.00 (m, 2H), 2.80 (d, *J =* 11.8 Hz,1H), 2.61 (dt, *J =* 13.5, 5.6 Hz, 1H), 2.47 - 2.35 (m, 2H), 0.94 (d, *J =* 6.2 Hz, 3H).

LC-MS, M/Z (ESI): 464.3 [M+H]⁺.

### Example 26: Preparation of compound 1-26

The synthetic route is as follows:

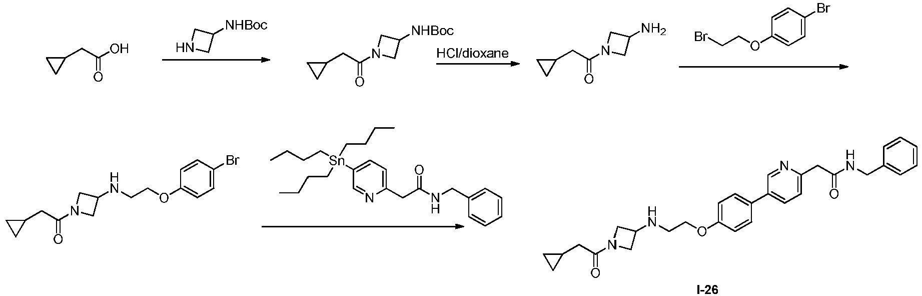

### Step 1: Preparation of tert-butyl (1-(2-cyclopropylacetyl)azetidin-3-yl)carbamate

2-Cyclopropylacetic acid (0.35 g, 3.5 mmol) was added to anhydrous dichloromethane (15 mL) at room temperature, then 3-(((ethylimino)methylene)amino)-*N*,*N*-dimethylpropan-1-amine hydrochloride (0.67 g, 3.5 mmol) and 1*H*-benzo[*d*][1,2,3]triazol-1-ol (0.54 g, 3.5 mmol) were added thereto, and the reaction mixture was stirred at room temperature for 10 minutes. Then *tert*-butyl azetidin-3-ylcarbamate (0.5 g, 2.9 mmol) was added thereto, and the reaction mixture was stirred at room temperature for another 5 hours. After the completion of the reaction was detected by TLC, the reaction mixture was diluted with dichloromethane (50 mL) and washed with saturated sodium bicarbonate solution/brine (50 mL/50 mL). The organic phase was dried over anhydrous sodium sulfate, filtered, concentrated, and the residue was purified by a thin-layer silica gel plate to obtain compound *tert*-butyl (1-(2-cyclopropylacetyl)azetidin-3-yl)carbamate (0.6 g, yield: 81%).

### Step 2: Preparation of 1-(3-aminoazetidin-1-yl)-2-cyclopropylethan-1-one

*tert*-Butyl (1-(2-cyclopropylacetyl)azetidin-3-yl)carbamate (0.6 g, 2.4 mmol) was added to anhydrous 1,4-dioxane (2 mL) at room temperature, then hydrochloric acid/1,4-dioxane (4.7 mL, 4 M) was added thereto with stirring, and the reaction mixture was stirred at room temperature for another 2 hours. After the completion of the reaction was detected by TLC, the reaction mixture was concentrated by rotary evaporation to remove the solvent to obtain compound 1-(3-aminoazetidin-1-yl)-2-cyclopropylethan-1-one hydrochloride (0.45 g, yield: 100%).

### Step 3: Preparation of 1-(3-((2-(4-bromophenoxy)ethyl)amino)azetidin-1-yl)-2-cyclopropylethan-1-one

1-(3-Aminoazetidin-1-yl)-2-cyclopropylethan-1-one hydrochloride (0.45 g, 2.4 mmol) was added to acetonitrile (5 mL) at room temperature, then potassium carbonate (0.98 g, 7.2 mmol) and 1-bromo-4-(2-bromoethoxy)benzene (0.73 g, 2.6 mmol) were added thereto, and the reaction mixture was stirred at 30°C for 8 hours. After the completion of the reaction was detected by TLC, the reaction mixture was concentrated by rotary evaporation to remove the solvent, and the residue was diluted with ethyl acetate (50 mL) and washed with water/brine (50 mL/50 mL). The organic phase was dried over anhydrous sodium sulfate, filtered, concentrated, and the residue was purified by thin-layer silica gel column chromatography to obtain compound 1-(3-((2-(4-bromophenoxy)ethyl)amino)azetidin-1-yl)-2-cyclopropylethan-1-one (0.2 g, yield: 24%).

### Step 4: Preparation of N-benzyl-2-(5-(4-(2-((1-(2-cyclopropylacetyl)azetidin-3-yl)amino)ethoxy)phenyl)pyridin-2-yl)acetamide (1-26)

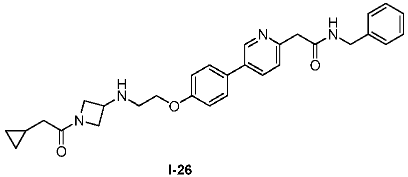

Compounds 1-(3-((2-(4-bromophenoxy)ethyl)amino)azetidin-1-yl)-2-cyclopropylethan-1-one (60 mg, 0.17 mmol) and *N-*benzyl-2-(5-(tributylstannyl)pyridin-2-yl)acetamide (110 mg, 0.21 mmol) were added to toluene (2 mL) at room temperature, then tetrakis(triphenylphosphine)palladium (20 mg, 0.017 mmol) was added thereto under a nitrogen atmosphere, and the reaction mixture was heated to 100°C and stirred for 10 hours. The reaction mixture was cooled to room temperature, diluted with water (50 mL), and extracted with ethyl acetate (20 mL × 3), and the phases were separated. The organic phases were combined, dried over anhydrous sodium sulfate, filtered, concentrated, and the residue was prepared by an alkaline preparation method to obtain compound *N*-benzyl-2-(5-(4-(3-hydroxy-3-methylbutoxy)phenyl)pyridin-2-yl)acetamide (I-26) (10.7 mg, yield: 9.1%).

¹H NMR (400 MHz, CDCl₃) δ8.71 (dd, *J* = 8.1, 2.2 Hz, 1H), 7.85 - 7.78 (m, 1H), 7.62 (s, 1H), 7.50 (d, *J =* 8.7 Hz, 2H), 7.32 (dd, *J* = 14.5, 7.3 Hz, 3H), 7.24 (d, *J* = 2.3 Hz, 2H), 7.00 (t, *J =* 9.2 Hz, 2H), 4.49 (d, *J* = 5.8 Hz, 2H), 4.34 - 4.18 (m, 2H), 4.11 (t, *J* = 5.0 Hz, 2H), 3.85 (dd, *J* = 8.9, 4.6 Hz, 1H), 3.82 (s, 2H),3.80 - 3.71 (m, 2H), 3.01 (h, *J* = 9.3 Hz, 2H), 2.04 (t, *J* = 6.6 Hz, 2H), 1.10 - 1.00 (m, 1H), 0.60 - 0.51(m, 2H), 0.15 (q, *J* = 4.8 Hz, 2H).

LC-MS, M/Z (ESI): 499.2 [M+H]⁺.

### Example 27: Preparation of compound 1-27

The synthetic route is as follows:

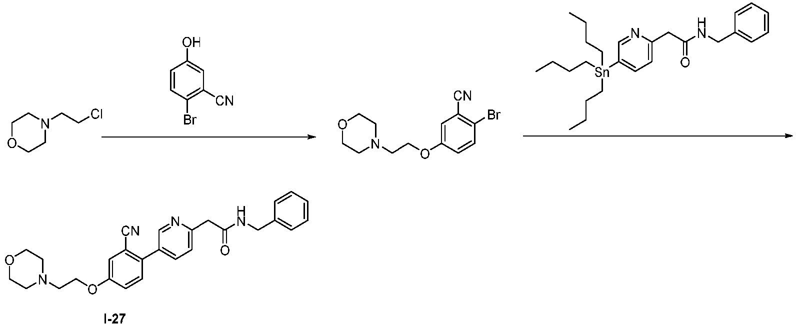

### Step 1: Preparation of 2-bromo-5-(2-morpholinoethoxy)benzonitrile

4-(2-Chloroethyl)morpholine (0.783 g, 4.21 mmol), 2-bromo-5-hydroxybenzonitrile (1 g, 5.05 mmol), and potassium carbonate (2.036 g, 14.73 mmol) were dissolved in acetonitrile (10 mL), and the reaction mixture was heated to 80°C and reacted for 10 hours. The reaction mixture was cooled to room temperature, added with sodium chloride aqueous solution (100 mL) and ethyl acetate (100 mL), and the phases were separated. The organic phase was concentrated and then separated by column chromatography (petroleum ether: ethyl acetate (V/V) = 2:1) to obtain compound 2-bromo-5-(2-morpholinoethoxy)benzonitrile (1.21 g, brown solid, yield: 92%).

### Step 2: N-benzyl-2-(5-(2-cyano-4-(2-morpholinoethoxy)phenyl)pyridin-2-yl)acetamide

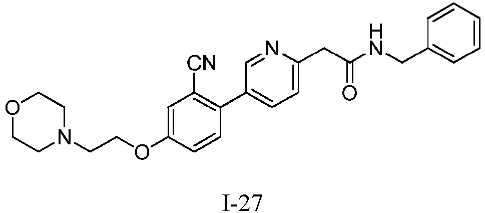

2-Bromo-5-(2-morpholinoethoxy)benzonitrile (100 mg, 0.32 mmol), *N*-benzyl-2-(5-(tributylstannyl)pyridin-2-yl)acetamide (248 mg, 0.482 mmol), and bis(triphenylphosphine)palladium(II) dichloride (22.56 mg, 0.032 mmol) were dissolved in dry 1,4-dioxane (15 mL), and then the reaction mixture was filled with nitrogen for protection, heated to 90°C, and reacted for 10 hours. The reaction mixture was cooled to room temperature, filtered, and the filtrate was concentrated, and then the residue was purified by silica gel column chromatography (petroleum ether: ethyl acetate (V/V) = 2:1) to obtain *N*-benzyl-2-(5-(2-cyano-4-(2-morpholinoethoxy)phenyl)pyridin-2-yl)acetamide (I-27) (5 mg, yield: 3.41%).

¹H NMR (400 MHz, DMSO-d₆) δ 8.66 (dd, *J* = 14.2, 3.9 Hz, 2H), 7.93 (dd, *J* = 8.0, 2.3 Hz, 1H), 7.58 (d, *J* = 9.0 Hz, 2H), 7.48 (d, *J* = 8.0 Hz, 1H), 7.40 (dd, *J* = 8.7, 2.6 Hz, 1H), 7.34 - 7.26 (m, 4H), 7.23 (t, *J* = 7.0 Hz, 1H), 4.31 (d, *J* = 5.8 Hz, 2H), 4.21 (t, *J* = 5.6 Hz, 2H), 3.76 (s, 2H), 3.56 (dd, *J* = 14.5, 10.0 Hz, 4H), 2.71 (t, *J* = 5.6 Hz, 2H), 2.50 - 2.45 (m, 4H).

LC-MS, M/Z (ESI): 457.2 [M+H]⁺.

### Example 28: Preparation of compound I-28

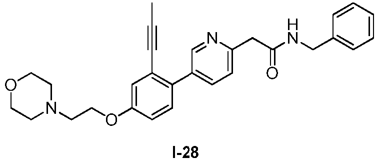

Compound *N-*benzyl-2-(5-(4-(2-morpholinoethoxy)-2-(prop-1-yn-1-yl)phenyl)pyridin-2-yl)acetamide (I-28) was prepared by referring to the preparation method of compound I-27. LC-MS, M/Z (ESI): 470.2 [M+H]⁺.

### Example 29: Preparation of compound I-29

The synthetic route is as follows:

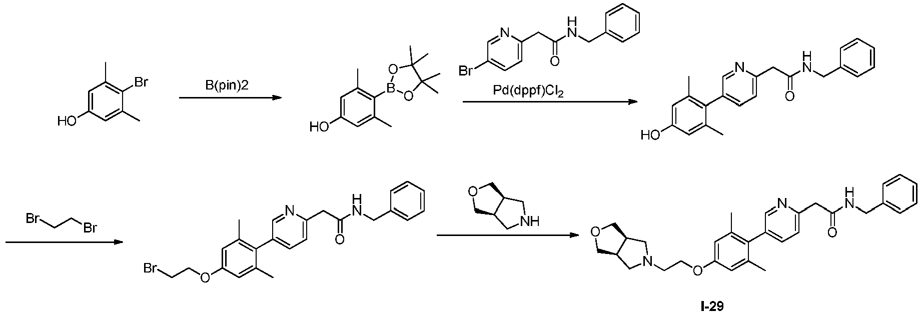

### Step 1: Preparation of 3,5-dimethyl-4-(4,4,5,5-tetramethyl-1,3,2-dioxaborolan-2-yl)phenol

4-Bromo-3,5-dimethylphenol (2 g, 9.95 mmol), potassium acetate (2.93 g, 29.8 mmol), bis(pinacolato)diboron (3.8 g, 14.9 mmol), and [1,1'-bis(diphenylphosphino)ferrocene]dichloropalladium(II) (364 mg, 0.49 mmol) were added to a reaction flask at room temperature. The system was replaced with nitrogen three times, then the solvent dioxane (40 mL) was added thereto, and the reaction mixture was heated and stirred at 100°C for 10 hours. After the completion of the reaction was detected by TLC, the reaction mixture was cooled to room temperature, concentrated by rotary evaporation to remove the solvent, and the residue was dissolved in ethyl acetate (100 mL), and washed with water (50 mL) and saturated brine (50 mL). The organic phase was dried over anhydrous sodium sulfate and subjected to rotary evaporation to obtain the crude product *N*-benzyl-2-(5-(4,4,5,5-tetramethyl-1,3,2-dioxaborolan-2-yl)pyridin-2-yl)acetamide (2.4 g, yield: 100%), which was directly used in the next step.

### Step 2: Preparation of N-benzyl-2-(5-(4-hydroxy-2,6-dimethylphenyl)pyridin-2-yl)acetamide

*N*-Benzyl-2-(5-bromopyridin-2-yl)acetamide (0.45 g, 1.5 mmol), the crude product N*N-*benzyl-2-(5-(4,4,5,5-tetramethyl-1,3,2-dioxaborolan-2-yl)pyridin-2-yl)acetamide (0.55 g, 2.2 mmol), potassium fluoride (0.34 g, 5.9 mmol), and [1,1'-bis(diphenylphosphino)ferrocene]dichloropalladium(II) (108 mg, 0.15 mmol) were added to a reaction flask at room temperature. The system was replaced with nitrogen three times, then the solvent dioxane (20 mL) was added thereto, and the reaction mixture was heated and stirred at 85°C for 10 hours. After the completion of the reaction was detected by TLC, the reaction mixture was cooled to room temperature, concentrated by rotary evaporation to remove the solvent to obtain a crude product, which was purified by preparative high-performance liquid chromatography to obtain *N*-benzyl-2-(5-(4-hydroxy-2,6-dimethylphenyl)pyridin-2-yl)acetamide (0.35 g, yield: 68%).

### Step 3: Preparation of N-benzyl-2-(5-(4-(2-bromoethoxy)-2,6-dimethylphenyl)pyridin-2-yl)acetamide

*N*-Benzyl-2-(5-(4-hydroxy-2,6-dimethylphenyl)pyridin-2-yl)acetamide (0.2 g, 0.58 mmol) and potassium carbonate (0.48 g, 3.46 mmol) were added to anhydrous acetonitrile (15 mL) at room temperature, heated to 85°C and stirred for 1 hour, then 1,2-dibromoethane (0.65 g, 3.46 mmol) was added thereto, and the reaction mixture was stirred at 85°C for another 10 hours. After the completion of the reaction was detected by TLC, the reaction mixture was cooled to room temperature, concentrated by rotary evaporation to remove the solvent to obtain a crude product, which was purified by silica gel column chromatography to obtain compound *N*-benzyl-2-(5-(4-(2-bromoethoxy)-2,6-dimethylphenyl)pyridin-2-yl)acetamide (0.2 g, yield: 76%).

### Step 4: N-benzyl-2-(5-(2,6-dimethyl-4-(2-((3aR,6aS)-tetrahydro-1H-furo[3,4-c]pyrrol-5(3H)-yl)ethoxy)phenyl)pyridin-2-yl)acetamide

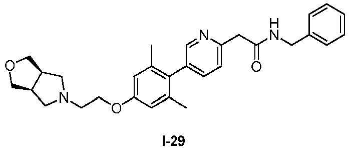

*N*-Benzyl-2-(5-(4-(2-bromoethoxy)-2,6-dimethylphenyl)pyridin-2-yl)acetamide (0.2 g, 0.47 mmol) was added to *N*,*N*-dimethylformamide (2 mL) at room temperature, then triethylamine (191 mg, 1.81 mmol), potassium iodide (312 mg, 1.81 mmol), and (3a*R*,6a*S*)-hexahydro-1*H*-furo[3,4-*c*]pyrrole (53 mg, 0.47 mmol) were added thereto, and the reaction mixture was stirred at 60°C for 8 hours. After the completion of the reaction was detected by TLC, the reaction mixture was purified by preparative high-performance liquid chromatography to obtain compound *N*-benzyl-2-(5-(2,6-dimethyl-4-(2-((3a*R*,6a*S*)-tetrahydro-1*H*-furo[3,4-*c*]pyrrol-5(3*H*)-yl)ethoxy)phenyl)pyridin-2-yl)acetamide (59.5 mg, yield: 26%).

¹H NMR (400 MHz,DMSO-d₆) δ 8.64 (t, 1H), 8.25 (d, 1H), 7.53 (dd, 1H), 7.42 (d, 1H), 7.34 - 7.19 (m, 4H), 6.74 (s, 2H), 4.32 (d, 2H), 4.06 (t, 2H), 3.77 - 3.66 (m, 4H), 3.39 (dd, 2H), 2.74 (t, 2H), 2.71 - 2.66 (m, 2H),2.66 - 2.60 (m, 2H), 2.40 (dd, 2H), 1.95 (s, 6H).

LC-MS, M/Z (ESI): 486.2 [M+H]⁺.

### Example 30: Preparation of compound 1-30

The synthetic route is as follows:

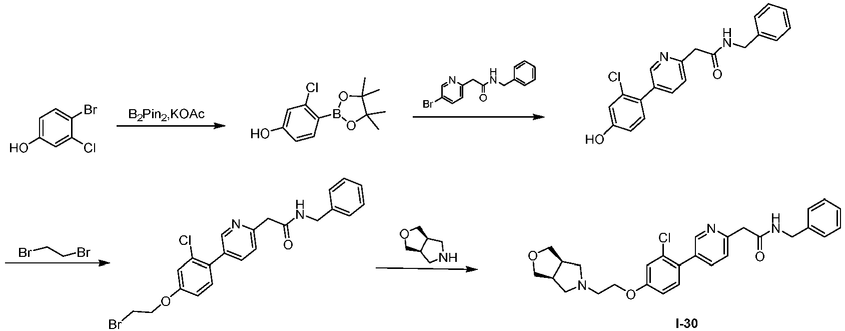

### Step 1: Preparation of 3-chloro-4-(4,4,5,5-tetramethyl-1,3,2-dioxaborolan-2-yl)phenol

Compounds 4-bromo-3-chlorophenol (5.0 g, 24.10 mmol), K₂CO₃ (9.99 g, 72.3 mmol), bis(pinacolato)diboron (12.24 g, 48.2 mmol), and [1,1'-bis(diphenylphosphino)ferrocene]dichloropalladium(II) (1.76 g, 2.41 mmol) were added to dioxane (80 mL) at room temperature, and the reaction mixture was stirred at 85°C for 12 hours under a nitrogen atmosphere. After the reaction was completed, the reaction mixture was diluted with water (100 mL) and extracted with ethyl acetate (50 mL × 3), and the phases were separated. The organic phases were combined, dried over anhydrous sodium sulfate, filtered, concentrated, and the residue was purified by column chromatography (petroleum ether: ethyl acetate (V/V) = 2:3) to obtain compound 3-chloro-4-(4,4,5,5-tetramethyl-1,3,2-dioxaborolan-2-yl)phenol (2.6 g, white solid, yield: 42.4%).

### Step 2: Preparation of N-benzyl-2-(5-(2-chloro-4-hydroxyphenyl)pyridin-2-yl)acetamide

Compounds 3-chloro-4-(4,4,5,5-tetramethyl-1,3,2-dioxaborolan-2-yl)phenol (2.1 g, 8.25 mmol), *N*-benzyl-2-(5-bromopyridin-2-yl)acetamide (2.77 g, 9.08 mmol), potassium carbonate (3.42 g, 24.75 mmol), and [1,1'-bis(diphenylphosphino)ferrocene]dichloropalladium(II) (0.065 g, 0.089 mmol) were added to dioxane (30 mL). The system was replaced with nitrogen, and the reaction mixture was heated to 85°C and stirred for 10 hours. The reaction mixture was diluted with water (100 mL) and extracted with ethyl acetate (30 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 3:2) to obtain compound *N*-benzyl-2-(5-(2-chloro-4-hydroxyphenyl)pyridin-2-yl)acetamide (2.6 g, white solid, yield: 42.4%).

### Step 3: N-benzyl-2-(5-(4-(2-bromoethoxy)-2-chlorophenyl)pyridin-2-yl)acetamide

*N*-Benzyl-2-(5-(2-chloro-4-hydroxyphenyl)pyridin-2-yl)acetamide (0.55 g, 1.56 mmol), 1,2-dibromoethane (1.75 g, 9.35 mmol), and potassium carbonate (1.29 g, 9.35 mmol) were placed in acetonitrile (6 mL), and the reaction mixture was heated to 85°C and stirred for 12 hours. The reaction mixture was diluted with water (5 mL) and extracted with ethyl acetate (5 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 3:1) to obtain compound N benzyl-2-(5-(4-(2-bromoethoxy)-2-chlorophenyl)pyridin-2-yl)acetamide (0.4 g, white solid, yield: 55.8%).

### Step 4: N-benzyl-2-(5-(2-chloro-4-(2-((3aR,6aS)-tetrahydro-1H-furo[3,4-c]pyrrol-5(3H)-yl)ethoxy)phenyl)pyridin-2-yl)acetamide (I-30)

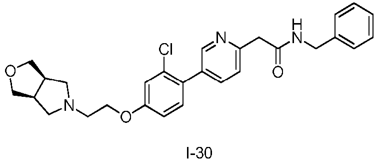

*N*-Benzyl-2-(5-(4-(2-bromoethoxy)-2-chlorophenyl)pyridin-2-yl)acetamide (0.2 g, 0.435 mmol), (3a*R*,6a*S*)-hexahydro-1*H*-furo[3,4-*c*]pyrrole (0.054 g, 0.479 mmol), and potassium carbonate (0.180 g, 1.30 mmol) were dissolved in acetonitrile (3 mL), and the reaction mixture was heated to 85°C and stirred for 12 hours. The reaction mixture was diluted with water (5 mL) and extracted with ethyl acetate (5 mL × 3). The phases were separated, and the organic phases were combined. The organic phase was dried over anhydrous sodium sulfate, filtered, concentrated, and the residue was purified by preparative high-performance liquid chromatography to obtain compound *N-*benzyl-2-(5-(2-chloro-4-(2-((3a*R*,6a*S*)-tetrahydro-1*H-*furo[3,4-*c*]pyrrol-5(3*H*)-yl)ethoxy)phenyl)pyridin-2-yl)acetamide (I-30) (0.1 g, yield: 46.7%).

¹H NMR (400 MHz, CDCl₃) δ 8.56 (d, 1H), 7.75 (dd, 1H), 7.68 (s, 1H), 7.28 (ddt, 6H), 7.06 (d, *J =* 2.5 Hz, 1H), 6.91 (dd, 1H), 4.50 (d, 2H), 4.13 (t, 2H), 3.83 (s, 2H), 3.77 (dd, 2H), 3.65 - 3.60 (m, 2H), 2.89 (t, 2H), 2.87 - 2.80 (m, 4H), 2.41 (dd, 2H).

LC-MS, M/Z (ESI): 492.3 [M+H]⁺.

### Example 31: Preparation of compound 1-31

The synthetic route is as follows:

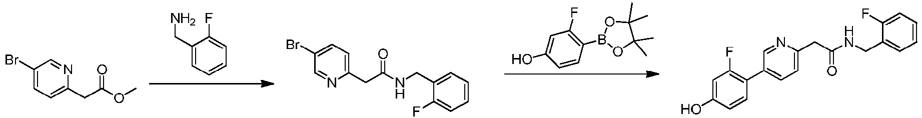

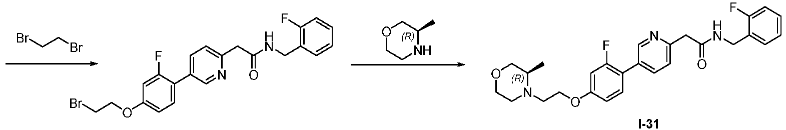

### Step 1: Preparation of 2-(5-bromopyridin-2-yl)-N-(2-fluorobenzyl)acetamide

Methyl 2-(5-bromopyridin-2-yl)acetate (30.0 g, 130.40 mmol) and 2-fluorobenzylamine (48.96 g, 391.20 mmol) were placed in a round-bottomed flask, heated to 120°C and stirred for 12 hours. The reaction mixture was cooled to room temperature, added with petroleum ether/ethyl acetate (V/V = 3:1, 500 mL), slurried at room temperature for 2 hours, and filtered. The filter cake was collected and dried under vacuum to obtain 2-(5-bromopyridin-2-yl)-N-(2-fluorobenzyl)acetamide (35.2 g, yield: 83.0%).

LC-MS, M/Z (ESI): 323.1 [M+H]⁺.

### Step 2: Preparation of 2-(5-(2-fluoro-4-hydroxyphenyl)pyridin-2-yl)-N-(2-fluorobenzyl)acetamide

3-Fluoro-4-(4,4,5,5-tetramethyl-1,3,2-dioxaborolan-2-yl)phenol (5.5 g, 21.61 mmol), 2-(5-bromopyridin-2-yl)-*N*-(2-fluorobenzyl)acetamide (6.98 g, 21.61 mmol), potassium carbonate (8.96 g, 64.83 mmol), and [1,1'-bis(diphenylphosphino)ferrocene]dichloropalladium(II) (1.96 g, 2.73 mmol) were dissolved in dioxane (120 mL) and water (3 mL), and the reaction mixture was heated to 85°C and stirred for 12 hours. The reaction mixture was diluted with water (200 mL) and extracted with ethyl acetate (200 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 3:1) to obtain 2-(5-(2-fluoro-4-hydroxyphenyl)pyridin-2-yl)-*N*-(2-fluorobenzyl)acetamide (4.0 g, yield: 58.4%).

LC-MS, M/Z (ESI): 355.1 [M+H]⁺.

### Step 3: Preparation of 2-(5-(4-(2-bromoethoxy)-2-phenyl)pyridin-2-yl)-N-(2-fluorobenzyl)acetamide

2-(5-(2-Fluoro-4-hydroxyphenyl)pyridin-2-yl)-*N*-(2-fluorobenzyl)acetamide (5.0 g, 13.48 mmol), 1,2-dibromoethane (15.20 g, 80.90 mmol), and potassium carbonate powder (11.18 g, 80.90 mmol) were placed in acetonitrile (50.0 mL), and the reaction mixture was heated to 85°C and stirred for 12 hours. The reaction mixture was diluted with water (100 mL) and extracted with ethyl acetate (50 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 1:1) to obtain 2-(5-(4-(2-bromoethoxy)-2-phenyl)pyridin-2-yl)-*N*-(2-fluorobenzyl)acetamide (3.0 g, yield: 58.0%).

LC-MS, M/Z (ESI): 461.1 [M+H]⁺.

### Step 4: Preparation of (R)-2-(5-(2-fluoro-4-(2-(3-methylmorpholino)ethoxy)phenyl)pyridin-2-yl)-N-(2-fluorobenzyl)acetamide (I-31)

2-(5-(4-(2-Bromoethoxy)-2-fluorophenyl)pyridin-2-yl)-*N-*(2-fluorobenzyl)acetamide (0.6 g, 1.3 mmol) was added to MeCN (20 mL) at room temperature, then K₂CO₃ (540 mg, 3.9 mmol), potassium iodide (26 mg, 0.13 mmol), and (*R*)-3-methylmorpholine (170 mg, 1.7 mmol) were sequentially added thereto, and the reaction mixture was stirred at 80°C for 8 hours. After the disappearance of the material was monitored by TLC, the reaction mixture was concentrated, and the residue was purified by column chromatography on silica gel (DCM: MeOH (V/V) = 20:1) to obtain compound (*R*)-2-(5-(2-fluoro-4-(2-(3-methylmorpholino)ethoxy)phenyl)pyridin-2-yl)-*N*-(2-fluorobenzyl)acetamide (I-31) (217.2 mg, yield: 34.6%).

¹H NMR (400 MHz, DMSO-d₆) δ 8.67 (s, 1H), 7.84 - 7.69 (m, 2H), 7.37 - 7.27 (m, 3H), 7.25 - 7.20 (m, 1H), 7.10 - 6.99 (m, 2H), 6.83 - 6.70 (m, 2H), 4.51 (dd, 2H), 4.16 - 4.06 (m, 2H), 3.85 - 3.78 (m, 3H), 3.73 - 3.62 (m, 2H), 3.27 (dd, 1H), 3.20 - 3.11 (m,1H), 2.90 - 2.81 (m, 1H), 2.80 - 2.70 (m, 1H), 2.62 - 2.51 (m, 2H), 1.04 (d, 3H).

LC-MS, M/Z (ESI): 482.1 [M+H]⁺.

### Example 32: Preparation of compound 1-32

The synthetic route is as follows:

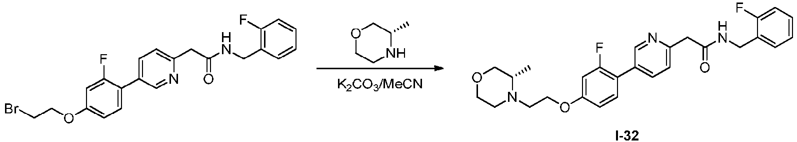

2-(5-(4-(2-Bromoethoxy)-2-fluorophenyl)pyridin-2-yl)-*N-*(2-fluorobenzyl)acetamide (2.0 g, 4.19 mmol) and (*S*)-3-methylmorpholine (0.47 g, 4.19 mmol) were dissolved in acetonitrile (20.0 mL), then potassium carbonate (1.74 g, 12.56 mmol) was added thereto, and the reaction mixture was heated to 85°C and stirred for 12 hours. The reaction mixture was cooled to room temperature, diluted with water (100 mL), and extracted with ethyl acetate (50 mL × 3). The phases were separated, and the organic phases were combined. The organic phase was dried over anhydrous sodium sulfate, filtered, and concentrated. The residue was purified by column chromatography on silica gel (petroleum ether: ethyl acetate (V/V) = 1:1) to obtain (*S*)-2-(5-(2-fluoro-4-(2-(3-methylmorpholino)ethoxy)phenyl)pyridin-2-yl)-*N*-(2-fluorobenzyl)acetamide (I-32) (200 mg, yield: 10%).

¹H NMR (400 MHz, CDCl₃) δ 8.67 (s, 1H), 7.83 - 7.78 (m, 1H), 7.77 - 7.70 (m, 1H), 7.37 - 7.19 (ddd, 4H), 7.10 - 6.99 (m, 2H), 6.78 (ddd, 2H), 4.53 (d, 2H), 4.16 - 4.08 (m, 2H), 3.86 - 3.75 (m, 3H), 3.73 - 3.62 (m, 2H), 3.27 (dd, 1H), 3.16 (dt, 1H), 2.85 (dt, 1H), 2.75 (dt, 1H), 2.61 - 2.50 (m, 2H), 1.04 (d, 3H).

LC-MS, M/Z (ESI): 482.5 [M+H]⁺.

### Example 33: Preparation of compound 1-33

The synthetic route is as follows:

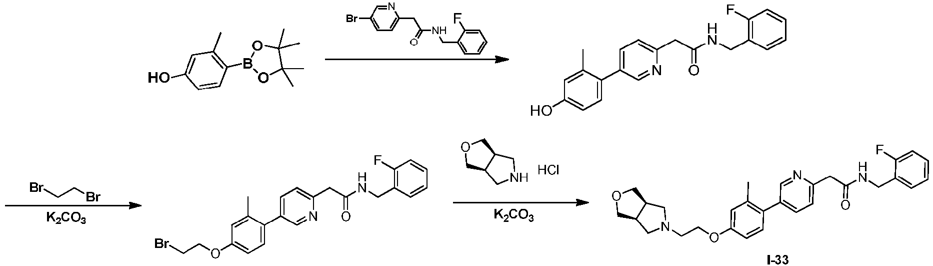

### Step 1: N-(2-fluorobenzyl)-2-(5-(4-hydroxy-2-methylphenyl)pyridin-2-yl)acetamide

Compounds 3-methyl-4-(4,4,5,5-tetramethyl-1,3,2-dioxaborolan-2-yl)phenol (1.15 g, 4.92 mmol) and 2-(5-bromopyridin-2-yl)-*N-*(2-fluorobenzyl)acetamide (1.0 g, 3.33 mmol) were added to 1,4-dioxane/water (10 mL/1 mL) at room temperature, then potassium fluoride (0.760 g, 13.1 mmol) was added thereto, and [1,1'-bis(diphenylphosphino)ferrocene]dichloropalladium(II) (117 mg, 0.16 mmol) was added thereto under a nitrogen atmosphere. The reaction mixture was heated to 90°C and stirred for 10 hours. The reaction mixture was cooled to room temperature, diluted with water (50 mL), and extracted with ethyl acetate (20 mL × 3), and the phases were separated. The organic phases were combined, dried over anhydrous sodium sulfate, filtered, concentrated, and the residue was purified by column chromatography on silica gel (DCM: MeOH (V/V) = 95:5) to obtain compound *N*-(2-fluorobenzyl)-2-(5-(4-hydroxy-2-methylphenyl)pyridin-2-yl)acetamide (840 mg, yield: 76%).

LC-MS, M/Z (ESI): 351.4 [M+H]⁺.

### Step 2: Preparation of 2-(5-(4-(2-bromoethoxy)-2-methylphenyl)pyridin-2-yl)-N-(2-fluorobenzyl)acetamide

*N*-(2-Fluorobenzyl)-2-(5-(4-hydroxy-2-methylphenyl)pyridin-2-yl)acetamide (0.8 g, 2.3 mmol) and potassium carbonate (1.89 g, 13.7 mmol) were added to anhydrous acetonitrile (15 mL) at room temperature, heated to 80°C and stirred for 1 hour, then 1,2-dibromoethane (2.55 g, 13.7 mmol) was added thereto, and the reaction mixture was stirred at 80°C for another 10 hours. The reaction mixture was cooled to room temperature, concentrated to remove the solvent, and the crude product was purified by silica gel column chromatography (DCM: MeOH (V/V) = 20:1) to obtain 2-(5-(4-(2-bromoethoxy)-2-methylphenyl)pyridin-2-yl)-*N*-(2-fluorobenzyl)acetamide (0.6 g, yield: 69%).

### Step 3: Preparation ofN-(2-fluorobenzyl)-2-(5-(2-methyl-4-(2-((3aR,6aS)-tetrahydro-1H-furo[3,4-c]pyrrol-5(3H)-yl)ethoxy)phenyl)pyridin-2-yl)acetamide

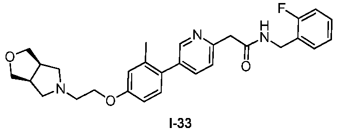

2-(5-(4-(2-Bromoethoxy)-2-methylphenyl)pyridin-2-yl)-*N-*(2-fluorobenzyl)acetamide (0.6 g, 1.31 mmol) was added to MeCN (20 mL) at room temperature, then K₂CO₃ (543 mg, 3.94 mmol), potassium iodide (33 mg, 0.2 mmol), and (3a*R*,6a*S*)-hexahydro-1*H*-furo[3,4-*c*]pyrrole hydrochloride (255 mg, 1.7 mmol) were added thereto, and the reaction mixture was stirred at 80°C for 8 hours. The reaction mixture was concentrated under reduced pressure, and the residue was purified by column chromatography on silica gel (DCM: MeOH (V/V) = 20:1) to obtain *N*-(2-fluorobenzyl)-2-(5-(2-methyl-4-(2-((3a*R*,6a*S*)-tetrahydro-1*H*-furo[3,4-*c*]pyrrol-5(3*H*)-yl)ethoxy)phenyl)pyridin-2-yl)acetamide (I-33) (248.3 mg, yield: 39%).

¹H NMR (400 MHz, DMSO-d₆) δ 8.63 (t, 1H), 8.43 - 8.39 (m, 1H), 7.69 (m, 1H), 7.40 - 7.26 (m, 3H), 7.20 - 7.08 (m, 3H), 6.91 (d, 1H), 6.85 (m, 1H), 4.34 (d, 2H), 4.07 (t, 2H), 3.75 - 3.64 (m, 4H), 3.38 (m, 2H), 2.74 (t, 2H), 2.70 - 2.60 (m, 4H), 2.38 (dd, 2H), 2.21 (s, 3H).

LC-MS, M/Z (ESI): 490.4 [M+H]⁺.

### Example 34: Preparation of compound I-34

The synthetic route is as follows:

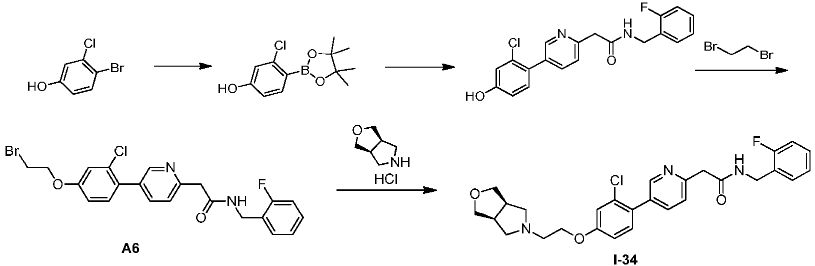

### Step 1: Preparation of 3-chloro-4-(4,4,5,5-tetramethyl-1,3,2-dioxaborolan-2-yl)phenol

4-Bromo-3-chlorophenol (6.0 g, 28.92 mmol), bis(pinacolato)diboron (11.02 g, 43.38 mmol), potassium acetate (8.582 g, 86.77 mmol), and [1,1'-bis(diphenylphosphino)ferrocene]dichloropalladium(II) (1.96 g, 2.73 mmol) were dissolved in dioxane (120 mL) and water (3 mL), and the reaction mixture was heated to 85°C and stirred for 12 hours. The reaction mixture was diluted with water (200 mL) and extracted with ethyl acetate (200 mL × 3). The phases were separated, and the organic phases were combined. The organic phase was dried over anhydrous sodium sulfate, filtered, concentrated, and the residue was purified by column chromatography on silica gel (petroleum ether: ethyl acetate (V/V) = 3:1) to obtain 3-chloro-4-(4,4,5,5-tetramethyl-1,3,2-dioxaborolan-2-yl)phenol as a white solid (5.5 g, yield: 74.0%).

LC-MS, M/Z (ESI): 255.1 [M+H]⁺.

### Step 2: Preparation of 2-(5-(2-chloro-4-hydroxyphenyl)pyridin-2-yl)-N-(2-fluorobenzyl)acetamide

3-Chloro-4-(4,4,5,5-tetramethyl-1,3,2-dioxaborolan-2-yl)phenol (5.5 g, 21.61 mmol), 2-(5-bromopyridin-2-yl)-*N*-(2-fluorobenzyl)acetamide (6.98 g, 21.61 mmol), potassium carbonate (8.96 g, 64.83 mmol), and [1,1'-bis(diphenylphosphino)ferrocene]dichloropalladium(II) (1.96 g, 2.73 mmol) were placed in 1,4-dioxane (120 mL) and water (3 mL), and the reaction mixture was heated to 85°C and stirred for 12 hours. The reaction mixture was diluted with water (200 mL) and extracted with ethyl acetate (200 mL × 3). The phases were separated, and the organic phases were combined. The organic phase was dried over anhydrous sodium sulfate, filtered, and concentrated. The residue was purified by column chromatography on silica gel (petroleum ether: ethyl acetate (V/V) = 3:1) to obtain 2-(5-(2-chloro-4-hydroxyphenyl)pyridin-2-yl)-*N-*(2-fluorobenzyl)acetamide as a white solid (5.0 g, yield: 62.4%).

LC-MS, M/Z (ESI): 371.1 [M+H]⁺.

### Step 3: Preparation of 2-(5-(4-(2-bromoethoxy)-2-chlorophenyl)pyridin-2-yl)-N-(2-fluorobenzyl)acetamide (A6)

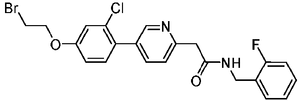

2-(5-(2-Chloro-4-hydroxyphenyl)pyridin-2-yl)-*N-*(2-fluorobenzyl)acetamide (5.0 g, 13.48 mmol), 1,2-dibromoethane (15.20 g, 80.90 mmol), and potassium carbonate powder (11.18 g, 80.90 mmol) were placed in acetonitrile (50 mL), and the reaction mixture was heated to 85°C and stirred for 12 hours. The reaction mixture was diluted with water (100 mL) and extracted with ethyl acetate (50 mL × 3). The phases were separated, and the organic phases were combined. The organic phase was dried over anhydrous sodium sulfate, filtered, and concentrated. The residue was purified by a chromatography column (petroleum ether: ethyl acetate (V/V) = 1:1) to obtain 2-(5-(4-(2-bromoethoxy)-2-chlorophenyl)pyridin-2-yl)-*N*-(2-fluorobenzyl)acetamide (A6, 4.0 g, yield: 62.1%).

LC-MS, M/Z (ESI): 477.1 [M+H]⁺.

### Step 4: Preparation of 2-(5-(2-chloro-4-(2-((3aR,6aS)-tetrahydro-1H-furo[3,4-c]pyrrol-5(3H)-yl)ethoxy)phenyl)pyridin-2-yl)-N-(2-fluorobenzyl)acetamide

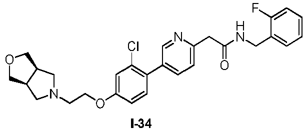

2-(5-(4-(2-Bromoethoxy)-2-chlorophenyl)pyridin-2-yl)-*N*-(2-fluorobenzyl)acetamide (A6, 2.0 g, 4.19 mmol), (3a*R*,6a*S*)-hexahydro-1*H*-furo[3,4-c]pyrrole hydrochloride (0.63 g, 4.19 mmol), and potassium carbonate (1.74 g, 12.56 mmol) were dissolved in acetonitrile (20.0 mL), and the reaction mixture was heated to 85°C and stirred for 12 hours. The reaction mixture was diluted with water (100 mL) and extracted with ethyl acetate (50 mL × 3). The phases were separated, and the organic phases were combined. The organic phase was dried over anhydrous sodium sulfate, filtered, and concentrated. The residue was sequentially purified by a chromatography column (petroleum ether: ethyl acetate (V/V) = 1:1) and purified by preparative high-performance liquid chromatography to obtain 2-(5-(2-chloro-4-(2-((3a*R*,6a*S*)-tetrahydro-1*H*-furo[3,4-c]pyrrol-5(3*H*)-yl)ethoxy)phenyl)pyridin-2-yl)-*N*-(2-fluorobenzyl)acetamide (I-34, 150 mg, yield: 7%).

¹H NMR (400 MHz, CDCl₃) δ 8.57 (d, 1H), 7.80 - 7.69 (m, 2H), 7.37 - 7.27 (m, 2H), 7.25 - 7.19 (m, 2H), 7.10 - 6.98 (m, 3H), 6.91 (dd, 1H), 4.54 (d, 2H), 4.14 (t, 2H), 3.82 (s, 2H), 3.79 - 3.72 (m, 2H), 3.64 (dd, 2H), 2.97 - 2.83 (m, 6H), 2.41 (dd, 2H).

The control compound I (trade name: Tirbanibulin) in the test examples of the present disclosure is a dual-action inhibitor of Src kinase and tubulin polymerization, which has been approved by the FDA and the European Union for the topical treatment of actinic keratosis on the face or scalp. The preparation of control compound I refers to patent WO 2008/002676 A2, and the control compound I has a structure as follows:

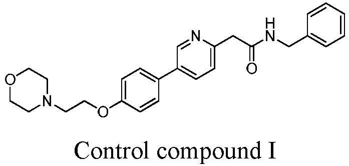

### Test example 1: Inhibition test of tubulin monomer polymerization

Tubulin polymerization assay kit (cytoskeleton, Cat. # BK011P) was used to conduct the compound inhibition test of tubulin monomer polymerization.

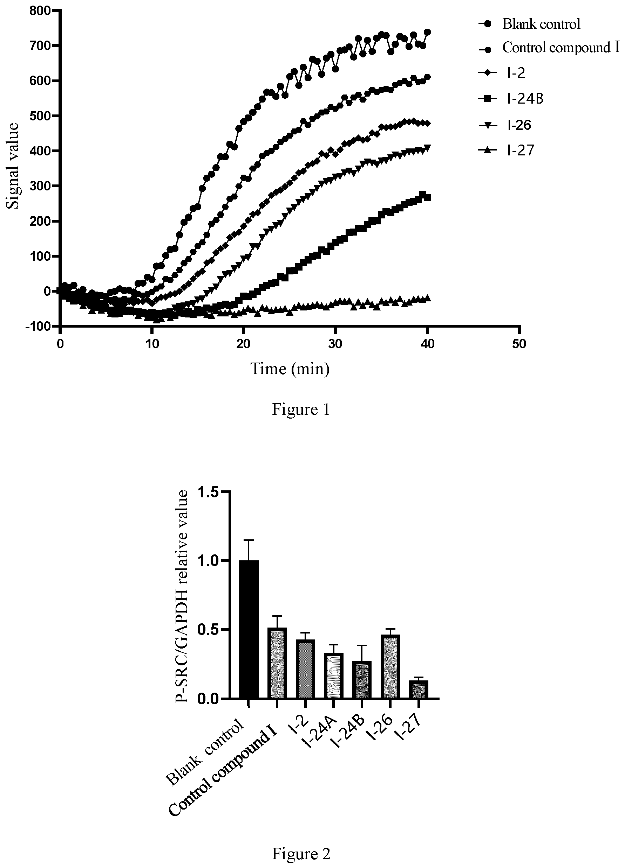

Before the test, the 96-well plate (Corning Costar, Cat. # 3686) supplied with the kit was first placed in a microplate reader (MD, SpectraMax M5) and heated to 37°C, maintained for 10 minutes. The 96-well plate was then removed, and 5 µL of 12.5 µM compound solution or blank solution was added thereto. The 96-well plate was then placed back into the microplate reader and incubated at 37°C for 1 minute to heat the compound solution to 37°C. The 96-well plate was removed and 50 µL of reaction mixture prepared according to the supplier's instructions was quickly added to each well, completing the sample addition within 1 minute to avoid the formation of bubbles. The 96-well plate was then immediately placed back into the microplate reader, shaken for 5 seconds, and using the Kinetic mode, continuous detection was carried out at 37°C for 30 to 60 minutes under the conditions of excitation light at 360 nm and emission light at 420 nm, with detection every 30 seconds. Taking the detection time as the X-axis and the fluorescence signal value as the Y-axis, the tubulin monomer polymerization curve was obtained. As shown in Figure 1 and Table 1, the larger the Vmax value and the higher the maximum fluorescence signal value of the polymerization curve, the lower the inhibitory efficiency of the compound.

**Table 1**

| Compound | Blank control | Control compound I | I-2 | I-24B | I-26 | I-27 |
|---|---|---|---|---|---|---|
| Maximum signal value | 739 | 611 | 478 | 266 | 408 | 0 |

The test results of compounds inhibiting tubulin polymerization are shown in Figure 1 and Table 1. The results show that the Vmax values and maximum fluorescence signal values of the polymerization curves corresponding to the compounds of the present disclosure are significantly smaller, indicating that the compounds of the present disclosure can significantly inhibit the polymerization of tubulin monomers and have the better inhibitory activity compared with the control compound I.

### Test example 2: Inhibition test of compounds on cell proliferation

Human cutaneous squamous cell carcinoma cell A-431 (ATCC, CRL-1555) and actinic keratosis patient-derived cell HT 297.T (ATCC, CRL-7782) proliferation assays were used to detect the inhibitory effect of small molecular compounds on cell proliferation.

A-431 cells and HT 297.T cells were cultured in DMEM medium containing 10% fetal bovine serum and grown at 37°C in a 5% CO₂ incubator. Cells on logarithmic phase were seeded into a 96-well cell culture plate at 1000 cells/well, 100 µL per well, and then cultured overnight at 37°C in a 5% CO₂ incubator. The next day, 100 µL of gradient-diluted 2× test compound solution was added to each well, with DMSO serving as a positive control, and 10 µM staurosporine (Aladdin, S102392) was set as a negative control group. After the addition of the compounds, the culture plate was incubated for another 4 days at 37°C in a 5% CO₂ incubator. After incubation, the Steady-Glo^{®} luciferase assay system (Promega, G9243) was used according to the supplier's instructions to measure the fluorescence signal value on the Envision 2104 Multilabel Reader. The inhibition rate was calculated by the following formula, then the curve was plotted with the Log value of the inhibitor concentration as the X-axis and the inhibition rate as the Y-axis, and the IC₅₀ was calculated using Graphpad 8.0. Inhibition % = (positive control group signal - test well signal) / (positive control group signal - negative control group signal) * 100

**Table 2: Inhibition results of test compounds on proliferation activity of A-431 cells**

| Test compound | A-431 IC₅₀ (nM) | Test compound | A-431 IC₅₀ (nM) |
|---|---|---|---|
| Control compound I | 40.30 | I-26 | 13.65 |
| I-1 | 11.61 | I-27 | 3.1 |
| I-2 | 22.60 | I-29 | 45.06 |
| I-3 | 14.35 | I-30 | 9.12 |
| I-4 | 8.67 | I-31 | 10.86 |
| I-5 | 16.28 | I-32 | 9.22 |
| I-18 | 22.34 | I-33 | 49.38 |
| I-24A | 6.74 | I-34 | 14.85 |
| I-24B | 6.47 | | |

**Table 3: Inhibition results of test compounds on proliferative activity of HT 297.T cells**

| Test compound | HT 297.T IC₅₀ (nM) |
|---|---|
| Control compound I | 85.38 |
| I-2 | 41.31 |
| I-4 | 21.94 |
| I-24A | 8.13 |
| I-24B | 8.51 |

The results of the cell proliferation inhibition test of the compounds are shown in Table 2 and Table 3. The results show that the compounds of the present disclosure can significantly inhibit cell proliferation, especially the inhibitory effect on HT 297.T cells derived from patients with actinic keratosis is significantly better than that of the control compound I, which indicates that the compounds of the present disclosure have better therapeutic effects on actinic keratosis.

### Test example 3: Thermodynamic solubility test

Phosphate buffer saline (PBS) with a pH of 7.4, FeSSIF solution with a pH of 6.5, and FaSSGF solution with a pH of 1.6 were prepared. The compound was accurately weighed and added to prepared phosphate buffer saline with a pH of 7.4, FeSSIF solution with a pH of 6.5, and FaSSGF solution with a pH of 1.6 to prepare into a solution with a concentration of 4 mg/mL. The solution was shaken at a speed of 1000 rpm for 1 hour, and then incubated overnight at room temperature. The incubated solution was centrifuged at 12,000 rpm for 10 minutes to remove undissolved particles, and the supernatant was transferred to a new centrifuge tube. After the supernatant was appropriately diluted, an acetonitrile solution containing internal standard was added, and a standard curve prepared with the same matrix was used for quantification.

**Table 4: Thermodynamic solubility test results**

| Test compound | Solubility (µg/mL) | |
|---|---|---|
| | FeSSIF (pH 6.5) | PBS (pH 7.4) |
| Control compound I | 60.50 | 9.770 |
| I-2 | 649.6 | 101.1 |
| I-24B | 69.76 | 11.32 |
| I-26 | 479.5 | 22.43 |
| I-27 | 833.2 | 85.67 |

The results of the thermodynamic solubility test are shown in Table 4. The results show that compared with the control compound I, the compounds of the present disclosure have greater thermodynamic solubility under neutral conditions and have good druggability.

### Test example 4: Pharmacokinetic test

For the pharmacokinetic test in mice, 3 male ICR mice, 20 to 25 g, were used, fasted overnight, and administered by tail vein injection (1 mg/kg or 5 mg/kg). Blood was collected before the administration, and at 15 min, 30 min, 1 hour, 2 hours, 4 hours, 8 hours, and 24 hours after the administration. Another 3 mice were taken and administered by oral gavage (5 mg/kg), and blood was collected before the administration, and at 15 min, 30 min, 1 hour, 2 hours, 4 hours, 8 hours, and 24 hours after the administration. Blood samples were centrifuged at 6800 g at 2 to 8°C for 6 min, and plasma was collected and stored at -80°C. The plasma at each time point was taken and added with an acetonitrile solution containing internal standard in 3 to 5 times the amount. The mixture was vortexed and mixed for 1 min, and centrifuged at 13,000 rpm at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

**Table 5: Results 1 of pharmacokinetic tests in mice**

| Test compound | Pharmacokinetic parameters of mice | |
|---|---|---|
| | Intravenous injection administration (1 mg/kg) | |
| | CL (L/h/kg) | AUC₀₋ₜ (h * ng/mL) |
| Control compound I | 1.88 | 525 |
| I-1 | 2.86 | 299 |
| I-2 | 2.07 | 430 |

**Table 6: Results 2 of pharmacokinetic tests in mice**

| Test compound | Pharmacokinetic parameters of mice | |
|---|---|---|
| | Intravenous injection administration (5 mg/kg) | |
| | CL (L/h/kg) | AUC₀₋ₜ (h * ng/mL) |
| Control compound I | 0.95 | 5253 |
| I-5 | 1.96 | 2627 |
| I-24A | 1.99 | 2518 |
| I-24B | 1.84 | 2779 |
| I-30 | 5.83 | 852 |

**Table 7: Results 3 of pharmacokinetic tests in mice**

| Test compound | Pharmacokinetic parameters of mice | |
|---|---|---|
| | Oral gavage administration (5 mg/kg) | |
| | Cₘₐₓ (ng/mL) | AUC₀₋ₜ (h * ng/mL) |
| Control compound I | 1010 | 1819 |
| I-2 | 322 | 443 |
| I-24A | 1193 | 1090 |
| I-24B | 1234 | 1420 |
| I-30 | 364 | 508 |

The results of the pharmacokinetic tests in mice are shown in Table 5, Table 6, and

Table 7. The results show that compared with the control compound I, the compounds of the present disclosure have rapid metabolism in mice, low potential systemic toxicity, and good druggability.

### Test example 5: Human liver microsome stability test

The human liver microsome stability test was performed by incubating the compound and human liver microsomes *in vitro.* First, the compound to be tested was prepared as a 10 mM stock solution in DMSO solvent, and then the compound was diluted to 0.5 mM with acetonitrile. Human liver microsomes (Corning) were diluted with PBS into a microsome/buffer solution, and the solution was used to dilute 0.5 mM compound into a working solution, in which the concentration of the compound was 1.5 µM, and the concentration of human liver microsomes was 0.75 mg/mL. A deep-well plate was taken, 30 µL of the working solution was added per well, and then 15 µL of pre-heated 6 mM NADPH solution was added thereto to initiate a reaction, and the reaction was incubated at 37°C. At 0 min, 5 min, 15 min, 30 min, and 45 min of the incubation, 135 µL of acetonitrile was added to the corresponding wells to terminate the reaction. After the reaction was terminated with acetonitrile at the last time point of 45 min, the deep-well plate was vortexed and shaken for 10 min (600 rpm), and then centrifuged for 15 min. After centrifugation, the supernatant was collected, and added with purified water in a ratio of 1:1 to perform LC-MS/MS detection. A ratio of a peak area of compound to a peak area of internal standard at each time point was obtained, and the peak area ratios of the compound at 5 min, 15 min, 30 min, and 45 min were compared with the peak area ratio at 0 min to calculate the remaining percentage of the compound at each time point. T_{1/2} was calculated by using Graphpad 5 software.

**Table 8: Results of human liver microsome stability test**

| Compound No. | T_{1/2} (min) |
|---|---|
| Control compound I | 32.23 |
| I-2 | 17.24 |
| I-4 | 47.47 |
| I-24B | 13.89 |
| I-26 | 42.78 |
| I-30 | 10.69 |

The results of the human liver microsome stability test are shown in Table 8. The results show that the compounds of the present disclosure are metabolized quickly by human liver microsomes, with low potential systemic toxicity and good druggability.

### Test example 6: Inhibition of Src signaling pathway by compounds

The Western Blot method was used to detect the compound's inhibition of p-SRC in A-431 skin cancer cells (ATCC, CRL-1555) to evaluate the inhibitory effect of the compound on the SRC signaling pathway.

A-431 cells were cultured in DMEM medium containing 10% fetal bovine serum and grown at 37°C in a 5% CO₂ incubator. 500,000 cells/well were seeded in a 12-well cell culture plate and cultured overnight at 37°C in a 5% CO₂ incubator. The next day, the culture medium was replaced and different concentrations of the compound were added, with DMSO serving as a control. After the addition of the compounds, the culture plate was cultured for another 24 hours at 37°C in a 5% CO₂ incubator. The cells were washed once with PBS, and lysed with RIPAlysis buffer (R0010, Solarbio) at low temperature for 30 minutes. Afterwards, the protein lysate was collected into a centrifuge tube, centrifuged at 14,000 g at 4°C for 10 minutes, and the supernatant was transferred to a new tube. The BCA protein concentration assay kit (P0009, Beyotime Biotechnology) was used to detect and calculate the protein concentration. A 5× protein loading buffer was used to quantify the protein of each sample consistently. The samples were boiled at 100°C for 5 minutes, and subjected to western blot to detect the level of p-SRC. The p-SRC antibody (Ab185617) was purchased from Abcam, and the GAPDH antibody (60004-1-Ig) was purchased from Proteintech.

The test results are shown in Figure 2. The results show that the compounds of the present disclosure can significantly inhibit the phosphorylation of SRC at 80 nM, have better p-SRC inhibitory activity compared with the control compound I, and can block the SRC downstream signaling pathway.

Although the examples of the present disclosure are illustrated and described above, it can be understood that the above examples are illustrative and should not be construed as limiting the present disclosure. Those skilled in the art can make changes, modifications, substitutions, and variations based on the above examples within the scope of the present disclosure.

## Claims

1. A diaryl compound of formula I, a tautomer, a stereoisomer, a solvate, a pharmaceutically acceptable salt, or a prodrug thereof, having a structure of: 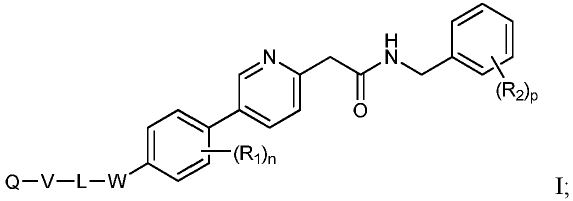
wherein W is selected from: -O-, -S-, -NH-, -N(C₁-C₆ alkyl)-;
L is C₁-C₆ alkylene;
V is absent or selected from: -O-, -S-, -NH-, -N(C₁-C₆ alkyl)-;
when V is absent, Q is ring A which is unsubstituted or substituted by m R₃, or ring B which is unsubstituted or substituted by m R₃;
when V is selected from: -O-, -S-, -NH-, -N(C₁-C₆ alkyl)-, Q is ring C which is unsubstituted or substituted by m R₃;
the ring A is a 6- to 15-membered heterocyclyl ring; and when ring A is a 6-membered heterocyclyl ring, the ring A is 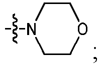
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
R₁, R₂, and R₃ are each independently hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, - CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₁, R₂, and R₃ are optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
n is 1, 2, or 3; when there is more than one R₁, R₁ groups are the same or different;
p is 1, 2, or 3; when there is more than one R₂, R₂ groups are the same or different;
m is 1, 2, or 3; when there is more than one R₃, R₃ groups are the same or different; provided that when Q is ring A, R₁ and R₂ are not hydrogen at the same time;
when the ring A is 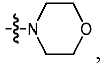
R₁, R₂, and R₃ are not hydrogen at the same time;
or when the ring A is 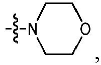
at least one of R₁ and R₂ is selected from: cyano, C₂-C₆ alkenyl, C₂-C₆ alkynyl, -C(O)NR₁₁R₁₂.

2. The diaryl compound, the tautomer, the stereoisomer, the solvate, the pharmaceutically acceptable salt, or the prodrug thereof according to claim 1, wherein W is selected from: -O-, - S-, -NH-;
preferably, W is -O-.

3. The diaryl compound, the tautomer, the stereoisomer, the solvate, the pharmaceutically acceptable salt, or the prodrug thereof according to claim 1, wherein L is -CH₂-, -CH₂CH₂-, - CH₂CH₂CH₂-;
preferably, L is -CH₂CH₂-.

4. The diaryl compound, the tautomer, the stereoisomer, the solvate, the pharmaceutically acceptable salt, or the prodrug thereof according to claim 1, wherein the ring A is a 7- to 15-membered monocyclic, fused, bridged, or spiro heterocyclyl ring;
preferably, the ring A comprises 1, 2, or 3 heteroatoms selected from N, O, or S;
when there is more than one heteroatom, the heteroatoms are the same or different;
preferably, the ring A comprises 1 N atom and 1 O atom.

5. The diaryl compound, the tautomer, the stereoisomer, the solvate, the pharmaceutically acceptable salt, or the prodrug thereof according to claim 1, wherein the ring A has a structure of 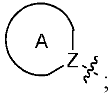
wherein Z represents C or N; preferably, Z is N;
preferably, the ring A further comprises 1 O atom;
preferably,

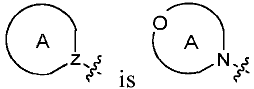

6. The diaryl compound, the tautomer, the stereoisomer, the solvate, the pharmaceutically acceptable salt, or the prodrug thereof according to claim 1, wherein ring B is a 6- to 12-membered sulfonyl-containing monocyclic, fused, spiro, or bridged heterocyclyl ring;
preferably, ring B is a 6- to 8-membered sulfonyl-containing monocyclic heterocyclyl ring;
preferably, ring B is a 7- to 12-membered sulfonyl-containing fused, spiro, or bridged heterocyclyl ring.

7. The diaryl compound, the tautomer, the stereoisomer, the solvate, the pharmaceutically acceptable salt, or the prodrug thereof according to claim 1, wherein ring B is 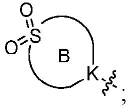 wherein K represents C or N.

8. The diaryl compound, the tautomer, the stereoisomer, the solvate, the pharmaceutically acceptable salt, or the prodrug thereof according to claim 1, having a structure of formula Ia, Ib, or Ic: 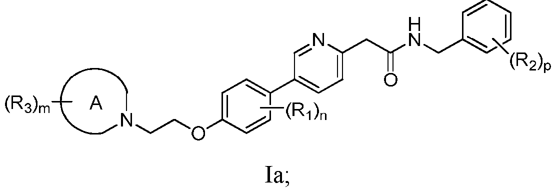 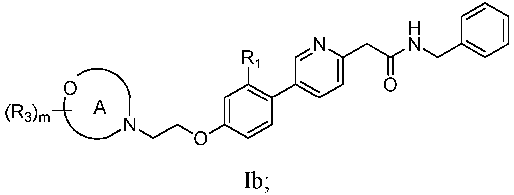 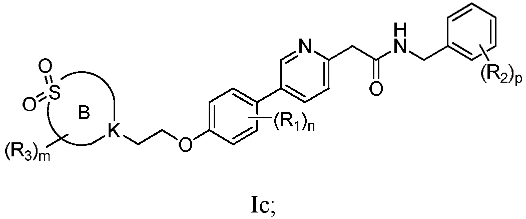
wherein K represents C or N;
ring A, ring B, m, n, p, R₁, R₂, and R₃ are as defined in claim 1.

9. The diaryl compound, the tautomer, the stereoisomer, the solvate, the pharmaceutically acceptable salt, or the prodrug thereof according to claim 1 or 8, wherein 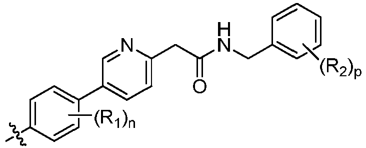 has a structure of

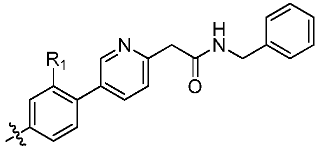

10. The diaryl compound, the tautomer, the stereoisomer, the solvate, the pharmaceutically acceptable salt, or the prodrug thereof according to claim 1 or 8, wherein the ring A is a 7- to 10-membered monocyclic heterocyclyl ring or a 7- to 12-membered fused, bridged, or spiro heterocyclyl ring.

11. The diaryl compound, the tautomer, the stereoisomer, the solvate, the pharmaceutically acceptable salt, or the prodrug thereof according to claim 1 or 8, wherein the ring A is selected from: 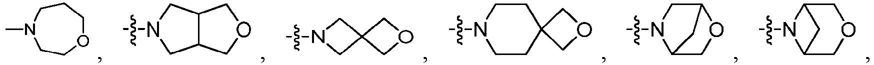 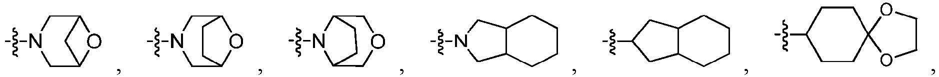 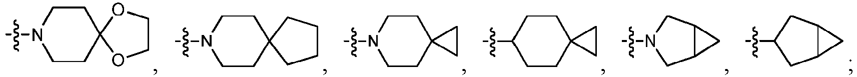 preferably, 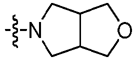 is

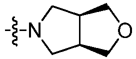

12. The diaryl compound, the tautomer, the stereoisomer, the solvate, the pharmaceutically acceptable salt, or the prodrug thereof according to claim 10, wherein R₁ and R₂ are each independently hydrogen or selected from: halogen, hydroxyl, amino, C₁-C₆ alkyl, -O-C₁-C₆ alkyl; the C₁-C₆ alkyl is optionally substituted by one or more than one halogen;
R₃ is selected from: halogen, hydroxyl, amino, C₁-C₆ alkyl, -O-C₁-C₆ alkyl; the C₁-C₆ alkyl is optionally substituted by one or more than one halogen;
and R₁ and R₂ are not hydrogen at the same time;
preferably, R₁, R₂, and R₃ are each independently selected from: halogen, C₁-C₆ alkyl; the C₁-C₆ alkyl is optionally substituted by one or more than one halogen;
more preferably, R₁, R₂, and R₃ are each independently selected from: fluorine, methyl, ethyl, propyl; the methyl, ethyl, and propyl are optionally substituted by one or more than one halogen;
preferably, the halogen is F.

13. The diaryl compound, the tautomer, the stereoisomer, the solvate, the pharmaceutically acceptable salt, or the prodrug thereof according to claim 1 or 8, wherein ring B is a 6- to 12-membered monocyclic, fused, spiro, or bridged heterocyclyl ring;
preferably, ring B is a 6- to 8-membered monocyclic heterocyclyl ring;
preferably, ring B is a 7- to 12-membered fused, spiro, or bridged heterocyclyl ring.

14. The diaryl compound, the tautomer, the stereoisomer, the solvate, the pharmaceutically acceptable salt, or the prodrug thereof according to claim 1 or 8, wherein the ring B is selected from:

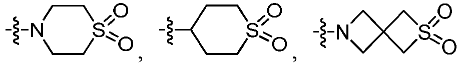

15. The diaryl compound, the tautomer, the stereoisomer, the solvate, the pharmaceutically acceptable salt, or the prodrug thereof according to claim 13, wherein R₁, R₂, and R₃ are each independently hydrogen or selected from: halogen, hydroxyl, amino, cyano, C₁-C₆ alkyl, -O-C₁-C₆ alkyl, -C(O)NR₁₁R₁₂;
R₁₁ and R₁₂ are each independently hydrogen or C₁-C₆ alkyl;
the C₁-C₆ alkyl is optionally substituted by one or more than one halogen;
preferably, R₁, R₂, and R₃ are each independently selected from: halogen, C₁-C₆ alkyl; the C₁-C₆ alkyl is optionally substituted by one or more than one halogen;
more preferably, R₁, R₂, and R₃ are each independently selected from: fluorine, methyl, ethyl, propyl; the C₁-C₆ alkyl is optionally substituted by one or more than one fluorine;
preferably, the halogen is F.

16. The diaryl compound, the tautomer, the stereoisomer, the solvate, the pharmaceutically acceptable salt, or the prodrug thereof according to claim 1, having a structure of Id: 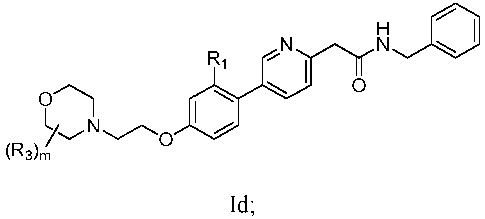
wherein m is 1 or 2; preferably, m is 1;
R₃ is hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₆ alkenyl, C₂-C₆ alkynyl, -C(O)NR₁₁R₁₂;
or R₁ and R₃ are each independently selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, - C(O)NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl; when there is more than one substituent, the substituents are the same or different.

17. The diaryl compound, the tautomer, the stereoisomer, the solvate, the pharmaceutically acceptable salt, or the prodrug thereof according to claim 16, wherein R₃ is hydrogen or selected from: halogen, hydroxyl, amino, cyano, C₁-C₆ alkyl, C₂-C₆ alkenyl, C₂-C₆ alkynyl, - O-C₁-C₆ alkyl, -COO-C₁-C₆ alkyl, -CO-C₁-C₆ alkyl, -C(O)NR₁₁R₁₂; R₁ is selected from: cyano, C₂-C₃ alkenyl, C₂-C₃ alkynyl, -C(O)NR₁₁R₁₂;
or R₁ and R₃ are each independently selected from: halogen, C₁-C₆ alkyl; the C₁-C₆ alkyl is optionally substituted by one or more than one substituent selected from the following: halogen, hydroxyl, amino, cyano, C₁-C₆ alkyl; when there is more than one substituent, the substituents are the same or different;
preferably, R₃ is hydrogen or selected from: halogen, methyl, ethyl, propyl; the methyl, ethyl, and propyl are optionally substituted by one or more than one halogen; R₁ is selected from: cyano, C₂-C₃ alkenyl, C₂-C₃ alkynyl;
preferably, R₁ and R₃ are each independently selected from: halogen, methyl, ethyl, propyl; the methyl, ethyl, and propyl are optionally substituted by one or more than one halogen;
preferably, the halogen is fluorine.

18. The diaryl compound, the tautomer, the stereoisomer, the solvate, the pharmaceutically acceptable salt, or the prodrug thereof according to claim 1, having a structure of Ie or If: 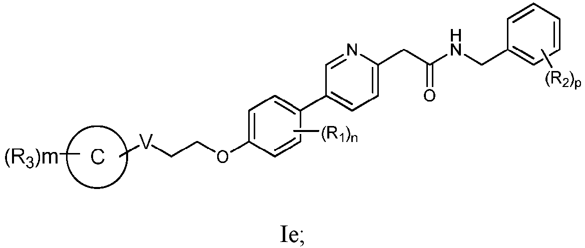 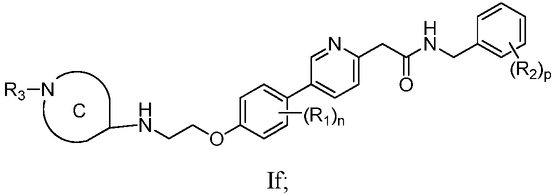
wherein ring C, R₁, R₂, n, and p are as defined in claim 1;
R₃ is C₁-C₆ alkyl, -CO-C₁-C₆ alkyl, -C(O)NR₁₁R₁₂, -COO-C₁-C₆ alkyl, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₃ is optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
preferably, ring C is a 4- to 8-membered heterocyclyl ring; preferably, ring C is a 4- to 6-membered heterocyclyl ring; more preferably, ring C is 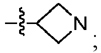
preferably, R₃ is -CO-C₁-C₆ alkyl;
preferably, the R₃ is optionally substituted by one or more than one substituent selected from the following: halogen, 3- to 6-membered cycloalkyl;
more preferably, R₃ is -CO-C₁-C₃ alkyl-cyclopropyl, -CO-C₁-C₃ alkyl-cyclobutyl.

19. The diaryl compound, the tautomer, the stereoisomer, the solvate, the pharmaceutically acceptable salt, or the prodrug thereof according to claim 1, wherein W is selected from: -O-, - S-, -NH-, -N(C₁-C₆ alkyl)-;
L is C₁-C₆ alkylene;
V is absent or selected from: -O-, -S-, -NH-, -N(C₁-C₆ alkyl)-;
when V is absent, Q is ring A which is unsubstituted or substituted by m R₃, or ring B which is unsubstituted or substituted by m R₃;
when V is selected from: -O-, -S-, -NH-, -N(C₁-C₆ alkyl)-, Q is ring C which is unsubstituted or substituted by m R₃;
the ring A is a 6- to 15-membered heterocyclyl ring; and when ring A is a 6-membered heterocyclyl ring, the ring A is 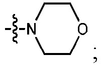
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
R₁, R₂, and R₃ are each independently hydrogen or selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, - CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂;
wherein R₁₁ and R₁₂ are each independently hydrogen or C₁-C₆ alkyl; or R₁₁ and R₁₂ together with the N atom to which they are attached form a 4- to 6-membered ring;
the R₁, R₂, and R₃ are optionally substituted by one or more than one substituent selected from the following: halogen, hydroxyl, amino, cyano, C₁-C₆ alkyl, 3- to 6-membered cycloalkyl; when there is more than one substituent, the substituents are the same or different;
n is 1, 2, or 3; when there is more than one R₁, R₁ groups are the same or different;
p is 1, 2, or 3; when there is more than one R₂, R₂ groups are the same or different;
m is 1, 2, or 3; when there is more than one R₃, R₃ groups are the same or different;
the diaryl compound of formula I satisfies 1, 2, or 3 of the following conditions:
(1) V is selected from: -O-, -S-, -NH-, -N(C₁-C₆ alkyl)-;
(2) Q is a 6- to 12-membered sulfonyl-containing heterocyclyl ring which is unsubstituted or substituted by m R₃;
(3) R₁ is selected from: hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂; or, R₁ is halogen, and ring A, ring B, or ring C is a fused ring, a bridged ring, or a spiro ring.

20. The diaryl compound, the tautomer, the stereoisomer, the solvate, the pharmaceutically acceptable salt, or the prodrug thereof according to claim 1 or 19, wherein the diaryl compound, the tautomer, the stereoisomer, the solvate, the pharmaceutically acceptable salt, or the prodrug thereof satisfies one or more than one of the following conditions:
a) V is selected from: -O-, -S-, -NH-, -N(C₁-C₆ alkyl)-;
b) Q is a 6- to 12-membered sulfonyl-containing heterocyclyl ring which is unsubstituted or substituted by m R₃;
c) R₁ is selected from: hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂, -COO-3- to 6-membered cycloalkyl, -CO-3- to 6-membered cycloalkyl, -S(O)₂-C₁-C₆ alkyl, -S(O)₂-3- to 6-membered cycloalkyl, -C(O)-C₁-C₆ alkyl-NR₁₁R₁₂.

21. The diaryl compound, the tautomer, the stereoisomer, the solvate, the pharmaceutically acceptable salt, or the prodrug thereof according to claim 20, wherein the diaryl compound, the tautomer, the stereoisomer, the solvate, the pharmaceutically acceptable salt, or the prodrug thereof satisfies one or more than one of the following conditions:
d) V is selected from -NH-;
e) Q is an unsubstituted 6- to 12-membered sulfonyl-containing heterocyclyl ring;
f) R₁ is selected from: cyano, C₁-C₆ alkyl, and C₂-C₆ alkynyl.

22. The diaryl compound, the tautomer, the stereoisomer, the solvate, the pharmaceutically acceptable salt, or the prodrug thereof according to claim 1 or 19, wherein the diaryl compound, the tautomer, the stereoisomer, the solvate, the pharmaceutically acceptable salt, or the prodrug thereof satisfies one of the following conditions:
g) R₁ is halogen, and ring A, ring B, or ring C is a fused ring, a bridged ring, or a spiro ring;
h) the diaryl compound of formula I is 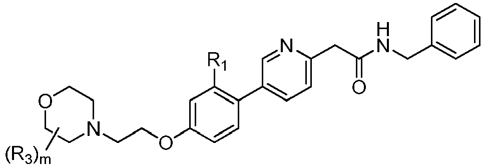 Id; m is 1 or 2; R₃ is selected from: halogen, hydroxyl, amino, cyano, C₁-C₆ alkyl, C₂-C₆ alkenyl, C₂-C₆ alkynyl, -O-C₁-C₆ alkyl, -COO-C₁-C₆ alkyl, -CO-C₁-C₆ alkyl, -C(O)NR₁₁R₁₂; R₁ is selected from: cyano, C₂-C₆ alkenyl, C₂-C₆ alkynyl, -C(O)NR₁₁R₁₂;
i) provided that when Q is ring A, R₁ and R₂ are not hydrogen at the same time; and when the ring A is 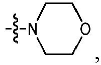 R₃ is not hydrogen, or at least one of R₁ and R₂ is selected from: cyano, C₂-C₆ alkenyl, C₂-C₆ alkynyl, -C(O)NR₁₁R₁₂.

23. The diaryl compound, the tautomer, the stereoisomer, the solvate, the pharmaceutically acceptable salt, or the prodrug thereof according to claim 1 or 19, wherein the diaryl compound, the tautomer, the stereoisomer, the solvate, the pharmaceutically acceptable salt, or the prodrug thereof satisfies one of the following conditions:
j) W is selected from -O-;
k) L is C₁-C₆ alkylene;
l) V is absent or selected from: -NH-; when V is absent, Q is ring A which is unsubstituted or substituted by m R₃, or ring B which is unsubstituted or substituted by m R₃; when V is selected from: -NH-, Q is ring C which is unsubstituted or substituted by m R₃; the ring A is a 6- to 15-membered heterocyclyl ring; and when ring A is a 6-membered heterocyclyl ring, the ring A is 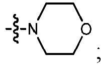 the ring B is a 6- to 12-membered sulfonyl-containing heterocyclyl ring; the ring C is a 4- to 15-membered heterocyclyl ring or a 6- to 12-membered sulfonyl-containing heterocyclyl ring; m is 1, 2, or 3; when there is more than one R₃, R₃ groups are the same or different; R₃ is C₁-C₆ alkyl or -CO-C₁-C₆ alkyl; R₃ is optionally substituted by one or more than one 3- to 6-membered cycloalkyl; when there is more than one substituent, the substituents are the same or different;
m) n is 1, 2, or 3; when there is more than one R₁, R₁ groups are the same or different; R₁ is hydrogen, halogen, cyano, unsubstituted C₁-C₆ alkyl, or unsubstituted C₂-C₆ alkynyl;
n) p is 1, 2, or 3; when there is more than one R₂, R₂ groups are the same or different; R₂ is hydrogen or halogen;
o) the diaryl compound of formula I satisfies 1, 2, or 3 of the following conditions: (1) V is selected from -NH-; (2) Q is a 6- to 12-membered sulfonyl-containing heterocyclyl ring which is unsubstituted or substituted by m R₃; (3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring.

24. The diaryl compound, the tautomer, the stereoisomer, the solvate, the pharmaceutically acceptable salt, or the prodrug thereof according to claim 1 or 19, wherein the diaryl compound, the tautomer, the stereoisomer, the solvate, the pharmaceutically acceptable salt, or the prodrug thereof is as described in any one of the following schemes:
scheme 1:
W is selected from -O-;
L is C₁-C₆ alkylene;
V is absent or selected from: -NH-;
when V is absent, Q is ring A which is unsubstituted or substituted by m R₃, or ring B which is unsubstituted or substituted by m R₃;
when V is selected from: -NH-, Q is ring C which is unsubstituted or substituted by m R₃;
the ring A is a 6- to 15-membered heterocyclyl ring; and when ring A is a 6-membered heterocyclyl ring, the ring A is 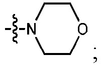
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
n is 1, 2, or 3; when there is more than one R₁, R₁ groups are the same or different;
R₁ is hydrogen, halogen, cyano, unsubstituted C₁-C₆ alkyl, or unsubstituted C₂-C₆ alkynyl;
p is 1, 2, or 3; when there is more than one R₂, R₂ groups are the same or different;
R₂ is hydrogen or halogen;
m is 1, 2, or 3; when there is more than one R₃, R₃ groups are the same or different;
R₃ is C₁-C₆ alkyl or -CO-C₁-C₆ alkyl; R₃ is optionally substituted by one or more than one 3- to 6-membered cycloalkyl; when there is more than one substituent, the substituents are the same or different;
the diaryl compound of formula I satisfies 1, 2, or 3 of the following conditions:
(1) V is selected from -NH-;
(2) Q is a 6- to 12-membered sulfonyl-containing heterocyclyl ring which is unsubstituted or substituted by m R₃;
(3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring;
scheme 2:
W is selected from -O-;
L is C₁-C₆ alkylene;
V is absent or selected from: -NH-;
when V is absent, Q is ring A which is unsubstituted or substituted by m R₃, or ring B which is unsubstituted or substituted by m R₃;
when V is selected from: -NH-, Q is ring C which is unsubstituted or substituted by m R₃;
the ring A is a 6- to 15-membered heterocyclyl ring; and when ring A is a 6-membered heterocyclyl ring, the ring A is 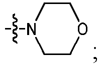
the ring B is a 6- to 12-membered sulfonyl-containing heterocyclyl ring;
the ring C is a 4- to 15-membered heterocyclyl ring or a 6- to 12-membered sulfonyl-containing heterocyclyl ring;
n is 1;
R₁ is hydrogen, halogen, cyano, unsubstituted C₁-C₆ alkyl, or unsubstituted C₂-C₆ alkynyl;
p is 1;
R₂ is hydrogen;
m is 1, 2, or 3; when there is more than one R₃, R₃ groups are the same or different;
R₃ is C₁-C₆ alkyl or -CO-C₁-C₆ alkyl; R₃ is optionally substituted by one or more than one 3- to 6-membered cycloalkyl; when there is more than one substituent, the substituents are the same or different;
the diaryl compound of formula I satisfies 1, 2, or 3 of the following conditions:
(1) V is selected from -NH-;
(2) Q is a 6- to 12-membered sulfonyl-containing heterocyclyl ring which is unsubstituted or substituted by m R₃;
(3) R₁ is cyano, unsubstituted C₁-C₆ alkyl, or unsubstituted C₂-C₆ alkynyl; or, R₁ is halogen, and ring A, ring B, or ring C is a fused ring, a bridged ring, or a spiro ring.

25. The diaryl compound, the tautomer, the stereoisomer, the solvate, the pharmaceutically acceptable salt, or the prodrug thereof according to claim 1 or 19, wherein the diaryl compound, the tautomer, the stereoisomer, the solvate, the pharmaceutically acceptable salt, or the prodrug thereof is as described in any one of the following schemes:
p) L is -CH₂-, -CH₂CH₂-, -CH₂CH₂CH₂-;
q) in the ring A, the 6- to 15-membered heterocyclyl ring is a 6- to 8-membered heterocyclyl ring;
r) in the ring A, the 6- to 15-membered heterocyclyl ring is a monocyclic ring, a fused ring, a bridged ring, or a spiro ring;
s) in the ring A, the 6- to 15-membered heterocyclyl ring is a saturated ring;
t) in the ring A, the heteroatoms in the 6- to 15-membered heterocyclyl ring are N and/or O;
u) in the ring A, the 6- to 15-membered heterocyclyl ring is connected to V through an N atom;
v) in the ring C, the 4- to 15-membered heterocyclyl ring is a 6- to 8-membered heterocyclyl ring;
w) in the ring C, the 4- to 15-membered heterocyclyl ring is a monocyclic ring, a fused ring, a bridged ring, or a spiro ring;
x) in the ring C, the 4- to 15-membered heterocyclyl ring is a saturated ring;
y) in the ring C, the heteroatoms in the 4- to 15-membered heterocyclyl ring are N and/or O;
z) in the ring C, the 4- to 15-membered heterocyclyl ring is connected to V through an N atom;
aa) in the ring B, the 6- to 12-membered sulfonyl-containing heterocyclyl ring is a 6- to 8-membered sulfonyl-containing heterocyclyl ring;
bb) in the ring B, the 6- to 12-membered sulfonyl-containing heterocyclyl ring is a monocyclic ring, a fused ring, a bridged ring, or a spiro ring;
cc) in the ring B, the 6- to 12-membered sulfonyl-containing heterocyclyl ring is a monocyclic ring or a spiro ring;
dd) in the ring B, the 6- to 12-membered sulfonyl-containing heterocyclyl ring is a saturated ring;
ee) in the ring B, the heteroatoms in the 6- to 12-membered sulfonyl-containing heterocyclyl ring are -S(=O)₂- and/or N;
ff) in the ring B, the 6- to 12-membered sulfonyl-containing heterocyclyl ring is connected to V through an N atom;
gg) in the ring C, the 6- to 12-membered sulfonyl-containing heterocyclyl ring is a 6- to 8-membered sulfonyl-containing heterocyclyl ring;
hh) in the ring C, the 6- to 12-membered sulfonyl-containing heterocyclyl ring is a monocyclic ring, a fused ring, a bridged ring, or a spiro ring;
ii) in the ring C, the 6- to 12-membered sulfonyl-containing heterocyclyl ring is a saturated ring;
jj) in the ring C, the heteroatoms in the 6- to 12-membered sulfonyl-containing heterocyclyl ring are -S(=O)₂- and/or N;
kk) in the ring C, the 6- to 12-membered sulfonyl-containing heterocyclyl ring is connected to V through an N atom;
ll) in the R₁, the halogen is fluorine or chlorine;
mm) in the R₁, the C₁-C₆ alkyl is methyl or ethyl;
nn) in the R₁, the C₂-C₆ alkynyl is ethynyl or propynyl;
oo) in the R₂, the halogen is fluorine or chlorine;
pp) in the R₃, the C₁-C₆ alkyl is methyl or ethyl;
qq) in the R₃, the C₁-C₆ alkyl in the "-CO-C₁-C₆ alkyl" is methyl or ethyl;
rr) the 4- to 6-membered ring formed by R₁₁ and R₁₂ together with the N atom to which they are attached is a 4- to 6-membered N-containing heterocycloalkyl ring or a 5- to 6-membered heteroaryl ring;
ss) the solvate is a hydrate.

26. The diaryl compound, the tautomer, the stereoisomer, the solvate, the pharmaceutically acceptable salt, or the prodrug thereof according to claim 25, wherein the diaryl compound, the tautomer, the stereoisomer, the solvate, the pharmaceutically acceptable salt, or the prodrug thereof is as described in any one of the following schemes:
tt) L is -CH₂CH₂-;
uu) the ring A is 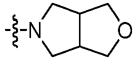 (for example, 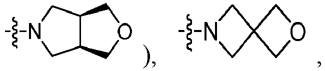 or 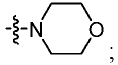
vv) in the ring C, the 4- to 15-membered heterocyclyl ring is 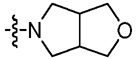 (for example, 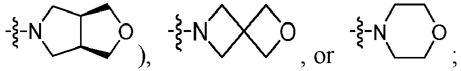
ww) in the ring B, the 6- to 12-membered sulfonyl-containing heterocyclyl ring is 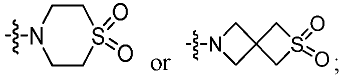
xx) in the ring C, the 6- to 12-membered sulfonyl-containing heterocyclyl ring is

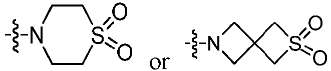

27. The diaryl compound, the tautomer, the stereoisomer, the solvate, the pharmaceutically acceptable salt, or the prodrug thereof according to claim 1 or 19, wherein the diaryl compound is any one of the following compounds:

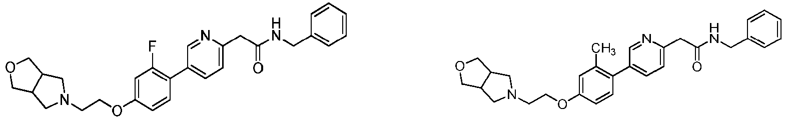

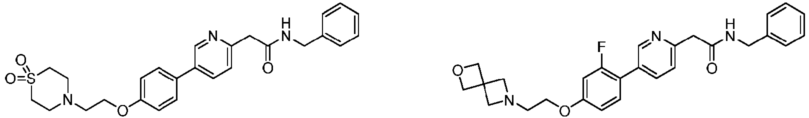

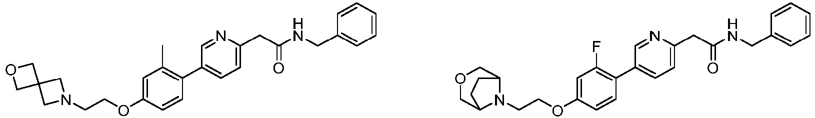

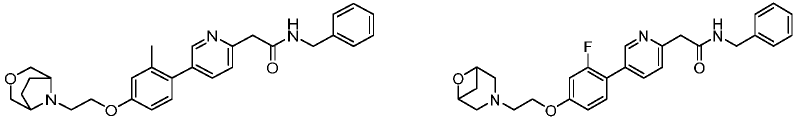

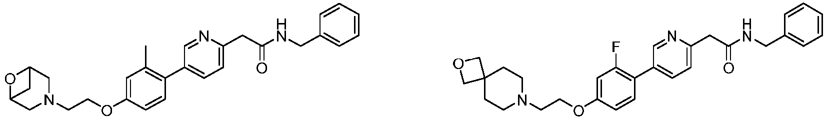

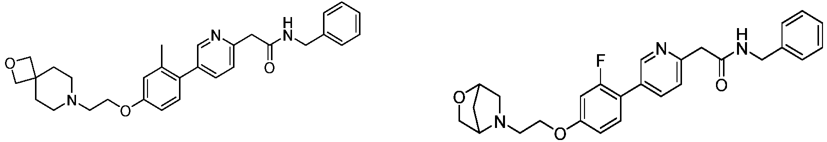

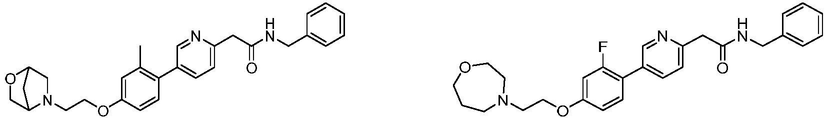

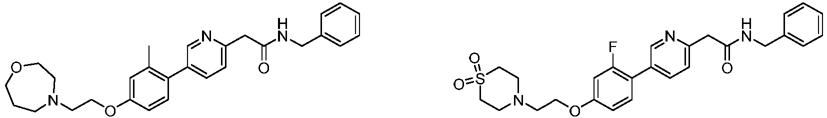

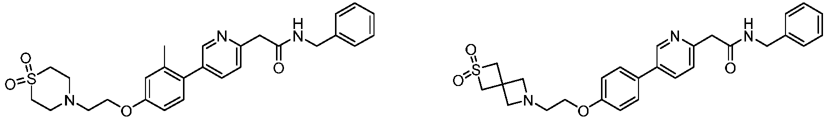

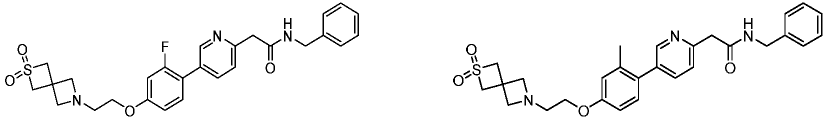

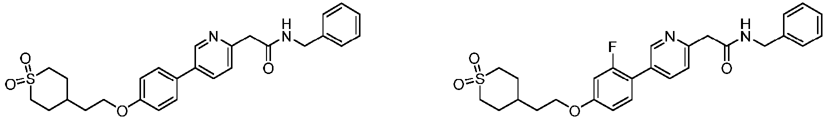

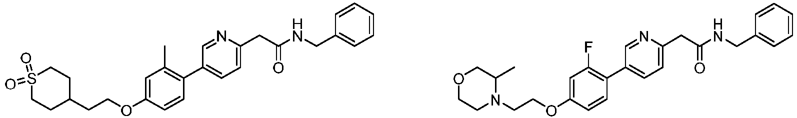

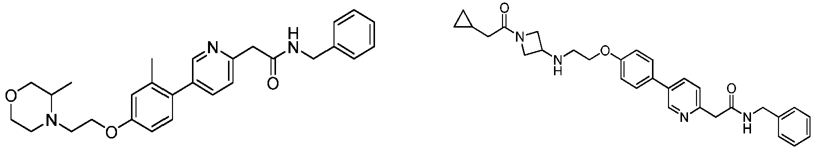

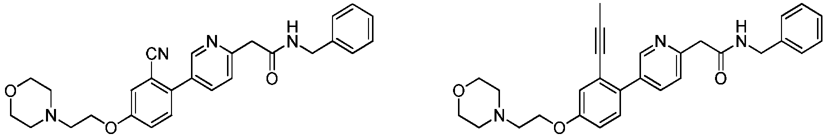

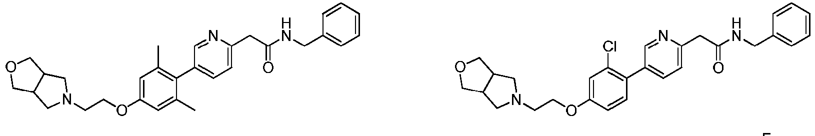

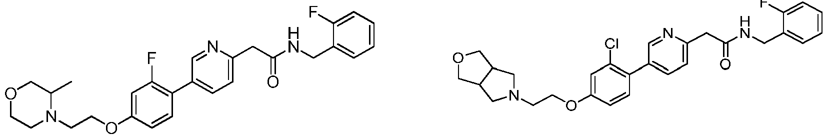

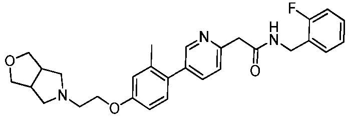

28. The diaryl compound, the tautomer, the stereoisomer, the solvate, the pharmaceutically acceptable salt, or the prodrug thereof according to claim 1 or 19, wherein the diaryl compound is any one of the following compounds:

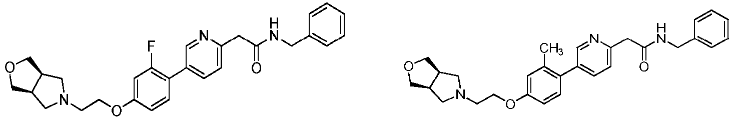

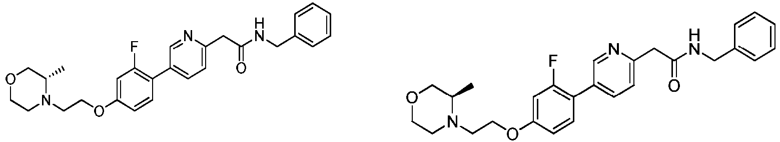

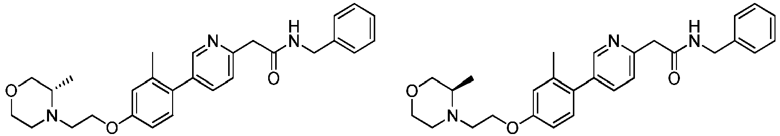

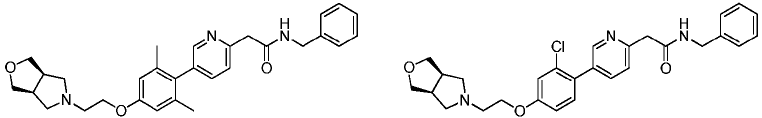

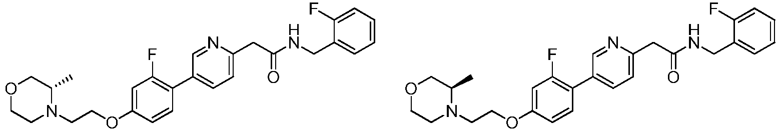

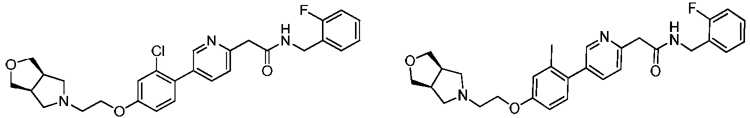

29. A compound B having a structure of 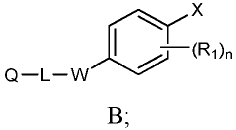
wherein Q, L, W, R₁, and n are as defined in claim 1 or 19;
X is halogen or substituent G;
the substituent G is selected from: borate group, boronic acid group, alkyltin, trifluoromethanesulfonic acid group, methanesulfonic acid group, *p*-toluenesulfonic acid group;
preferably, the halogen is chlorine, bromine, or iodine;
preferably, the compound B is a compound B used as an intermediate.

30. The compound B according to claim 29, wherein the compound B has the following structures: 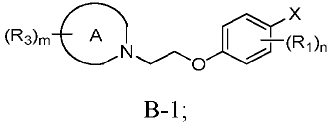 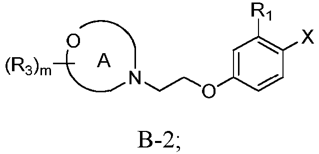 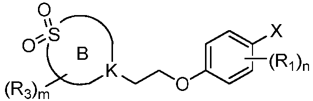 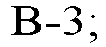 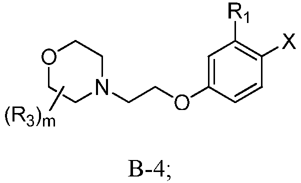 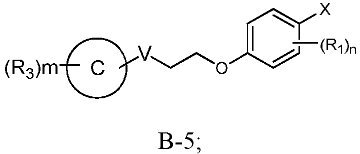 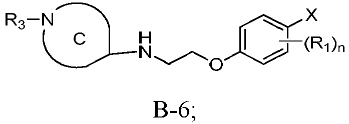
wherein K represents C or N;
ring A, ring B, m, n, p, R₁, R₂, and R₃ are as defined in claim 1 or 19;
X is halogen or substituent G;
the substituent G is selected from: borate group, boronic acid group, alkyltin, trifluoromethanesulfonic acid group, methanesulfonic acid group, *p*-toluenesulfonic acid group;
preferably, the halogen is chlorine, bromine, or iodine.

31. The compound B according to claim 29 or 30, wherein the ring A is a 7- to 15-membered monocyclic, fused, bridged, or spiro heterocyclyl ring;
preferably, the ring A is a 7- to 10-membered monocyclic heterocyclyl ring or a 7- to 12-membered fused, bridged, or spiro heterocyclyl ring;
preferably, the ring A comprises 1 N atom and 1 O atom.

32. The compound B according to claim 29 or 30, wherein ring B is a 6- to 12-membered monocyclic, fused, spiro, or bridged heterocyclyl ring;
preferably, ring B is a 6- to 8-membered monocyclic heterocyclyl ring;
preferably, ring B is a 7- to 12-membered fused, spiro, or bridged heterocyclyl ring.

33. A method for preparing the diaryl compound, the tautomer, the stereoisomer, the solvate, the pharmaceutically acceptable salt, or the prodrug thereof according to any one of claims 1 to 28, comprising:
obtaining the diaryl compound by reacting intermediate B according to any one of claims 29 to 32 with an intermediate C under an alkaline condition; 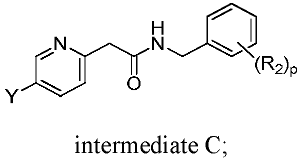
wherein R₂ and p are as defined in claim 1 or 19;
Y is halogen or substituent G;
the substituent G is selected from: borate group, boronic acid group, alkyltin, trifluoromethanesulfonic acid group, methanesulfonic acid group, *p*-toluenesulfonic acid group;
when X in intermediate B is halogen, Y is G;
when X in intermediate B is G, X is halogen;
preferably, the halogen is chlorine, bromine, or iodine;
preferably, intermediate C has a structure of

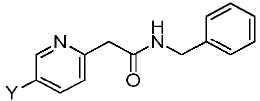

34. A pharmaceutical composition, comprising the diaryl compound, the tautomer, the stereoisomer, the solvate, the pharmaceutically acceptable salt, or the prodrug thereof according to any one of claims 1 to 28.

35. A pharmaceutical composition, comprising the diaryl compound, the tautomer, the stereoisomer, the solvate, the pharmaceutically acceptable salt, or the prodrug thereof according to any one of claims 1 to 28, and a pharmaceutically acceptable carrier and/or other active drug.

36. A use of the diaryl compound, the tautomer, the stereoisomer, the solvate, the pharmaceutically acceptable salt, or the prodrug thereof according to any one of claims 1 to 28, or a use of the pharmaceutical composition according to claim 34, or a use of the pharmaceutical composition according to claim 35, comprising:
1) inhibiting tubulin polymerization and/or Src kinase;
2) preventing and/or treating a disease related to tubulin polymerization and/or Src kinase;
3) preparing a tubulin polymerization and/or Src kinase inhibitor;
4) preparing a drug, pharmaceutical composition, or formulation for preventing and/or treating a disease related to tubulin polymerization and/or Src kinase;
preferably, the disease related to tubulin polymerization and/or Src kinase comprises tumor and skin disease;
preferably, the drug is an external preparation;
preferably, the drug is transdermally administered.

37. A use of the diaryl compound, the tautomer, the stereoisomer, the solvate, the pharmaceutically acceptable salt, or the prodrug thereof according to any one of claims 1 to 28, or the pharmaceutical composition according to claim 34, or the pharmaceutical composition according to claim 35 in the preparation of a drug for the treatment of tumor and/or skin disease;
preferably, the drug is an external preparation;
preferably, the drug is transdermally administered.

38. The use according to claim 37, wherein the tumor comprises: solid tumor, sarcoma, and hematological cancer;
preferably comprising: breast cancer, ovarian cancer, prostate cancer, cervical cancer, testicular cancer, colon cancer, colorectal cancer, liver cancer, non-small cell lung cancer, squamous cell carcinoma, small cell lung cancer, gastric cancer, gastrointestinal stromal tumor, pancreatic cancer, bladder cancer, germ cell tumor, mast cell tumor, mastocytosis, glioblastoma, neuroblastoma, astrocytoma, melanoma, B-cell lymphoma, T-cell lymphoma, slowly progressive lymphoma, Hodgkin lymphoma, non-Hodgkin lymphoma, acute myeloid leukemia, acute lymphoblastic leukemia, chronic myeloid leukemia, chronic lymphocytic leukemia, myeloma, and/or myelodysplastic syndrome.

39. The use according to claim 37, wherein the skin disease comprises: actinic keratosis, psoriasis, atopic dermatitis, psoriasis, vitiligo, roseola, and/or systemic lupus erythematosus.

40. A use of the diaryl compound, the tautomer, the stereoisomer, the solvate, the pharmaceutically acceptable salt, or the prodrug thereof according to any one of claims 1 to 28, or the pharmaceutical composition according to claim 34, or the pharmaceutical composition according to claim 35 for treating or preventing tumor and/or skin disease.

41. A method for inhibiting Src kinase, or preventing and/or treating a disease related to Src kinase, comprising the steps of: administering to a subject in need thereof the diaryl compound, the tautomer, the stereoisomer, the solvate, the pharmaceutically acceptable salt, or the prodrug thereof according to any one of claims 1 to 28, or the pharmaceutical composition according to claim 34, or the pharmaceutical composition according to claim 35.

42. A method for inhibiting tubulin, or preventing and/or treating a disease related to tubulin, comprising the steps of: administering to a subject in need thereof the diaryl compound, the tautomer, the stereoisomer, the solvate, the pharmaceutically acceptable salt, or the prodrug thereof according to any one of claims 1 to 28, or the pharmaceutical composition according to claim 34, or the pharmaceutical composition according to claim 35.
